# EUROPEAN PATENT SPECIFICATION

(11) **EP 1 233 783 B1**
(45) Date of publication and mention of the grant of the patent: **09.03.2005**
(21) Application number: 00982225.5
(22) Date of filing: 22.11.2000
(51) Int. Cl.: A61K 39/295, C07K 19/00, A61P 31/12, C12N 15/62, C12N 5/10

(54) **HBV/HCV VIRUS-LIKE PARTICLE**
HBV/HCV Virus-ohnliche Particle
PARTICULE DE TYPE VIRUS HBV/HCV

(30) Priority: 24.11.1999 US 167224 P
(43) Date of publication of application: 28.08.2002
(60) Divisional application: 04078119.7
(73) Proprietor: CHIRON CORPORATION, Emeryville, California 94608 (US)
(72) Inventor: SELBY, Mark, Chiron Corporation, Emeryville, CA 94608 (US); GLAZER, Edward, Chiron Corporation, Emeryville, CA 94608 (US); HOUGHTON, Michael, Chiron Corporation, Emeryville, CA 94608 (US)
(74) Representative: Hallybone, Huw George
(86) International application number: PCT/US2000/032249
(87) International publication number: WO 2001/038358

(56) References cited:
- EP-A- 0 201 416
- US-A- 5 098 704
- LEE IHL-HEE ET AL: "Presentation of the hydrophilic domains of hepatitis C viral E2 envelope glycoprotein on hepatitis B surface antigen particles." JOURNAL OF MEDICAL VIROLOGY, vol. 50, no. 2, 1996, pages 145-151, XP000990962 ISSN: 0146-6615 cited in the application
- DATABASE WPI Section Ch, Week 199814 Derwent Publications Ltd., London, GB; Class B04, AN 1998-156879 XP002164673 LEE: & KR 97 010 787 A (LG CHEM LTD), 27 March 1997 (1997-03-27)
- MAJOR MARIAN E ET AL: "DNA-based immunization with chimeric vectors for the induction of immune responses against the hepatitis C virus nucleocapsid." JOURNAL OF VIROLOGY, vol. 69, no. 9, 1995, pages 5798-5805, XP002164671 ISSN: 0022-538X
- INCHAUSPE G ET AL: "DNA vaccination for the induction of immune responses against hepatitis C virus proteins" VACCINE,GB,BUTTERWORTH SCIENTIFIC. GUILDFORD, vol. 15, no. 8, 1 June 1997 (1997-06-01), pages 853-856, XP004075669 ISSN: 0264-410X cited in the application
- NAKANO ISAO ET AL: "Immunization with plasmid DNA encoding hepatitis C virus envelope E2 antigenic domains induces antibodies whose immune reactivity is linked to the injection mode." JOURNAL OF VIROLOGY, vol. 71, no. 9, 1997, pages 7101-7109, XP002164672 ISSN: 0022-538X cited in the application

### SEQUENCE LISTING

<110> Chiron Corporation
   Selby, Mark
   Glazer, Edward
   Houghton, Michael
<120> HBV/HCV VIRUS-LIKE PARTICLE
<130> PP01635.003
<140>
   <141>
<160> 7
<170> Patent In Ver. 2.0
<210> 1
   <211> 4276
   <212> DNA
   <213> Artificial Sequence
<220>
   <223> Description of Artificial Sequence: plasmid pCMVII
<400> 1
   tcgcgcgttt cggtgatgac ggtgaaaacc tctgacacat gcagctcccg gagacggtca 60
   cagcttgtct gtaagcggat gccgggagca gacaagcccg tcagggcgcg tcagcgggtg 120
   ttggcgggtg tcggggctgg cttaactatg cggcatcaga gcagattgta ctgagagtgc 180
   accatatgaa gctttttgca aaagcctagg cctccaaaaa agcctcctca ctacttctgg 240
   aatagctcag aggccgaggc ggcctcggcc tctgcataaa taaaaaaaat tagtcagcca 300
   tggggcggag aatgggcgga actgggcggg gagggaatta ttggctattg gccattgcat 360
   acgttgtatc tatatcataa tatgtacatt tatattggct catgtccaat atgaccgcca 420
   tgttgacatt gattattgac tagttattaa tagtaatcaa ttacggggtc attagttcat 480
   agcccatata tggagttccg cgttacataa cttacggtaa atggcccgcc tggctgaccg 540
   cccaacgacc cccgcccatt gacgtcaata atgacgtatg ttcccatagt aacgccaata 600
   gggactttcc attgacgtca atgggtggag tatttacggt aaactgccca cttggcagta 660
   catcaagtgt atcatatgcc aagtccgccc cctattgacg tcaatgacgg taaatggccc 720
   gcctggcatt atgcccagta catgacctta cgggactttc ctacttggca gtacatctac 780
   gtattagtca tcgctattac catggtgatg cggttttggc agtacaccaa tgggcgtgga 840
   tagcggtttg actcacgggg atttccaagt ctccacccca ttgacgtcaa tgggagtttg 900
   ttttggcacc aaaatcaacg ggactttcca aaatgtcgta ataaccccgc cccgttgacg 960
   caaatgggcg gtaggcgtgt acggtgggag gtctatataa gcagagctcg tttagtgaac 1020
   cgtcagatcg cctggagacg ccatccacgc tgttttgacc tccatagaag acaccgggac 1080
   cgatccagcc tccgcggccg ggaacggtgc attggaacgc ggattccccg tgccaagagt 1140
   gacgtaagta ccgcctatag actctatagg cacacccctt tggctcttat gcatgctata 1200
   ctgtttttgg cttggggcct atacaccccc gctccttatg ctataggtga tggtatagct 1260
   tagcctatag gtgtgggtta ttgaccatta ttgaccactc ccctattggt gacgatactt 1320
   tccattacta atccataaca tggctctttg ccacaactat ctctattggc tatatgccaa 1380
   tactctgtcc ttcagagact gacacggact ctgtattttt acaggatggg gtccatttat 1440
   tatttacaaa ttcacatata caacaacgcc gtcccccgtg cccgcagttt ttattaaaca 1500
   tagcgtggga tctccgacat ctcgggtacg tgttccggac atgggctctt ctccggtagc 1560
   ggcggagctt ccacatccga gccctggtcc catccgtcca gcggctcatg gtcgctcggc 1620
   agctccttgc tcctaacagt ggaggccaga cttaggcaca gcacaatgcc caccaccacc 1680
   agtgtgccgc acaaggccgt ggcggtaggg tatgtgtctg aaaatgagct cggagattgg 1740
   gctcgcacct ggacgcagat ggaagactta aggcagcggc agaagaagat gcaggcagct 1800
   gagttgttgt attctgataa gagtcagagg taactcccgt tgcggtgctg ttaacggtgg 1860
   agggcagtgt agtctgagca gtactcgttg ctgccgcgcg cgccaccaga cataatagct 1920
   gacagactaa cagactgttc ctttccatgg gtcttttctg cagtcaccgt cgtcgaccta 1980
   agaattcaga ctcgagcaag tctagaaagg cgcgccaaga tatcaaggat ccactacgcg 2040
   ttagagctcg ctgatcagcc tcgactgtgc cttctagttg ccagccatct gttgtttgcc 2100
   cctcccccgt gccttccttg accctggaag gtgccactcc cactgtcctt tcctaataaa 2160
   atgaggaaat tgcatcgcat tgtctgagta ggtgtcattc tattctgggg ggtggggtgg 2220
   ggcaggacag caagggggag gattgggaag acaatagcag gcatgctggg gagctcttcc 2280
   gcttcctcgc tcactgactc gctgcgctcg gtcgttcggc tgcggcgagc ggtatcagct 2340
   cactcaaagg cggtaatacg gttatccaca gaatcagggg ataacgcagg aaagaacatg 2400
   tgagcaaaag gccagcaaaa ggccaggaac cgtaaaaagg ccgcgttgct ggcgtttttc 2460
   cataggctcc gcccccctga cgagcatcac aaaaatcgac gctcaagtca gaggtggcga 2520
   aacccgacag gactataaag ataccaggcg tttccccctg gaagctccct cgtgcgctct 2580
   cctgttccga ccctgccgct taccggatac ctgtccgcct ttctcccttc gggaagcgtg 2640
   gcgctttctc aatgctcacg ctgtaggtat ctcagttcgg tgtaggtcgt tcgctccaag 2700
   ctgggctgtg tgcacgaacc ccccgttcag cccgaccgct gcgccttatc cggtaactat 2760
   cgtcttgagt ccaacccggt aagacacgac ttatcgccac tggcagcagc cactggtaac 2820
   aggattagca gagcgaggta tgtaggcggt gctacagagt tcttgaagtg gtggcctaac 2880
   tacggctaca ctagaaggac agtatttggt atctgcgctc tgctgaagcc agttaccttc 2940
   ggaaaaagag ttggtagctc ttgatccggc aaacaaacca ccgctggtag cggtggtttt 3000
   tttgtttgca agcagcagat tacgcgcaga aaaaaaggat ctcaagaaga tcctttgatc 3060
   ttttctacgg ggtctgacgc tcagtggaac gaaaactcac gttaagggat tttggtcatg 3120
   agattatcaa aaaggatctt cacctagatc cttttaaatt aaaaatgaag ttttaaatca 3180
   atctaaagta tatatgagta aacttggtct gacagttacc aatgcttaat cagtgaggca 3240
   cctatctcag cgatctgtct atttcgttca tccatagttg cctgactccc cgtcgtgtag 3300
   ataactacga tacgggaggg cttaccatct ggccccagtg ctgcaatgat accgcgagac 3360
   ccacgctcac cggctccaga tttatcagca ataaaccagc cagccggaag ggccgagcgc 3420
   agaagtggtc ctgcaacttt atccgcctcc atccagtcta ttaattgttg ccgggaagct 3480
   agagtaagta gttcgccagt taatagtttg cgcaacgttg ttgccattgc tacaggcatc 3540
   gtggtgtcac gctcgtcgtt tggtatggct tcattcagct ccggttccca acgatcaagg 3600
   cgagttacat gatcccccat gttgtgcaaa aaagcggtta gctccttcgg tcctccgatc 3660
   gttgtcagaa gtaagttggc cgcagtgtta tcactcatgg ttatggcagc actgcataat 3720
   tctcttactg tcatgccatc cgtaagatgc ttttctgtga ctggtgagta ctcaaccaag 3780
   tcattctgag aatagtgtat gcggcgaccg agttgctctt gcccggcgtc aatacgggat 3840
   aataccgcgc cacatagcag aactttaaaa gtgctcatca ttggaaaacg ttcttcgggg 3900
   cgaaaactct caaggatctt accgctgttg agatccagtt cgatgtaacc cactcgtgca 3960
   cccaactgat cttcagcatc ttttactttc accagcgttt ctgggtgagc aaaaacagga 4020
   aggcaaaatg ccgcaaaaaa gggaataagg gcgacacgga aatgttgaat actcatactc 4080
   ttcctttttc aatattattg aagcatttat cagggttatt gtctcatgag cggatacata 4140
   tttgaatgta tttagaaaaa taaacaaata ggggttccgc gcacatttcc ccgaaaagtg 4200
   ccacctgacg tctaagaaac cattattatc atgacattaa cctataaaaa taggcgtatc 4260
   acgaggccct ttcgtc 4276
<210> 2
   <211> 5128
   <212> DNA
   <213> Artificial Sequence
<220>
<223> Description of Artificial Sequence: plasmid pCMVII-pS2-SAg
<220>
   <221> CDS
   <222> (1988)..(2830)
<400> 2
   tcgcgcgttt cggtgatgac ggtgaaaacc tctgacacat gcagctcccg gagacggtca 60
   cagcttgtct gtaagcggat gccgggagca gacaagcccg tcagggcgcg tcagcgggtg 120
   ttggcgggtg tcggggctgg cttaactatg cggcatcaga gcagattgta ctgagagtgc 180
   accatatgaa gctttttgca aaagcctagg cctccaaaaa agcctcctca ctacttctgg 240
   aatagctcag aggccgaggc ggcctcggcc tctgcataaa taaaaaaaat tagtcagcca 300
   tggggcggag aatgggcgga actgggcggg gagggaatta ttggctattg gccattgcat 360
   acgttgtatc tatatcataa tatgtacatt tatattggct catgtccaat atgaccgcca 420
   tgttgacatt gattattgac tagttattaa tagtaatcaa ttacggggtc attagttcat 480
   agcccatata tggagttccg cgttacataa cttacggtaa atggcccgcc tggctgaccg 540
   cccaacgacc cccgcccatt gacgtcaata atgacgtatg ttcccatagt aacgccaata 600
   gggactttcc attgacgtca atgggtggag tatttacggt aaactgccca cttggcagta 660
   catcaagtgt atcatatgcc aagtccgccc cctattgacg tcaatgacgg taaatggccc 720
   gcctggcatt atgcccagta catgacctta cgggactttc ctacttggca gtacatctac 780
   gtattagtca tcgctattac catggtgatg cggttttggc agtacaccaa tgggcgtgga 840
   tagcggtttg actcacgggg atttccaagt ctccacccca ttgacgtcaa tgggagtttg 900
   ttttggcacc aaaatcaacg ggactttcca aaatgtcgta ataaccccgc cccgttgacg 960
   caaatgggcg gtaggcgtgt acggtgggag gtctatataa gcagagctcg tttagtgaac 1020
   cgtcagatcg cctggagacg ccatccacgc tgttttgacc tccatagaag acaccgggac 1080
   cgatccagcc tccgcggccg ggaacggtgc attggaacgc ggattccccg tgccaagagt 1140
   gacgtaagta ccgcctatag actctatagg cacacccctt tggctcttat gcatgctata 1200
   ctgtttttgg cttggggcct atacaccccc gctccttatg ctataggtga tggtatagct 1260
   tagcctatag gtgtgggtta ttgaccatta ttgaccactc ccctattggt gacgatactt 1320
   tccattacta atccataaca tggctctttg ccacaactat ctctattggc tatatgccaa 1380
   tactctgtcc ttcagagact gacacggact ctgtattttt acaggatggg gtccatttat 1440
   tatttacaaa ttcacatata caacaacgcc gtcccccgtg cccgcagttt ttattaaaca 1500
   tagcgtggga tctccgacat ctcgggtacg tgttccggac atgggctctt ctccggtagc 1560
   ggcggagctt ccacatccga gccctggtcc catccgtcca gcggctcatg gtcgctcggc 1620
   agctccttgc tcctaacagt ggaggccaga cttaggcaca gcacaatgcc caccaccacc 1680
   agtgtgccgc acaaggccgt ggcggtaggg tatgtgtctg aaaatgagct cggagattgg 1740
   gctcgcacct ggacgcagat ggaagactta aggcagcggc agaagaagat gcaggcagct 1800
   gagttgttgt attctgataa gagtcagagg taactcccgt tgcggtgctg ttaacggtgg 1860
   agggcagtgt agtctgagca gtactcgttg ctgccgcgcg cgccaccaga cataatagct 1920
   gacagactaa cagactgttc ctttccatgg gtcttttctg cagtcaccgt cgtcgaccta 1980
   agaattc atg cag tgg aac tcc act gcc ttc cac caa act ctg cag gat    2029
           Met Gln Trp Asn Ser Thr Ala Phe His Gln Thr Leu Gln Asp
            1               5                  10
   ccc aga gtc agg ggt ctg tat ctt cct gct ggt ggc tcc agt tca gga    2077
   Pro Arg Val Arg Gly Leu Tyr Leu Pro Ala Gly Gly Ser Ser Ser Gly
    15                  20                  25                  30
   aca gta aac cct gct ccg aat att gcc tct cac atc tcg tca atc tcc    2125
   Thr Val Asn Pro Ala Pro Asn Ile Ala Ser His Ile Ser Ser Ile Ser
                    35                  40                  45
   gcg agg act ggg gac cct gtg acg aac atg gag aac atc aca tca gga    2173
   Ala Arg Thr Gly Asp Pro Val Thr Asn Met Glu Asn Ile Thr Ser Gly
                50                  55                  60
   ttc cta gga ccc ctg ctc gtg tta cag gcg ggg ttt ttc ttg ttg aca    2221
   Phe Leu Gly Pro Leu Leu Val Leu Gln Ala Gly Phe Phe Leu Leu Thr
            65                  70                  75
   aga atc ctc aca ata ccg cag agt cta gac tcg tgg tgg act tct ctc    2269
   Arg Ile Leu Thr Ile Pro Gln Ser Leu Asp Ser Trp Trp Thr Ser Leu
        80                  85                  90
   aat ttt cta ggg gga tct ccc gtg tgt ctt ggc caa aat tcg cag tcc    2317
   Asn Phe Leu Gly Gly Ser Pro Val Cys Leu Gly Gln Asn Ser Gln Ser
    95                 100                 105                 110
   cca acc tcc aat cac tca cca acc tcc tgt cct cca att tgt cct ggt    2365
   Pro Thr Ser Asn His Ser Pro Thr Ser Cys Pro Pro Ile Cys Pro Gly
                   115                 120                 125
   tat cgc tgg atg tgt ctg cgg cgt ttt atc ata ttc ctc ttc atc ctg    2413
   Tyr Arg Trp Met Cys Leu Arg Arg Phe Ile Ile Phe Leu Phe Ile Leu
               130                 135                 140
   ctg cta tgc ctc atc ttc tta ttg gtt ctt ctg gat tat caa ggt atg    2461
   Leu Leu Cys Leu Ile Phe Leu Leu Val Leu Leu Asp Tyr Gln Gly Met
           145                 150                 155
   ttg ccc gtt tgt cct cta att cca gga tca aca aca acc agt acg gga    2509
   Leu Pro Val Cys Pro Leu Ile Pro Gly Ser Thr Thr Thr Ser Thr Gly
       160                 165                 170
   cca tgc aaa acc tgc acg act cct gct caa ggc aac tct atg ttt ccc    2557
   Pro Cys Lys Thr Cys Thr Thr Pro Ala Gln Gly Asn Ser Met Phe Pro
   175 180 185 190
   tca tgt tgc tgt aca aaa cct acg gat gga aat tgc acc tgt att ccc 2605
   Ser Cys Cys Cys Thr Lys Pro Thr Asp Gly Asn Cys Thr Cys Ile Pro
   195 200 205
   atc cca tcg tcc tgg gct ttc gca aaa tac cta tgg gag tgg gcc tca 2653
   Ile Pro Ser Ser Trp Ala Phe Ala Lys Tyr Leu Trp Glu Trp Ala Ser
   210 215 220
   gtc cgt ttc tct tgg ctc agt tta cta gtg cca ttt gtt cag tgg ttc 2701
   Val Arg Phe Ser Trp Leu Ser Leu Leu Val Pro Phe Val Gln Trp Phe
   225 230 235
   gta ggg ctt tcc ccc act gtt tgg ctt tca gct ata tgg atg atg tgg 2749
   Val Gly Leu Ser Pro Thr Val Trp Leu Ser Ala Ile Trp Met Met Trp
   240 245 250
   tat tgg ggg cca agt ctg tac agc atc gtg agt ccc ttt ata ccg ctg 2797
   Tyr Trp Gly Pro Ser Leu Tyr Ser Ile Val Ser Pro Phe Ile Pro Leu
   255 260 265 270
   tta cca att ttc ttt tgt ctc tgg gta tac att taagaattca gactcgagca 2850
   Leu Pro Ile Phe Phe Cys Leu Trp Val Tyr Ile
   275 280
   agtctagaaa ggcgcgccaa gatatcaagg atccactacg cgttagagct cgctgatcag 2910
   cctcgactgt gccttctagt tgccagccat ctgttgtttg cccctccccc gtgccttcct 2970
   tgaccctgga aggtgccact cccactgtcc tttcctaata aaatgaggaa attgcatcgc 3030
   attgtctgag taggtgtcat tctattctgg ggggtggggt ggggcaggac agcaaggggg 3090
   aggattggga agacaatagc aggcatgctg gggagctctt ccgcttcctc gctcactgac 3150
   tcgctgcgct cggtcgttcg gctgcggcga gcggtatcag ctcactcaaa ggcggtaata 3210
   cggttatcca cagaatcagg ggataacgca ggaaagaaca tgtgagcaaa aggccagcaa 3270
   aaggccagga accgtaaaaa ggccgcgttg ctggcgtttt tccataggct ccgcccccct 3330
   gacgagcatc acaaaaatcg acgctcaagt cagaggtggc gaaacccgac aggactataa 3390
   agataccagg cgtttccccc tggaagctcc ctcgtgcgct ctcctgttcc gaccctgccg 3450
   cttaccggat acctgtccgc ctttctccct tcgggaagcg tggcgctttc tcaatgctca 3510
   cgctgtaggt atctcagttc ggtgtaggtc gttcgctcca agctgggctg tgtgcacgaa 3570
   ccccccgttc agcccgaccg ctgcgcctta tccggtaact atcgtcttga gtccaacccg 3630
   gtaagacacg acttatcgcc actggcagca gccactggta acaggattag cagagcgagg 3690
   tatgtaggcg gtgctacaga gttcttgaag tggtggccta actacggcta cactagaagg 3750
   acagtatttg gtatctgcgc tctgctgaag ccagttacct tcggaaaaag agttggtagc 3810
   tcttgatccg gcaaacaaac caccgctggt agcggtggtt tttttgtttg caagcagcag 3870
   attacgcgca gaaaaaaagg atctcaagaa gatcctttga tcttttctac ggggtctgac 3930
   gctcagtgga acgaaaactc acgttaaggg attttggtca tgagattatc aaaaaggatc 3990
   ttcacctaga tccttttaaa ttaaaaatga agttttaaat caatctaaag tatatatgag 4050
   taaacttggt ctgacagtta ccaatgctta atcagtgagg cacctatctc agcgatctgt 4110
   ctatttcgtt catccatagt tgcctgactc cccgtcgtgt agataactac gatacgggag 4170
   ggcttaccat ctggccccag tgctgcaatg ataccgcgag acccacgctc accggctcca 4230
   gatttatcag caataaacca gccagccgga agggccgagc gcagaagtgg tcctgcaact 4290
   ttatccgcct ccatccagtc tattaattgt tgccgggaag ctagagtaag tagttcgcca 4350
   gttaatagtt tgcgcaacgt tgttgccatt gctacaggca tcgtggtgtc acgctcgtcg 4410
   tttggtatgg cttcattcag ctccggttcc caacgatcaa ggcgagttac atgatccccc 4470
   atgttgtgca aaaaagcggt tagctccttc ggtcctccga tcgttgtcag aagtaagttg 4530
   gccgcagtgt tatcactcat ggttatggca gcactgcata attctcttac tgtcatgcca 4590
   tccgtaagat gcttttctgt gactggtgag tactcaacca agtcattctg agaatagtgt 4650
   atgcggcgac cgagttgctc ttgcccggcg tcaatacggg ataataccgc gccacatagc 4710
   agaactttaa aagtgctcat cattggaaaa cgttcttcgg ggcgaaaact ctcaaggatc 4770
   ttaccgctgt tgagatccag ttcgatgtaa cccactcgtg cacccaactg atcttcagca 4830
   tcttttactt tcaccagcgt ttctgggtga gcaaaaacag gaaggcaaaa tgccgcaaaa 4890
   aagggaataa gggcgacacg gaaatgttga atactcatac tcttcctttt tcaatattat 4950
   tgaagcattt atcagggtta ttgtctcatg agcggataca tatttgaatg tatttagaaa 5010
   aataaacaaa taggggttcc gcgcacattt ccccgaaaag tgccacctga cgtctaagaa 5070
   accattatta tcatgacatt aacctataaa aataggcgta tcacgaggcc ctttcgtc 5128
<210> 3
   <211> 281
   <212> PRT.
   <213> Artificial Sequence
<220>
   <223> Description of Artificial Sequence: plasmid pCMVII-pS2-SAg
<400> 3
   Met Gln Trp Asn Ser Thr Ala Phe His Gln Thr Leu Gln Asp Pro Arg
   1               5                   10                  15
   Val Arg Gly Leu Tyr Leu Pro Ala Gly Gly Ser Ser Ser Gly Thr Val
               20                  25                  30
   Asn Pro Ala Pro Asn Ile Ala Ser His Ile Ser Ser Ile Ser Ala Arg
           35                  40                  45
   Thr Gly Asp Pro Val Thr Asn Met Glu Asn Ile Thr Ser Gly Phe Leu
       50                  55                  60
   Gly Pro Leu Leu Val Leu Gln Ala Gly Phe Phe Leu Leu Thr Arg Ile
   65                  70                  75                  80
   Leu Thr Ile Pro Gln Ser Leu Asp Ser Trp Trp Thr Ser Leu Asn Phe
                   85                  90                  95
   Leu Gly Gly Ser Pro Val Cys Leu Gly Gln Asn Ser Gln Ser Pro Thr
               100                 105                 110
   Ser Asn His Ser Pro Thr Ser Cys Pro Pro Ile Cys Pro Gly Tyr Arg
           115                 120                 125
   Trp Met Cys Leu Arg Arg Phe Ile Ile Phe Leu Phe Ile Leu Leu Leu
       130                 135                 140
   Cys Leu Ile Phe Leu Leu Val Leu Leu Asp Tyr Gln Gly Met Leu Pro
   145                 150                 155                 160
   Val Cys Pro Leu Ile Pro Gly Ser Thr Thr Thr Ser Thr Gly Pro Cys
                   165                 170                 175
   Lys Thr Cys Thr Thr Pro Ala Gln Gly Asn Ser Met Phe Pro Ser Cys
               180                 185                 190
   Cys Cys Thr Lys Pro Thr Asp Gly Asn Cys Thr Cys Ile Pro Ile Pro
           195                 200                 205
   Ser Ser Trp Ala Phe Ala Lys Tyr Leu Trp Glu Trp Ala Ser Val Arg
       210                 215                 220
   Phe Ser Trp Leu Ser Leu Leu Val Pro Phe Val Gln Trp Phe Val Gly
   225                 230                 235                 240
   Leu Ser Pro Thr Val Trp Leu Ser Ala Ile Trp Met Met Trp Tyr Trp
                   245                 250                 255
   Gly Pro Ser Leu Tyr Ser Ile Val Ser Pro Phe Ile Pro Leu Leu Pro
               260                 265                 270
   Ile Phe Phe Cys Leu Trp Val Tyr Ile
           275                 280
<210> 4
   <211> 5459
   <212> DNA
   <213> Artificial Sequence
<220>
   <223> Description of Artificial Sequence: pCMVII opti 330 E1/SAg
<220>
   <221> CDS
   <222> (1992) .. (3161)
<400> 4
   tcgcgcgttt cggtgatgac ggtgaaaacc tctgacacat gcagctcccg gagacggtca 60
   cagcttgtct gtaagcggat gccgggagca gacaagcccg tcagggcgcg tcagcgggtg 120
   ttggcgggtg tcggggctgg cttaactatg cggcatcaga gcagattgta ctgagagtgc 180
   accatatgaa gctttttgca aaagcctagg cctccaaaaa agcctcctca ctacttctgg 240
   aatagctcag aggccgaggc ggcctcggcc tctgcataaa taaaaaaaat tagtcagcca 300
   tggggcggag aatgggcgga actgggcggg gagggaatta ttggctattg gccattgcat 360
   acgttgtatc tatatcataa tatgtacatt tatattggct catgtccaat atgaccgcca 420
   tgttgacatt gattattgac tagttattaa tagtaatcaa ttacggggtc attagttcat 480
   agcccatata tggagttccg cgttacataa cttacggtaa atggcccgcc tggctgaccg 540
   cccaacgacc cccgcccatt gacgtcaata atgacgtatg ttcccatagt aacgccaata 600
   gggactttcc attgacgtca atgggtggag tatttacggt aaactgccca cttggcagta 660
   catcaagtgt atcatatgcc aagtccgccc cctattgacg tcaatgacgg taaatggccc 720
   gcctggcatt atgcccagta catgacctta cgggactttc ctacttggca gtacatctac 780
   gtattagtca tcgctattac catggtgatg cggttttggc agtacaccaa tgggcgtgga 840
   tagcggtttg actcacgggg atttccaagt ctccacccca ttgacgtcaa tgggagtttg 900
   ttttggcacc aaaatcaacg ggactttcca aaatgtcgta ataaccccgc cccgttgacg 960
   caaatgggcg gtaggcgtgt acggtgggag gtctatataa gcagagctcg tttagtgaac 1020
   cgtcagatcg cctggagacg ccatccacgc tgttttgacc tccatagaag acaccgggac 1080
   cgatccagcc tccgcggccg ggaacggtgc attggaacgc ggattccccg tgccaagagt 1140
   gacgtaagta ccgcctatag actctatagg cacacccctt tggctcttat gcatgctata 1200
   ctgtttttgg cttggggcct atacaccccc gctccttatg ctataggtga tggtatagct 1260
   tagcctatag gtgtgggtta ttgaccatta ttgaccactc ccctattggt gacgatactt 1320
   tccattacta atccataaca tggctctttg ccacaactat ctctattggc tatatgccaa 1380
   tactctgtcc ttcagagact gacacggact ctgtattttt acaggatggg gtccatttat 1440
   tatttacaaa ttcacatata caacaacgcc gtcccccgtg cccgcagttt ttattaaaca 1500
   tagcgtggga tctccgacat ctcgggtacg tgttccggac atgggctctt ctccggtagc 1560
   ggcggagctt ccacatccga gccctggtcc catccgtcca gcggctcatg gtcgctcggc 1620
   agctccttgc tcctaacagt ggaggccaga cttaggcaca gcacaatgcc caccaccacc 1680
   agtgtgccgc acaaggccgt ggcggtaggg tatgtgtctg aaaatgagct cggagattgg 1740
   gctcgcacct ggacgcagat ggaagactta aggcagcggc agaagaagat gcaggcagct 1800
   gagttgttgt attctgataa gagtcagagg taactcccgt tgcggtgctg ttaacggtgg 1860
   agggcagtgt agtctgagca gtactcgttg ctgccgcgcg cgccaccaga cataatagct 1920
   gacagactaa cagactgttc ctttccatgg gtcttttctg cagtcaccgt cgtcgacgaa 1980
   ttcaagcaat c atg gat gca atg aag aga ggg ctc tgc tgt gtg ctg ctg  2030
                Met Asp Ala Met Lys Arg Gly Leu Cys Cys Val Leu Leu
                 1               5                  10
   ctg tgt gga gca gtc ttc gtt tcg ccc agc gct agc tac cag gtg cgc   2078
   Leu Cys Gly Ala Val Phe Val Ser Pro Ser Ala Ser Tyr Gln Val Arg
       15                  20                  25
   aac agc acc ggc ctg tac cac gtg acc aac gac tgc ccc aac agc agc   2126
   Asn Ser Thr Gly Leu Tyr His Val Thr Asn Asp Cys Pro Asn Ser Ser
    30                  35                  40                  45
   atc gtg tac gag gcc gcc gac gcc atc ctg cac acc ccc ggc tgc gtg   2174
   Ile Val Tyr Glu Ala Ala Asp Ala Ile Leu His Thr Pro Gly Cys Val
                    50                  55                  60
   ccc tgc gtg cgc gag ggc aac gcc agc cgc tgc tgg gtg gcc atg acc   2222
   Pro Cys Val Arg Glu Gly Asn Ala Ser Arg Cys Trp Val Ala Met Thr
                65                  70                  75
   ccc acc gtg gcc acc cgc gac ggc aag ctg ccc gcc acc cag ctg cgc   2270
   Pro Thr Val Ala Thr Arg Asp Gly Lys Leu Pro Ala Thr Gln Leu Arg
            80                  85                  90
   cgc cac atc gac ctg ctg gtg ggc agc gcc acc ctg tgc agc gcc ctg   2318
   Arg His Ile Asp Leu Leu Val Gly Ser Ala Thr Leu Cys Ser Ala Leu
        95                 100                 105
   tac gtg ggc gac ctg tgc ggc agc gtg ttc ctg gtg ggc cag ctg ttc   2366
   Tyr Val Gly Asp Leu Cys Gly Ser Val Phe Leu Val Gly Gln Leu Phe
   110                 115                 120                 125
   acc ttc agc ccc cgc cgc cac tgg acc acc cag ggc tgc aac tgc agc   2414
   Thr Phe Ser Pro Arg Arg His Trp Thr Thr Gln Gly Cys Asn Cys Ser
                   130                 135                 140
   atc tac ccc ggc cac atc acc ggc cac cgc atg gcc tgg gac atg atg   2462
   Ile Tyr Pro Gly His Ile Thr Gly His Arg Met Ala Trp Asp Met Met
               145                 150                 155
   atg aac tgg agc ccc acc acc atg gag aac atc aca tca gga ttc cta   2510
   Met Asn Trp Ser Pro Thr Thr Met Glu Asn Ile Thr Ser Gly Phe Leu
           160                 165                 170
   gga ccc ctg ctc gtg tta cag gcg ggg ttt ttc ttg ttg aca aga atc   2558
   Gly Pro Leu Leu Val Leu Gln Ala Gly Phe Phe Leu Leu Thr Arg Ile
       175                 180                 185
   ctc aca ata ccg cag agt cta gac tcg tgg tgg act tct ctc aat ttt   2606
   Leu Thr Ile Pro Gln Ser Leu Asp Ser Trp Trp Thr Ser Leu Asn Phe
   190                 195                 200                 205
   cta ggg gga tct ccc gtg tgt ctt ggc caa aat tcg cag tcc cca acc   2654
   Leu Gly Gly Ser Pro Val Cys Leu Gly Gln Asn Ser Gln Ser Pro Thr
                   210                 215                 220
   tcc aat cac tca cca acc tcc tgt cct cca att tgt cct ggt tat cgc   2702
   Ser Asn His Ser Pro Thr Ser Cys Pro Pro Ile Cys Pro Gly Tyr Arg
               225                 230                 235
   tgg atg tgt ctg cgg cgt ttt atc ata ttc ctc ttc atc ctg ctg cta   2750
   Trp Met Cys Leu Arg Arg Phe Ile Ile Phe Leu Phe Ile Leu Leu Leu
           240                 245                 250
   tgc ctc atc ttc tta ttg gtt ctt ctg gat tat caa ggt atg ttg ccc   2798
   Cys Leu Ile Phe Leu Leu Val Leu Leu Asp Tyr Gln Gly Met Leu Pro
       255                 260                 265
   gtt tgt cct cta att cca gga tca aca aca acc agt acg gga cca tgc   2846
   Val Cys Pro Leu Ile Pro Gly Ser Thr Thr Thr Ser Thr Gly Pro Cys
   270                 275                 280                 285
   aaa acc tgc acg act cct gct caa ggc aac tct atg ttt ccc tca tgt   2894
   Lys Thr Cys Thr Thr Pro Ala Gln Gly Asn Ser Met Phe Pro Ser Cys
                   290                 295                 300
   tgc tgt aca aaa cct acg gat gga aat tgc acc tgt att ccc atc cca   2942
   Cys Cys Thr Lys Pro Thr Asp Gly Asn Cys Thr Cys Ile Pro Ile Pro
               305                 310                 315
   tcg tcc tgg gct ttc gca aaa tac cta tgg gag tgg gcc tca gtc cgt   2990
   Ser Ser Trp Ala Phe Ala Lys Tyr Leu Trp Glu Trp Ala Ser Val Arg
           320                 325                 330
   ttc tct tgg ctc agt tta cta gtg cca ttt gtt cag tgg ttc gta ggg   3038
   Phe Ser Trp Leu Ser Leu Leu Val Pro Phe Val Gln Trp Phe Val Gly
       335                 340                 345
   ctt tcc ccc act gtt tgg ctt tca gct ata tgg atg atg tgg tat tgg   3086
   Leu Ser Pro Thr Val Trp Leu Ser Ala Ile Trp Met Met Trp Tyr Trp
   350                 355                 360                 365
   ggg cca agt ctg tac agc atc gtg agt ccc ttt ata ccg ctg tta cca   3134
   Gly Pro Ser Leu Tyr Ser Ile Val Ser Pro Phe Ile Pro Leu Leu Pro
                   370                 375                 380
   att ttc ttt tgt ctc tgg gta tac att taagaattca gactcgagca         3181
   Ile Phe Phe Cys Leu Trp Val Tyr Ile
               385                 390
   agtctagaaa ggcgcgccaa gatatcaagg atccactacg cgttagagct cgctgatcag 3241
   cctcgactgt gccttctagt tgccagccat ctgttgtttg cccctccccc gtgccttcct 3301
   tgaccctgga aggtgccact cccactgtcc tttcctaata aaatgaggaa attgcatcgc 3361
   attgtctgag taggtgtcat tctattctgg ggggtggggt ggggcaggac agcaaggggg 3421
   aggattggga agacaatagc aggcatgctg gggagctctt ccgcttcctc gctcactgac 3481
   tcgctgcgct cggtcgttcg gctgcggcga gcggtatcag ctcactcaaa ggcggtaata 3541
   cggttatcca cagaatcagg ggataacgca ggaaagaaca tgtgagcaaa aggccagcaa 3601
   aaggccagga accgtaaaaa ggccgcgttg ctggcgtttt tccataggct ccgcccccct 3661
   gacgagcatc acaaaaatcg acgctcaagt cagaggtggc gaaacccgac aggactataa 3721
   agataccagg cgtttccccc tggaagctcc ctcgtgcgct ctcctgttcc gaccctgccg 3781
   cttaccggat acctgtccgc ctttctccct tcgggaagcg tggcgctttc tcaatgctca 3841
   cgctgtaggt atctcagttc ggtgtaggtc gttcgctcca agctgggctg tgtgcacgaa 3901
   ccccccgttc agcccgaccg ctgcgcctta tccggtaact atcgtcttga gtccaacccg 3961
   gtaagacacg acttatcgcc actggcagca gccactggta acaggattag cagagcgagg 4021
   tatgtaggcg gtgctacaga gttcttgaag tggtggccta actacggcta cactagaagg 4081
   acagtatttg gtatctgcgc tctgctgaag ccagttacct tcggaaaaag agttggtagc 4141
   tcttgatccg gcaaacaaac caccgctggt agcggtggtt tttttgtttg caagcagcag 4201
   attacgcgca gaaaaaaagg atctcaagaa gatcctttga tcttttctac ggggtctgac 4261
   gctcagtgga acgaaaactc acgttaaggg attttggtca tgagattatc aaaaaggatc 4321
   ttcacctaga tccttttaaa ttaaaaatga agttttaaat caatctaaag tatatatgag 4381
   taaacttggt ctgacagtta ccaatgctta atcagtgagg cacctatctc agcgatctgt 4441
   ctatttcgtt catccatagt tgcctgactc cccgtcgtgt agataactac gatacgggag 4501
   ggcttaccat ctggccccag tgctgcaatg ataccgcgag acccacgctc accggctcca 4561
   gatttatcag caataaacca gccagccgga agggccgagc gcagaagtgg tcctgcaact 4621
   ttatccgcct ccatccagtc tattaattgt tgccgggaag ctagagtaag tagttcgcca 4681
   gttaatagtt tgcgcaacgt tgttgccatt gctacaggca tcgtggtgtc acgctcgtcg 4741
   tttggtatgg cttcattcag ctccggttcc caacgatcaa ggcgagttac atgatccccc 4801
   atgttgtgca aaaaagcggt tagctccttc ggtcctccga tcgttgtcag aagtaagttg 4861
   gccgcagtgt tatcactcat ggttatggca gcactgcata attctcttac tgtcatgcca 4921
   tccgtaagat gcttttctgt gactggtgag tactcaacca agtcattctg agaatagtgt 4981
   atgcggcgac cgagttgctc ttgcccggcg tcaatacggg ataataccgc gccacatagc 5041
   agaactttaa aagtgctcat cattggaaaa cgttcttcgg ggcgaaaact ctcaaggatc 5101
   ttaccgctgt tgagatccag ttcgatgtaa cccactcgtg cacccaactg atcttcagca 5161
   tcttttactt tcaccagcgt ttctgggtga gcaaaaacag gaaggcaaaa tgccgcaaaa 5221
   aagggaataa gggcgacacg gaaatgttga atactcatac tcttcctttt tcaatattat 5281
   tgaagcattt atcagggtta ttgtctcatg agcggataca tatttgaatg tatttagaaa 5341
   aataaacaaa taggggttcc gcgcacattt ccccgaaaag tgccacctga cgtctaagaa 5401
   accattatta tcatgacatt aacctataaa aataggcgta tcacgaggcc ctttcgtc 5459
<210> 5
   <211> 390
   <212> PRT
   <213> Artificial Sequence
<220>
   <223> Description of Artificial Sequence: pCMVII opti 330 E1/SAg
<400> 5
   Met Asp Ala Met Lys Arg Gly Leu Cys Cys Val Leu Leu Leu Cys Gly
   1               5                   10                  15
   Ala Val Phe Val Ser Pro Ser Ala Ser Tyr Gln Val Arg Asn Ser Thr
               20                  25                  30
   Gly Leu Tyr His Val Thr Asn Asp Cys Pro Asn Ser Ser Ile Val Tyr
           35                  40                  45
   Glu Ala Ala Asp Ala Ile Leu His Thr Pro Gly Cys Val Pro Cys Val
       50                  55                  60
   Arg Glu Gly Asn Ala Ser Arg Cys Trp Val Ala Met Thr Pro Thr Val
   65                  70                  75                  80
   Ala Thr Arg Asp Gly Lys Leu Pro Ala Thr Gln Leu Arg Arg His Ile
                   85                  90                  95
   Asp Leu Leu Val Gly Ser Ala Thr Leu Cys Ser Ala Leu Tyr Val Gly
               100                 105                 110
   Asp Leu Cys Gly Ser Val Phe Leu Val Gly Gln Leu Phe Thr Phe Ser
           115                 120                 125
   Pro Arg Arg His Trp Thr Thr Gln Gly Cys Asn Cys Ser Ile Tyr Pro
       130                 135                 140
   Gly His Ile Thr Gly His Arg Met Ala Trp Asp Met Met Met Asn Trp
   145                 150                 155                 160
   Ser Pro Thr Thr Met Glu Asn Ile Thr Ser Gly Phe Leu Gly Pro Leu
                   165                 170                 175
   Leu Val Leu Gln Ala Gly Phe Phe Leu Leu Thr Arg Ile Leu Thr Ile
               180                 185                 190
   Pro Gln Ser Leu Asp Ser Trp Trp Thr Ser Leu Asn Phe Leu Gly Gly
           195                 200                 205
   Ser Pro Val Cys Leu Gly Gln Asn Ser Gln Ser Pro Thr Ser Asn His
           210                 215                 220
   Ser Pro Thr Ser Cys Pro Pro Ile Cys Pro Gly Tyr Arg Trp Met Cys
   225                 230                 235                 240
   Leu Arg Arg Phe Ile Ile Phe Leu Phe Ile Leu Leu Leu Cys Leu Ile
                   245                 250                 255
   Phe Leu Leu Val Leu Leu Asp Tyr Gln Gly Met Leu Pro Val Cys Pro
               260                 265                 270
   Leu Ile Pro Gly Ser Thr Thr Thr Ser Thr Gly Pro Cys Lys Thr Cys
           275                 280                 285
   Thr Thr Pro Ala Gln Gly Asn Ser Met Phe Pro Ser Cys Cys Cys Thr
       290                 295                 300
   Lys Pro Thr Asp Gly Asn Cys Thr Cys Ile Pro Ile Pro Ser Ser Trp
   305                 310                 315                 320
   Ala Phe Ala Lys Tyr Leu Trp Glu Trp Ala Ser Val Arg Phe Ser Trp
                   325                 330                 335
   Leu Ser Leu Leu Val Pro Phe Val Gln Trp Phe Val Gly Leu Ser Pro
               340                 345                 350
   Thr Val Trp Leu Ser Ala Ile Trp Met Met Trp Tyr Trp Gly Pro Ser
           355                 360                 365
   Leu Tyr Ser Ile Val Ser Pro Phe Ile Pro Leu Leu Pro Ile Phe Phe
       370                 375                 380
   Cys Leu Trp Val Tyr Ile
   385                 390
<210> 6
   <211> 5882
   <212> DNA
   <213> Artificial Sequence
<220>
   <223> Description of Artificial Sequence: plasmid pCMV-II-E2661-sAg
<220>
   <221> CDS
   <222> (1992)..(3584)
<400> 6
   tcgcgcgttt cggtgatgac ggtgaaaacc tctgacacat gcagctcccg gagacggtca 60
   cagcttgtct gtaagcggat gccgggagca gacaagcccg tcagggcgcg tcagcgggtg 120
   ttggcgggtg tcggggctgg cttaactatg cggcatcaga gcagattgta ctgagagtgc 180
   accatatgaa gctttttgca aaagcctagg cctccaaaaa agcctcctca ctacttctgg 240
   aatagctcag aggccgaggc ggcctcggcc tctgcataaa taaaaaaaat tagtcagcca 300
   tggggcggag aatgggcgga actgggcggg gagggaatta ttggctattg gccattgcat 360
   acgttgtatc tatatcataa tatgtacatt tatattggct catgtccaat atgaccgcca 420
   tgttgacatt gattattgac tagttattaa tagtaatcaa ttacggggtc attagttcat 480
   agcccatata tggagttccg cgttacataa cttacggtaa atggcccgcc tggctgaccg 540
   cccaacgacc cccgcccatt gacgtcaata atgacgtatg ttcccatagt aacgccaata 600
   gggactttcc attgacgtca atgggtggag tatttacggt aaactgccca cttggcagta 660
   catcaagtgt atcatatgcc aagtccgccc cctattgacg tcaatgacgg taaatggccc 720
   gcctggcatt atgcccagta catgacctta cgggactttc ctacttggca gtacatctac 780
   gtattagtca tcgctattac catggtgatg cggttttggc agtacaccaa tgggcgtgga 840
   tagcggtttg actcacgggg atttccaagt ctccacccca ttgacgtcaa tgggagtttg 900
   ttttggcacc aaaatcaacg ggactttcca aaatgtcgta ataaccccgc cccgttgacg 960
   caaatgggcg gtaggcgtgt acggtgggag gtctatataa gcagagctcg tttagtgaac 1020
   cgtcagatcg cctggagacg ccatccacgc tgttttgacc tccatagaag acaccgggac 1080
   cgatccagcc tccgcggccg ggaacggtgc attggaacgc ggattccccg tgccaagagt 1140
   gacgtaagta ccgcctatag actctatagg cacacccctt tggctcttat gcatgctata 1200
   ctgtttttgg cttggggcct atacaccccc gctccttatg ctataggtga tggtatagct 1260
   tagcctatag gtgtgggtta ttgaccatta ttgaccactc ccctattggt gacgatactt 1320
   tccattacta atccataaca tggctctttg ccacaactat ctctattggc tatatgccaa 1380
   tactctgtcc ttcagagact gacacggact ctgtattttt acaggatggg gtccatttat 1440
   tatttacaaa ttcacatata caacaacgcc gtcccccgtg cccgcagttt ttattaaaca 1500
   tagcgtggga tctccgacat ctcgggtacg tgttccggac atgggctctt ctccggtagc 1560
   ggcggagctt ccacatccga gccctggtcc catccgtcca gcggctcatg gtcgctcggc 1620
   agctccttgc tcctaacagt ggaggccaga cttaggcaca gcacaatgcc caccaccacc 1680
   agtgtgccgc acaaggccgt ggcggtaggg tatgtgtctg aaaatgagct cggagattgg 1740
   gctcgcacct ggacgcagat ggaagactta aggcagcggc agaagaagat gcaggcagct 1800
   gagttgttgt attctgataa gagtcagagg taactcccgt tgcggtgctg ttaacggtgg 1860
   agggcagtgt agtctgagca gtactcgttg ctgccgcgcg cgccaccaga cataatagct 1920
   gacagactaa cagactgttc ctttccatgg gtcttttctg cagtcaccgt cgtcgacgaa 1980
   ttcaagcaat c atg gat gca atg aag aga ggg ctc tgc tgt gtg ctg ctg  2030
                Met Asp Ala Met Lys Arg Gly Leu Cys Cys Val Leu Leu
                1               5                   10
   ctg tgt gga gca gtc ttc gtt tcg ccc agc gct agc gaa acc cac gtc    2078
   Leu Cys Gly Ala Val Phe Val Ser Pro Ser Ala Ser Glu Thr His Val
        15                  20                  25
   acc ggg gga agt gcc ggc cac act gtg tct gga ttt gtt agc ctc ctc    2126
   Thr Gly Gly Ser Ala Gly His Thr Val Ser Gly Phe Val Ser Leu Leu
    30                  35                  40                  45
   gca cca ggc gcc aag cag aac gtc cag ctg atc aac acc aac ggc agt    2174
   Ala Pro Gly Ala Lys Gln Asn Val Gln Leu Ile Asn Thr Asn Gly Ser
                    50                  55                  60
   tgg cac ctc aat agc acg gcc ctg aac tgc aat gat agc ctc aac acc    2222
   Trp His Leu Asn Ser Thr Ala Leu Asn Cys Asn Asp Ser Leu Asn Thr
                65                  70                  75
   ggc tgg ttg gca ggg ctt ttc tat cac cac aag ttc aac tct tca ggc    2270
   Gly Trp Leu Ala Gly Leu Phe Tyr His His Lys Phe Asn Ser Ser Gly
            80                  85                  90
   tgt cct gag agg cta gcc agc tgc cga ccc ctt acc gat ttt gac cag    2318
   Cys Pro Glu Arg Leu Ala Ser Cys Arg Pro Leu Thr Asp Phe Asp Gln
        95                 100                 105
   ggc tgg ggc cct atc agt tat gcc aac gga agc ggc ccc gac cag cgc    2366
   Gly Trp Gly Pro Ile Ser Tyr Ala Asn Gly Ser Gly Pro Asp Gln Arg
   110                 115                 120                 125
   ccc tac tgc tgg cac tac ccc cca aaa cct tgc ggt att gtg ccc gcg    2414
   Pro Tyr Cys Trp His Tyr Pro Pro Lys Pro Cys Gly Ile Val Pro Ala
                   130                 135                 140
   aag agt gtg tgt ggt ccg gta tat tgc ttc act ccc agc ccc gtg gtg    2462
   Lys Ser Val Cys Gly Pro Val Tyr Cys Phe Thr Pro Ser Pro Val Val
               145                 150                 155
   gtg gga acg acc gac agg tcg ggc gcg ccc acc tac agc tgg ggt gaa    2510
   Val Gly Thr Thr Asp Arg Ser Gly Ala Pro Thr Tyr Ser Trp Gly Glu
           160                 165                 170
   aat gat acg gac gtc ttc gtc ctt aac aat acc agg cca ccg ctg ggc    2558
   Asn Asp Thr Asp Val Phe Val Leu Asn Asn Thr Arg Pro Pro Leu Gly
       175                 180                 185
   aat tgg ttc ggt tgt acc tgg atg aac tca act gga ttc acc aaa gtg    2606
   Asn Trp Phe Gly Cys Thr Trp Met Asn Ser Thr Gly Phe Thr Lys Val
   190                 195                 200                 205
   tgc gga gcg cct cct tgt gtc atc gga ggg gcg ggc aac aac acc ctg    2654
   Cys Gly Ala Pro Pro Cys Val Ile Gly Gly Ala Gly Asn Asn Thr Leu
                   210                 215                 220
   cac tgc ccc act gat tgc ttc cgc aag cat ccg gac gcc aca tac tct    2702
   His Cys Pro Thr Asp Cys Phe Arg Lys His Pro Asp Ala Thr Tyr Ser
               225                 230                 235
   cgg tgc ggc tcc ggt ccc tgg atc aca ccc agg tgc ctg gtc gac tac    2750
   Arg Cys Gly Ser Gly Pro Trp Ile Thr Pro Arg Cys Leu Val Asp Tyr
           240                 245                 250
   ccg tat agg ctt tgg cat tat cct tgt acc atc aac tac acc ata ttt    2798
   Pro Tyr Arg Leu Trp His Tyr Pro Cys Thr Ile Asn Tyr Thr Ile Phe
       255                 260                 265
   aaa atc agg atg tac gtg gga ggg gtc gaa cac agg ctg gaa gct gcc    2846
   Lys Ile Arg Met Tyr Val Gly Gly Val Glu His Arg Leu Glu Ala Ala
   270                 275                 280                 285
   tgc aac tgg acg cgg ggc gaa cgt tgc gat ctg gaa gat agg gac agg    2894
   Cys Asn Trp Thr Arg Gly Glu Arg Cys Asp Leu Glu Asp Arg Asp Arg
                   290                 295                 300
   tcc gag atc gat atg gag aac atc aca tca gga ttc cta gga ccc ctg    2942
   Ser Glu Ile Asp Met Glu Asn Ile Thr Ser Gly Phe Leu Gly Pro Leu
               305                 310                 315
   ctc gtg tta cag gcg ggg ttt ttc ttg ttg aca aga atc ctc aca ata    2990
   Leu Val Leu Gln Ala Gly Phe Phe Leu Leu Thr Arg Ile Leu Thr Ile
           320                 325                 330
   ccg cag agt cta gac tcg tgg tgg act tct ctc aat ttt cta ggg gga    3038
   Pro Gln Ser Leu Asp Ser Trp Trp Thr Ser Leu Asn Phe Leu Gly Gly
       335                 340                 345
   tct ccc gtg tgt ctt ggc caa aat tcg cag tcc cca acc tcc aat cac    3086
   Ser Pro Val Cys Leu Gly Gln Asn Ser Gln Ser Pro Thr Ser Asn His
   350                 355                 360                 365
   tca cca acc tcc tgt cct cca att tgt cct ggt tat cgc tgg atg tgt    3134
   Ser Pro Thr Ser Cys Pro Pro Ile Cys Pro Gly Tyr Arg Trp Met Cys
                   370                 375                 380
   ctg cgg cgt ttt atc ata ttc ctc ttc atc ctg ctg cta tgc ctc atc    3182
   Leu Arg Arg Phe Ile Ile Phe Leu Phe Ile Leu Leu Leu Cys Leu Ile
               385                 390                 395
   ttc tta ttg gtt ctt ctg gat tat caa ggt atg ttg ccc gtt tgt cct    3230
   Phe Leu Leu Val Leu Leu Asp Tyr Gln Gly Met Leu Pro Val Cys Pro
           400                 405                 410
   cta att cca gga tca aca aca acc agt acg gga cca tgc aaa acc tgc    3278
   Leu Ile Pro Gly Ser Thr Thr Thr Ser Thr Gly Pro Cys Lys Thr Cys
       415                 420                 425
   acg act cct gct caa ggc aac tct atg ttt ccc tca tgt tgc tgt aca    3326
   Thr Thr Pro Ala Gln Gly Asn Ser Met Phe Pro Ser Cys Cys Cys Thr
   430                 435                 440                 445
   aaa cct acg gat gga aat tgc acc tgt att ccc atc cca tcg tcc tgg    3374
   Lys Pro Thr Asp Gly Asn Cys Thr Cys Ile Pro Ile Pro Ser Ser Trp
                   450                 455                 460
   gct ttc gca aaa tac cta tgg gag tgg gcc tca gtc cgt ttc tct tgg    3422
   Ala Phe Ala Lys Tyr Leu Trp Glu Trp Ala Ser Val Arg Phe Ser Trp
               465                 470                 475
   ctc agt tta cta gtg cca ttt gtt cag tgg ttc gta ggg ctt tcc ccc    3470
   Leu Ser Leu Leu Val Pro Phe Val Gln Trp Phe Val Gly Leu Ser Pro
           480                 485                 490
   act gtt tgg ctt tca gct ata tgg atg atg tgg tat tgg ggg cca agt   3518
   Thr Val Trp Leu Ser Ala Ile Trp Met Met Trp Tyr Trp Gly Pro Ser
       495                 500                 505
   ctg tac agc atc gtg agt ccc ttt ata ccg ctg tta cca att ttc ttt   3566
   Leu Tyr Ser Ile Val Ser Pro Phe Ile Pro Leu Leu Pro Ile Phe Phe
   510                 515                 520                 525
   tgt ctc tgg gta tac att taagaattca gactcgagca agtctagaaa          3614
   Cys Leu Trp Val Tyr Ile
                   530
   ggcgcgccaa gatatcaagg atccactacg cgttagagct cgctgatcag cctcgactgt 3674
   gccttctagt tgccagccat ctgttgtttg cccctccccc gtgccttcct tgaccctgga 3734
   aggtgccact cccactgtcc tttcctaata aaatgaggaa attgcatcgc attgtctgag 3794
   taggtgtcat tctattctgg ggggtggggt ggggcaggac agcaaggggg aggattggga 3854
   agacaatagc aggcatgctg gggagctctt ccgcttcctc gctcactgac tcgctgcgct 3914
   cggtcgttcg gctgcggcga gcggtatcag ctcactcaaa ggcggtaata cggttatcca 3974
   cagaatcagg ggataacgca ggaaagaaca tgtgagcaaa aggccagcaa aaggccagga 4034
   accgtaaaaa ggccgcgttg ctggcgtttt tccataggct ccgcccccct gacgagcatc 4094
   acaaaaatcg acgctcaagt cagaggtggc gaaacccgac aggactataa agataccagg 4154
   cgtttccccc tggaagctcc ctcgtgcgct ctcctgttcc gaccctgccg cttaccggat 4214
   acctgtccgc ctttctccct tcgggaagcg tggcgctttc tcaatgctca cgctgtaggt 4274
   atctcagttc ggtgtaggtc gttcgctcca agctgggctg tgtgcacgaa ccccccgttc 4334
   agcccgaccg ctgcgcctta tccggtaact atcgtcttga gtccaacccg gtaagacacg 4394
   acttatcgcc actggcagca gccactggta acaggattag cagagcgagg tatgtaggcg 4454
   gtgctacaga gttcttgaag tggtggccta actacggcta cactagaagg acagtatttg 4514
   gtatctgcgc tctgctgaag ccagttacct tcggaaaaag agttggtagc tcttgatccg 4574
   gcaaacaaac caccgctggt agcggtggtt tttttgtttg caagcagcag attacgcgca 4634
   gaaaaaaagg atctcaagaa gatcctttga tcttttctac ggggtctgac gctcagtgga 4694
   acgaaaactc acgttaaggg attttggtca tgagattatc aaaaaggatc ttcacctaga 4754
   tccttttaaa ttaaaaatga agttttaaat caatctaaag tatatatgag taaacttggt 4814
   ctgacagtta ccaatgctta atcagtgagg cacctatctc agcgatctgt ctatttcgtt 4874
   catccatagt tgcctgactc cccgtcgtgt agataactac gatacgggag ggcttaccat 4934
   ctggccccag tgctgcaatg ataccgcgag acccacgctc accggctcca gatttatcag 4994
   caataaacca gccagccgga agggccgagc gcagaagtgg tcctgcaact ttatccgcct 5054
   ccatccagtc tattaattgt tgccgggaag ctagagtaag tagttcgcca gttaatagtt 5114
   tgcgcaacgt tgttgccatt gctacaggca tcgtggtgtc acgctcgtcg tttggtatgg 5174
   cttcattcag ctccggttcc caacgatcaa ggcgagttac atgatccccc atgttgtgca 5234
   aaaaagcggt tagctccttc ggtcctccga tcgttgtcag aagtaagttg gccgcagtgt 5294
   tatcactcat ggttatggca gcactgcata attctcttac tgtcatgcca tccgtaagat 5354
   gcttttctgt gactggtgag tactcaacca agtcattctg agaatagtgt atgcggcgac 5414
   cgagttgctc ttgcccggcg tcaatacggg ataataccgc gccacatagc agaactttaa 5474
   aagtgctcat cattggaaaa cgttcttcgg ggcgaaaact ctcaaggatc ttaccgctgt 5534
   tgagatccag ttcgatgtaa cccactcgtg cacccaactg atcttcagca tcttttactt 5594
   tcaccagcgt ttctgggtga gcaaaaacag gaaggcaaaa tgccgcaaaa aagggaataa 5654
   gggcgacacg gaaatgttga atactcatac tcttcctttt tcaatattat tgaagcattt 5714
   atcagggtta ttgtctcatg agcggataca tatttgaatg tatttagaaa aataaacaaa 5774
   taggggttcc gcgcacattt ccccgaaaag tgccacctga cgtctaagaa accattatta 5834
   tcatgacatt aacctataaa aataggcgta tcacgaggcc ctttcgtc 5882
<210> 7
   <211> 531
   <212> PRT
   <213> Artificial Sequence
<220>
   <223> Description of Artificial Sequence: plasmid pCMV-II-E2661-sAg
<400> 7
   Met Asp Ala Met Lys Arg Gly Leu Cys Cys Val Leu Leu Leu Cys Gly
   1 5 10 15
   Ala Val Phe Val Ser Pro Ser Ala Ser Glu Thr His Val Thr Gly Gly
   20 25 30
   Ser Ala Gly His Thr Val Ser Gly Phe Val Ser Leu Leu Ala Pro Gly
   35 40 45
   Ala Lys Gln Asn Val Gln Leu Ile Asn Thr Asn Gly Ser Trp His Leu
   50 55 60
   Asn Ser Thr Ala Leu Asn Cys Asn Asp Ser Leu Asn Thr Gly Trp Leu
   65 70 75 80
   Ala Gly Leu Phe Tyr His His Lys Phe Asn Ser Ser Gly Cys Pro Glu
   85 90 95
   Arg Leu Ala Ser Cys Arg Pro Leu Thr Asp Phe Asp Gln Gly Trp Gly
   100 105 110
   Pro Ile Ser Tyr Ala Asn Gly Ser Gly Pro Asp Gln Arg Pro Tyr Cys
   115 120 125
   Trp His Tyr Pro Pro Lys Pro Cys Gly Ile Val Pro Ala Lys Ser Val
   130 135 140
   Cys Gly Pro Val Tyr Cys Phe Thr Pro Ser Pro Val Val Val Gly Thr
   145 150 155 160
   Thr Asp Arg Ser Gly Ala Pro Thr Tyr Ser Trp Gly Glu Asn Asp Thr
   165 170 175
   Asp Val Phe Val Leu Asn Asn Thr Arg Pro Pro Leu Gly Asn Trp Phe
   180 185 190
   Gly Cys Thr Trp Met Asn Ser Thr Gly Phe Thr Lys Val Cys Gly Ala
   195 200 205
   Pro Pro Cys Val Ile Gly Gly Ala Gly Asn Asn Thr Leu His Cys Pro
   210 215 220
   Thr Asp Cys Phe Arg Lys His Pro Asp Ala Thr Tyr Ser Arg Cys Gly
   225 230 235 240
   Ser Gly Pro Trp Ile Thr Pro Arg Cys Leu Val Asp Tyr Pro Tyr Arg
   245 250 255
   Leu Trp His Tyr Pro Cys Thr Ile Asn Tyr Thr Ile Phe Lys Ile Arg
   260 265 270
   Met Tyr Val Gly Gly Val Glu His Arg Leu Glu Ala Ala Cys Asn Trp
   275 280 285
   Thr Arg Gly Glu Arg Cys Asp Leu Glu Asp Arg Asp Arg Ser Glu Ile
   290 295 300
   Asp Met Glu Asn Ile Thr Ser Gly Phe Leu Gly Pro Leu Leu Val Leu
   305 310 315 320
   Gln Ala Gly Phe Phe Leu Leu Thr Arg Ile Leu Thr Ile Pro Gln Ser
   325 330 335
   Leu Asp Ser Trp Trp Thr Ser Leu Asn Phe Leu Gly Gly Ser Pro Val
   340 345 350
   Cys Leu Gly Gln Asn Ser Gln Ser Pro Thr Ser Asn His Ser Pro Thr
   355 360 365
   Ser Cys Pro Pro Ile Cys Pro Gly Tyr Arg Trp Met Cys Leu Arg Arg
   370 375 380
   Phe Ile Ile Phe Leu Phe Ile Leu Leu Leu Cys Leu Ile Phe Leu Leu
   385 390 395 400
   Val Leu Leu Asp Tyr Gln Gly Met Leu Pro Val Cys Pro Leu Ile Pro
   405 410 415
   Gly Ser Thr Thr Thr Ser Thr Gly Pro Cys Lys Thr Cys Thr Thr Pro
   420 425 430
   Ala Gln Gly Asn Ser Met Phe Pro Ser Cys Cys Cys Thr Lys Pro Thr
   435 440 445
   Asp Gly Asn Cys Thr Cys Ile Pro Ile Pro Ser Ser Trp Ala Phe Ala
   450 455 460
   Lys Tyr Leu Trp Glu Trp Ala Ser Val Arg Phe Ser Trp Leu Ser Leu
   465 470 475 480
   Leu Val Pro Phe Val Gln Trp Phe Val Gly Leu Ser Pro Thr Val Trp
   485 490 495
   Leu Ser Ala Ile Trp Met Met Trp Tyr Trp Gly Pro Ser Leu Tyr Ser
   500 505 510
   Ile Val Ser Pro Phe Ile Pro Leu Leu Pro Ile Phe Phe Cys Leu Trp
   515 520 525
   Val Tyr Ile
   530

## Description

### TECHNICAL FIELD OF THE INVENTION

The invention is related to the area of recombinant vaccines. It is particularly related to the field of chimeric antigens and virus-like particles for use in vaccines, especially combination vaccines for Hepatitis B virus (HBV) and Hepatitis C virus (HCV).

### BACKGROUND OF THE INVENTION

Hepatitis C virus (HCV) infects approximately 1 % of the world's population and causes serious health problems. Over 75% of acutely infected individuals eventually progress to a chronic carrier state that can result in cirrhosis, liver failure, and hepatocellular carcinoma. A very small fraction of chronically infected patients clear HCV naturally and resolve chronic hepatitis. *See* Alter *et al*. (1992) N. Engl. J. Med. 327:1899-1905; Resnick and Koff. (1993) Arch. Intern. Med. 153:1672-1677; Seeff (1995) Gastrointest. Dis. 6:20-27; Tong *et al*. (1995) N. Engl. J. Med. 332:1463-1466. Immunization against E2 glycoproteins of some flaviviruses (*see e.g.,* Konishi et al., (1992) Virology 188: 714-720), including HCV (Ishii et al., (1998) Hepatology 28: 1117-1120), may protect against infection. However, attempts to express recombinant HCV E1 and E2 glyocoproteins have been frustrated by the fact that these proteins are not secreted from the host cell but are retained within the endoplasmic reticulum (Dubuisson et al. (1994) J. Virology 68: 6147-6160).

One approach to making vaccines for HCV and other viruses which has been attempted is to prepare chimeric antigens consisting of fusions of hepatitis B virus surface antigen (HBsAg) with a heterologous antigen, for example a portion of an HCV protein. *See, e.g.,* Inchauspe et al. (1998) Dev. Biol. Stand. 92: 162-168; Nakano et al. (1997) J. Virol. (1997) 71: 7101-7109; and Inchauspe et al. (1997) Vaccine 15: 853-856. The use of HBsAg is attractive for the production of immunogenic compositions such as vaccines because HBsAg is highly immunogenic and is secreted from cultured cells in the form of virus-like particles (U.S. Patent 5,098,704). Attempts to introduce small portions of viral proteins into HBsAg have succeeded in the production of virus-like particles (*see e.g*., Delpeyroux et al. (1990) J. Virology 64: 6090-6100, who inserted an 11 amino acid segment of polio virus capsid protein into HBsAg). However, in one study only two out of six fusion proteins containing HBsAg combined with different hydrophillic domains ofHCV E2 were secreted into the culture medium as virus-like particles (Lee et al. (1996) J. Med. Virol. 50: 145-151), possibly because the E2 inserts were too large or hydrophilic. The insertion site of heterologous epitopes into HBsAg may be an important factor. A study which inserted an epitope of HBV nucleocapsid (HBcAg) at various positions into HBsAg found that insertion into an internal site in HBsAg resulted in a chimeric protein that was immunogenic for HBcAg, while insertion at the C-terminus was weakly immunogenic (Schodel et al. (1992) J. Virology 66: 106-114). Insertion at the N-terminus prevented surface access of the HBcAg epitope in the resultant particles and was non-immunogenic (*Id.*). Apparently, the molecular context in which an epitope is presented is important in determining immunogenicity, probably because of subtle alterations of protein secondary and tertiary structure. This principle was further illustrated by Eckhart et al. ((1996) J. Gen. Virol. 77: 2001-2008), who introduced a conserved, six amino acid epitope of HIV-1 gp41 protein into influenza hemagglutinin and obtained neutralizing antibodies, but could not generate neutralizing antibodies when the same epitope was inserted into HBsAg. Smaller isolated epitopes are more likely to be sensitive to such effects than larger portions of an immunogenic protein.

Currently there is no method available for expressing entire E1 or E2 glycoproteins ofHCV in virus-like particles for use in immunization. Available methods limit chimeric proteins based on HBsAg to the insertion of only small isolated domains of E2, which may or may not have a native immunogenic structure. Thus, there remains a need in the art for methods and materials that can be used to express HCV antigens in an immunogenic form in virus-like particles.

### SUMMARY OF THE INVENTION

It is an object of the invention to provide HBV/HCV chimeric antigens for use in immunogenic compositions. It is a further object of the invention to provide virus-like particles comprising HBV/HCV chimeric antigens and methods and materials for producing such virus-like particles. It is another object of the invention to provide HBV/HCV combination vaccines. These and other objects of the invention are provided by one or more of the embodiments described below.

One embodiment of the invention provides a virus-like particle for use as an immunogen or as a component of a vaccine. The virus-like particle comprises a first HBsAg (hepatitis B virus surface antigen) and a chimeric antigen. The chimeric antigen comprises a second HBsAg which is linked to an HCV immunogenic polypeptide. The first and the second HBsAg each comprise a substantially complete S domain.

Another embodiment of the invention provides another virus-like particle for use as an immunogen or as a component of a vaccine. The virus-like particle comprises a first HBsAg and first and second chimeric antigens. The first chimeric antigen comprises a second HBsAg which is linked to a first immunogenic polypeptide comprising an HCV E1 glycoprotein or a fragment thereof. The second chimeric antigen comprises a third HBsAg which is linked to a second immunogenic polypeptide comprising an HCV E2 glycoprotein or a fragment thereof The first, second and third HBsAg each comprise a substantially complete S domain.

A further embodiment of the invention provides a method of producing virus-like particles. A cell is cultured in a culture medium, whereby the cell expresses virus-like particles comprising a first HBsAg and a chimeric antigen. The chimeric antigen comprises a second HBsAg which is linked to an HCV immunogenic polypeptide. The first and the second HBsAg each comprise a substantially complete S domain. The virus-like particles are then isolated from the culture medium.

Yet another embodiment is a method of producing a cell line that expresses virus-like particles. A cell is transfected with a vector that expresses virus-like particles comprising a first HBsAg and a chimeric antigen. The chimeric antigen comprises a second HBsAg which is linked to an HCV immunogenic polypeptide. The first and the second HBsAg each comprise a substantially complete S domain. The cell is cultured to produce a cell line that expresses the virus-like particles.

Still other embodiments of the invention are cell lines that express virus-like particles. In one cell line, the virus-like particles comprise a first HBsAg and a chimeric antigen. The chimeric antigen comprises a second HBsAg which is linked to an HCV immunogenic polypeptide. The first and the second HBsAg each comprise a substantially complete S domain. In another type of cell line, the virus-like particles comprise a first HBsAg and first and second chimeric antigens. The first chimeric antigen comprises a second HBsAg which is linked to a first immunogenic polypeptide comprising an HCV E1 glycoprotein or a fragment thereof, and the second chimeric antigen comprises a third HBsAg which is linked to a second immunogenic polypeptide comprising an HCV E2 glycoprotein or a fragment thereof. The first, second, and third HBsAg each comprise a substantially complete S domain.

The invention thus provides the art with novel methods and materials for the production of HBV/HCV combination vaccines.

### BRIEF DESCRIPTION OF THE DRAWINGS

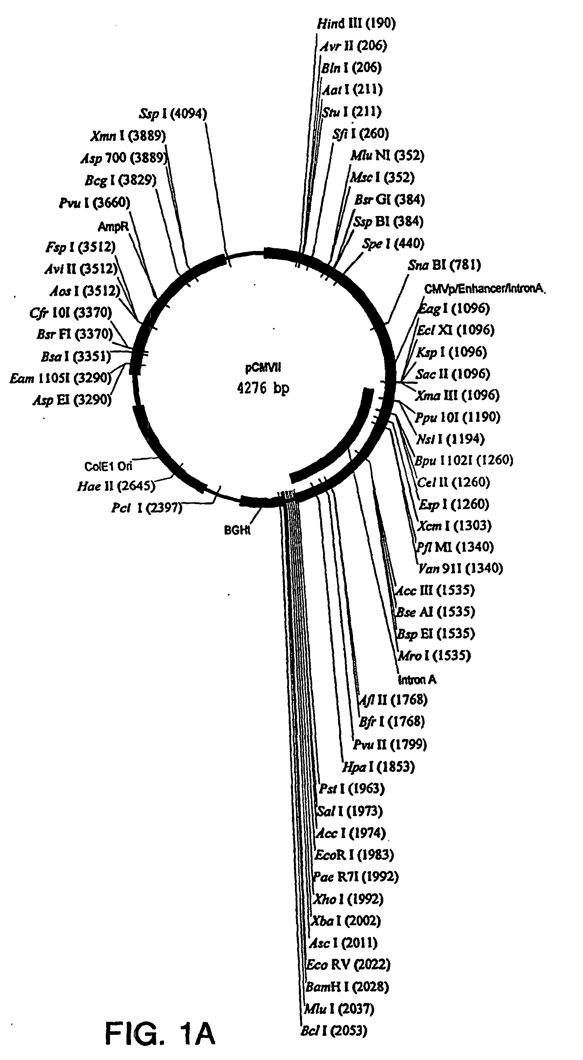

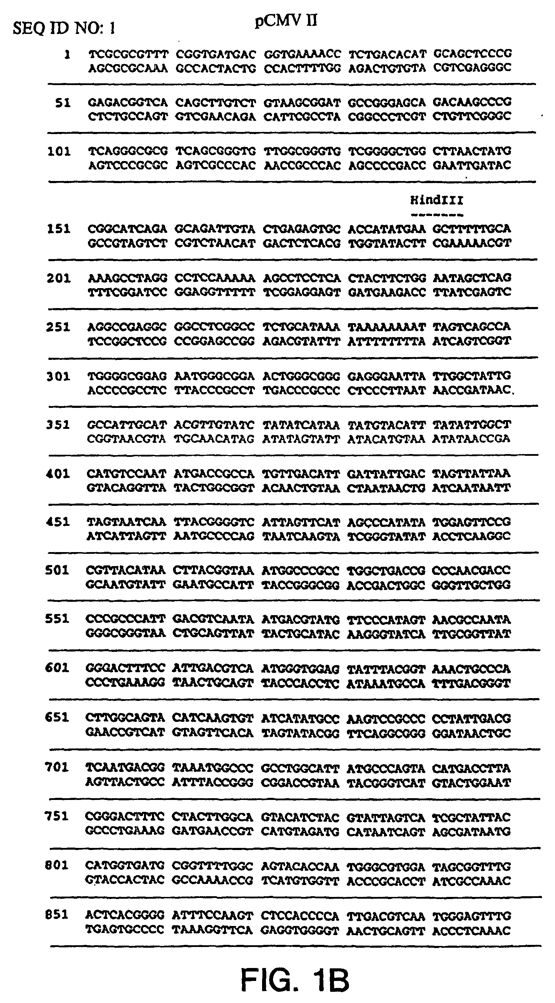

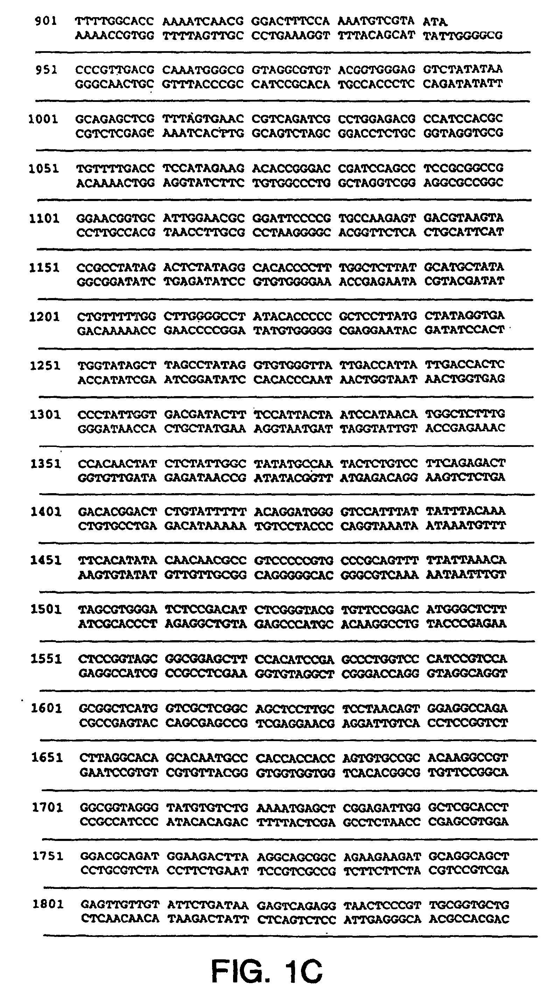

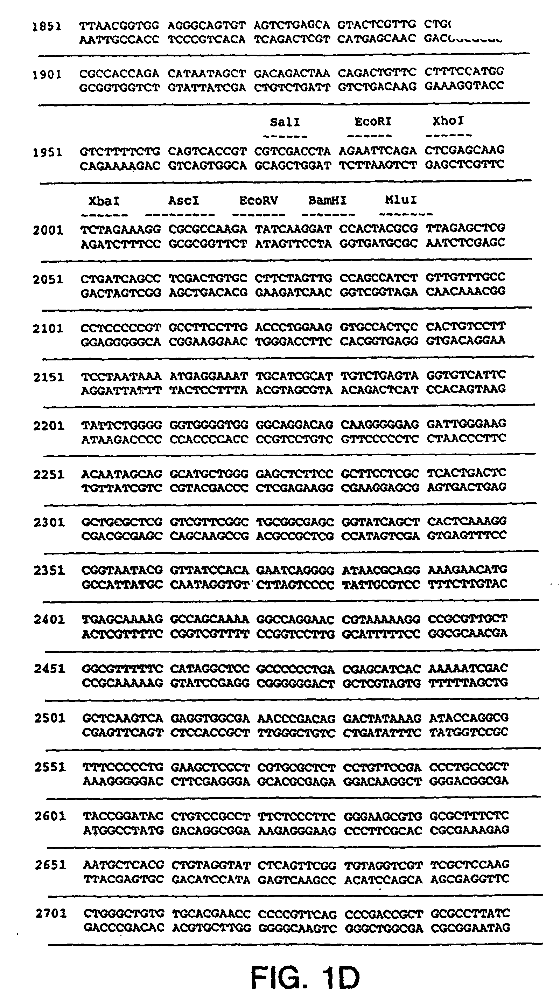

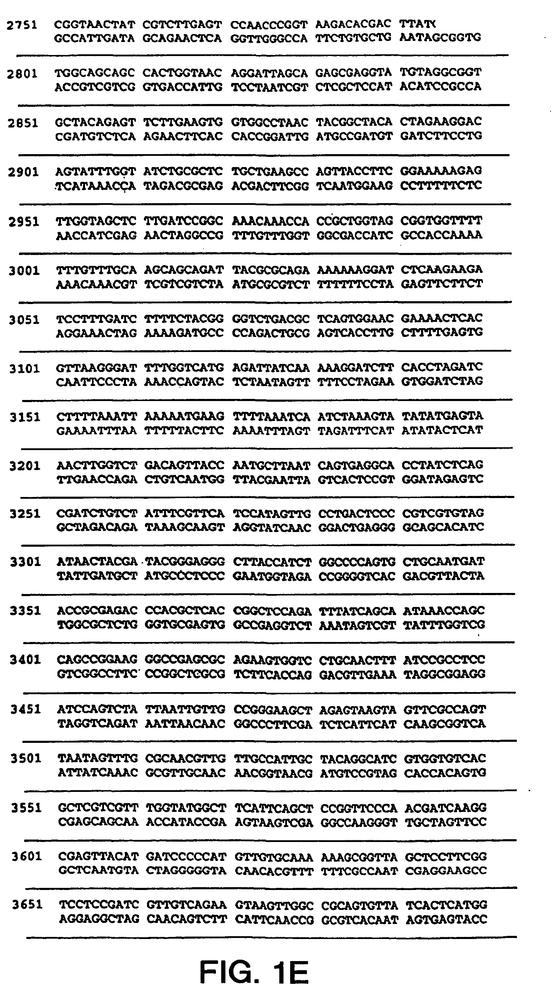

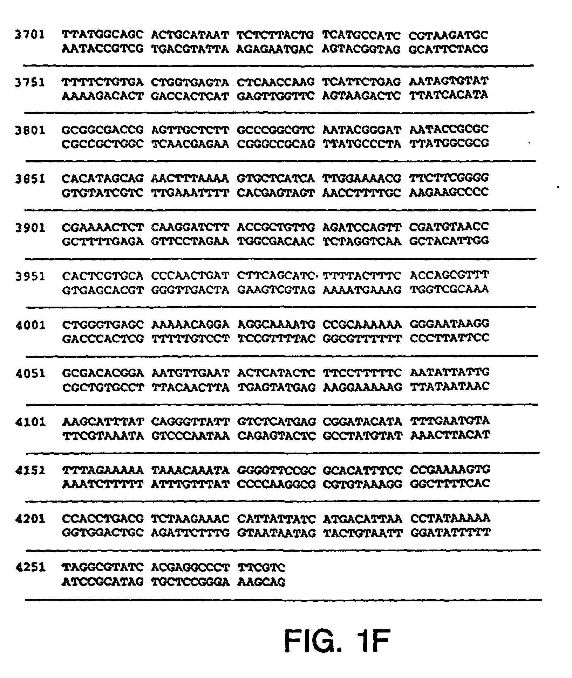

Figures 1A and 1B-1F show the expression vector pCMVn (Fig. 1A) and its nucleotide sequence (Figs. 1B-1F; SEQ ID NO:1).

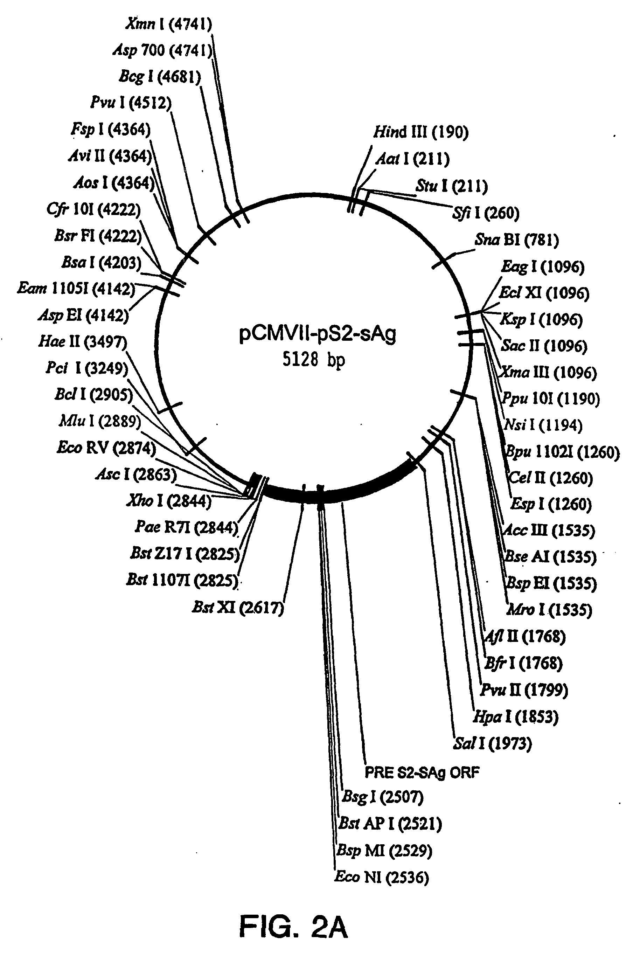

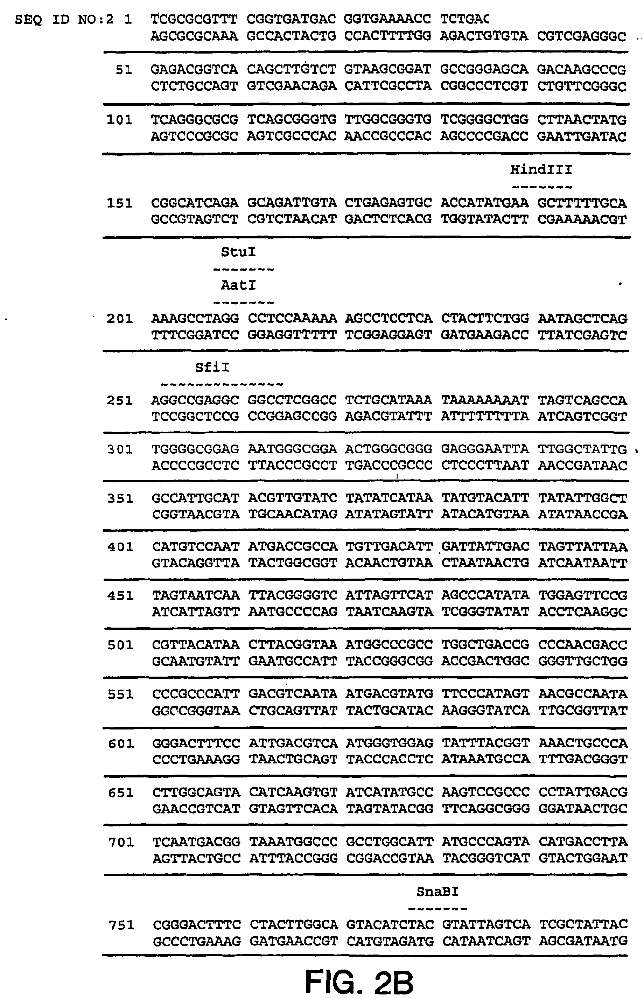

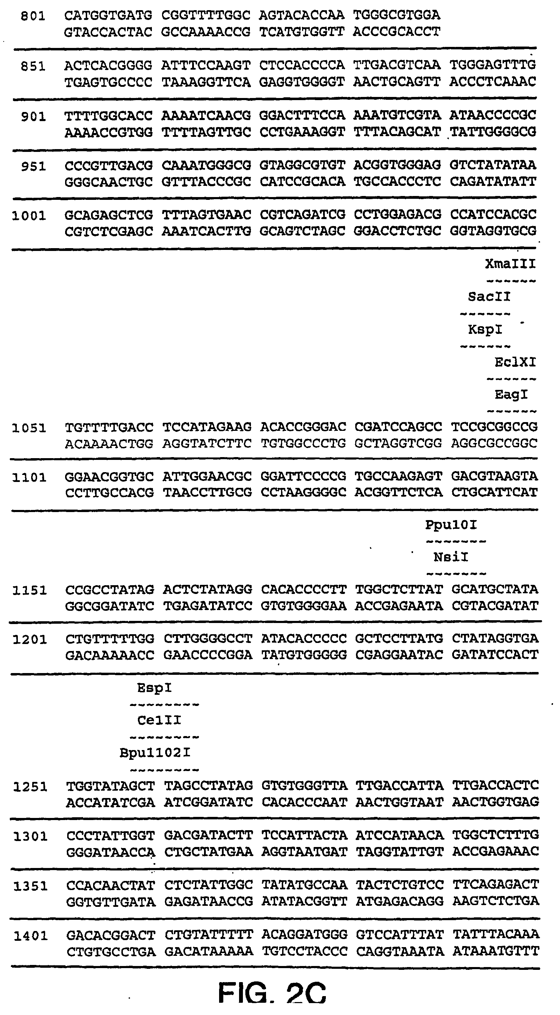

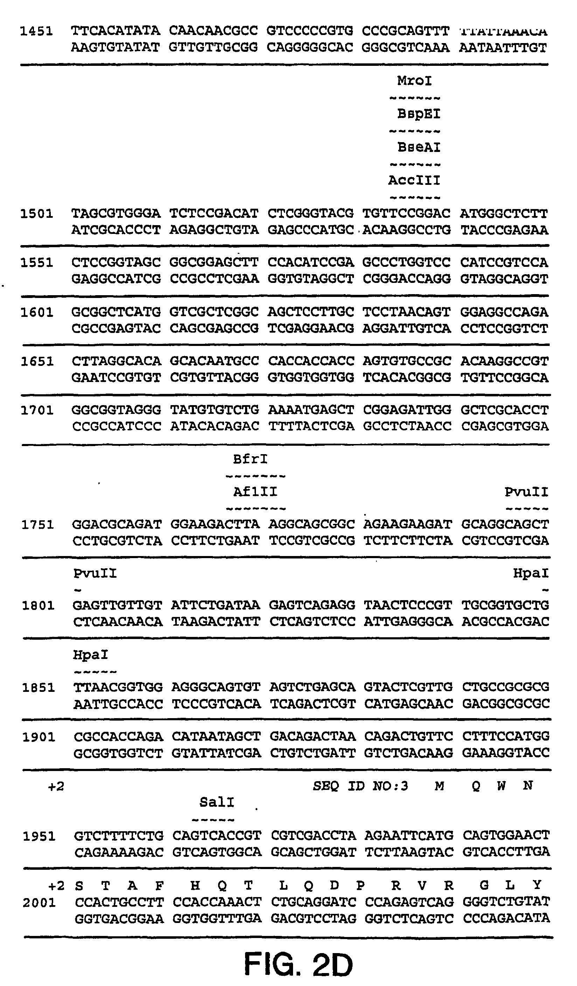

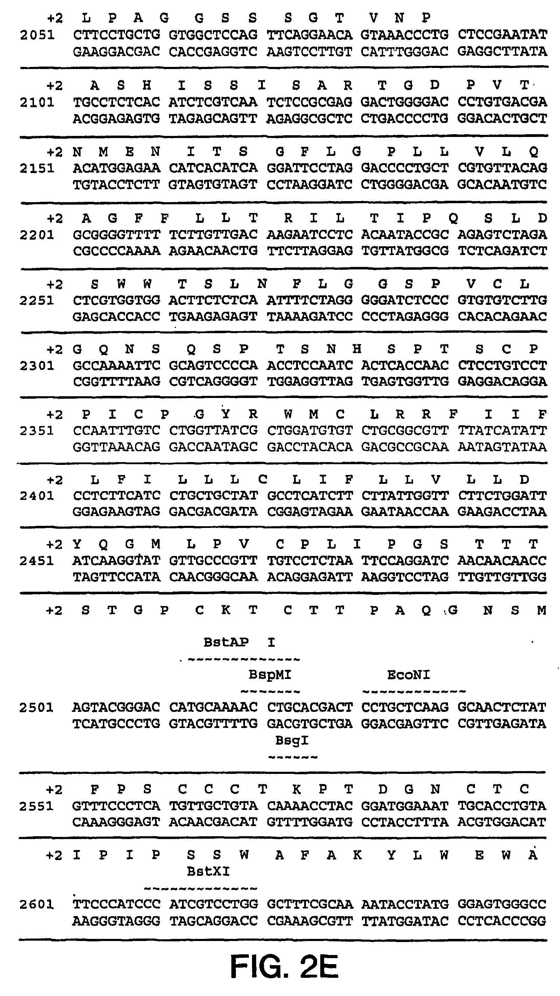

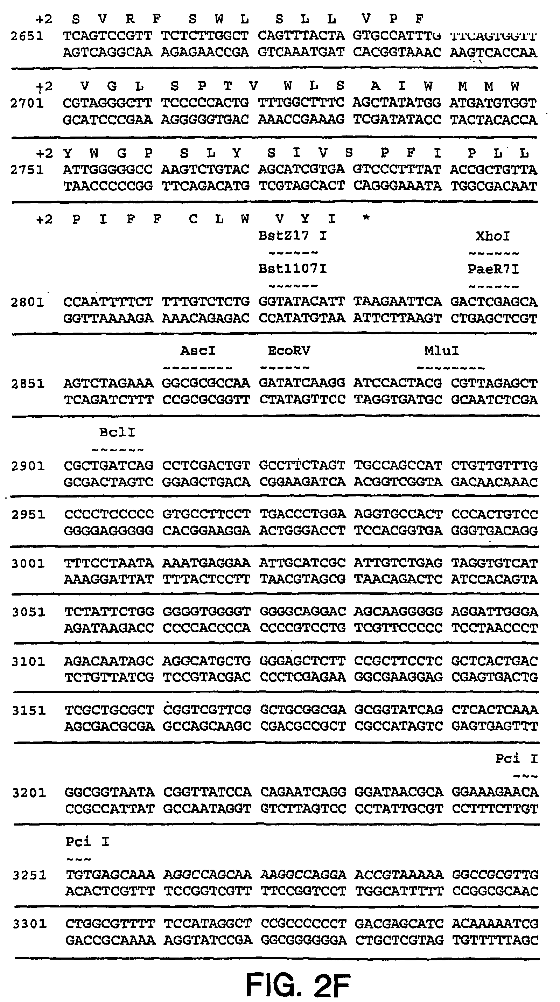

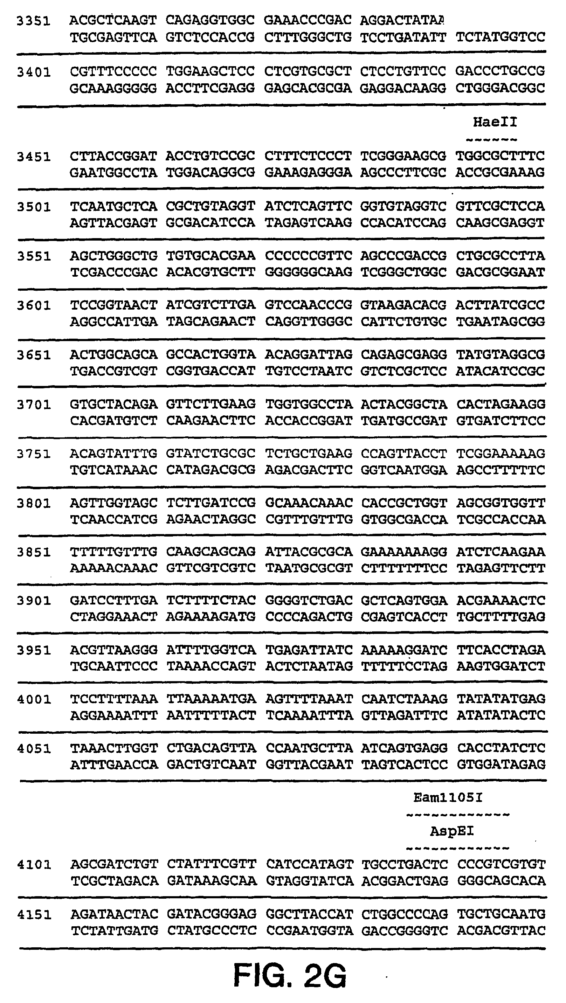

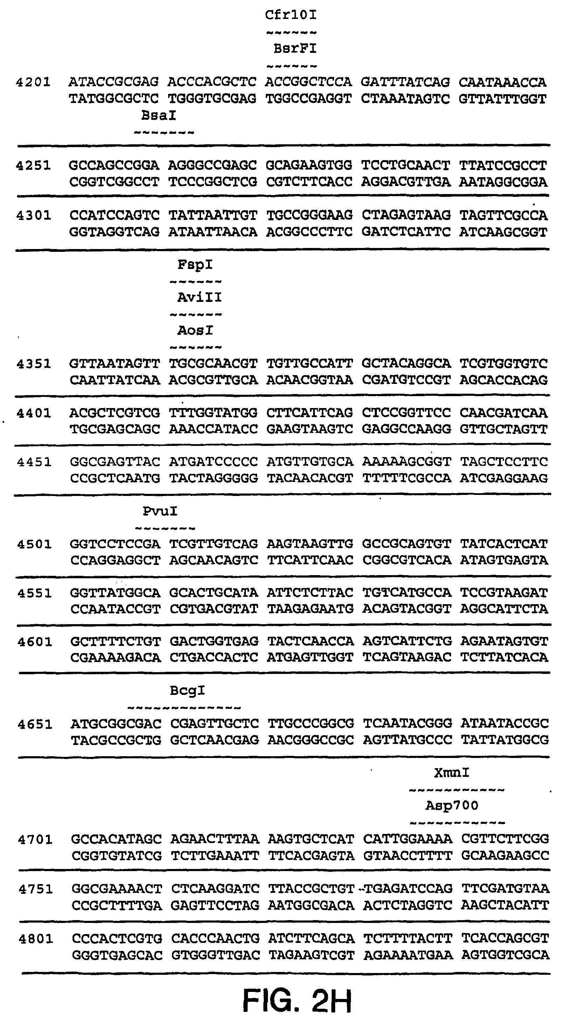

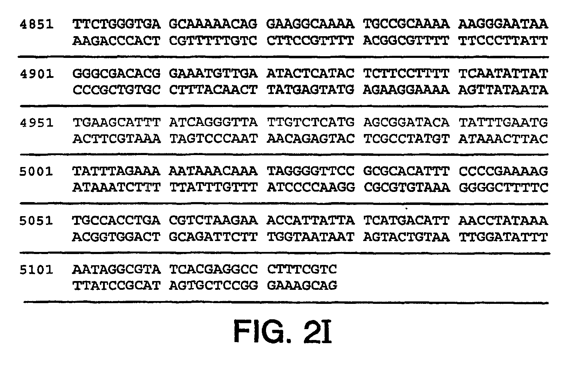

Figures 2A and 2B-2I show the expression vector pCMVII-pS2-sAg (Fig. 2A) and its nucleotide sequence (Figs. 2B-2I and SEQ ID NO:2; preS2 coding sequence begins at nucleotide position 1988 and ends at nucleotide base 2152, and SAg coding sequence begins at base 2153 and ends at 2830). The amino acid sequence of the encoded preS2-S polypeptide is also displayed in Figs. 2B-2I and in SEQ ID NO:3.

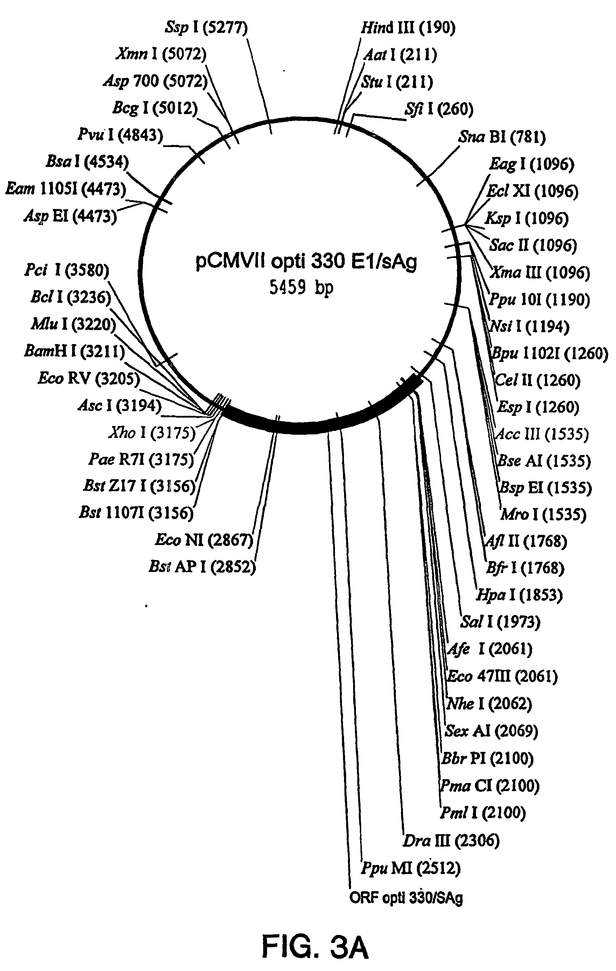

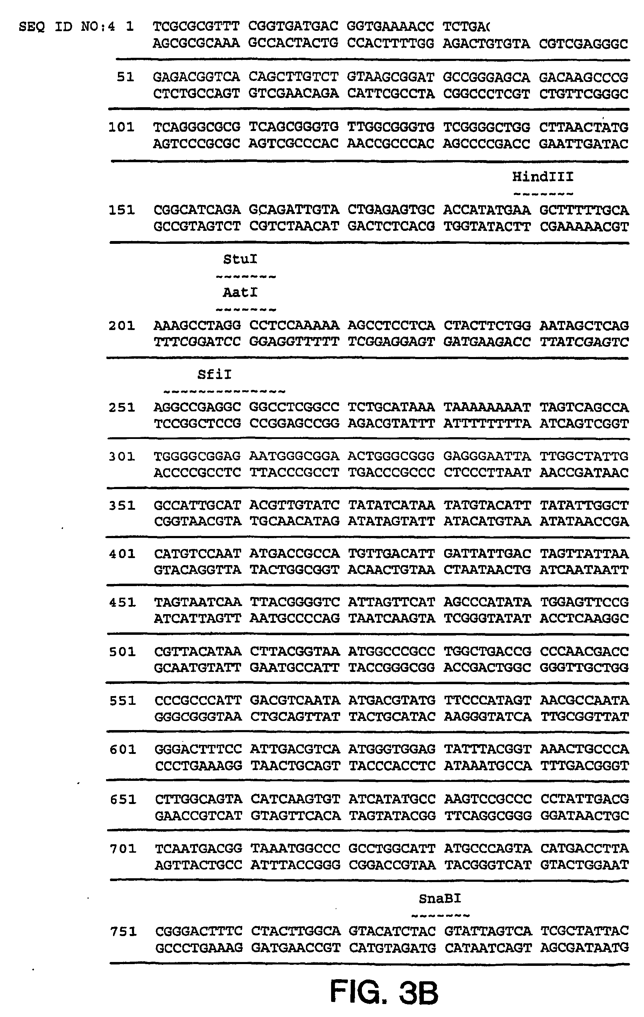

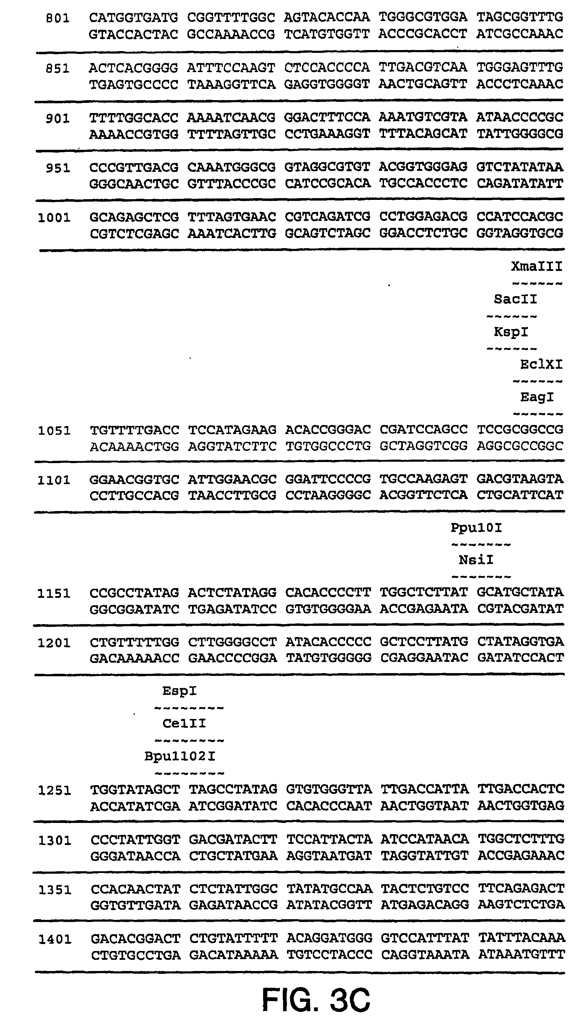

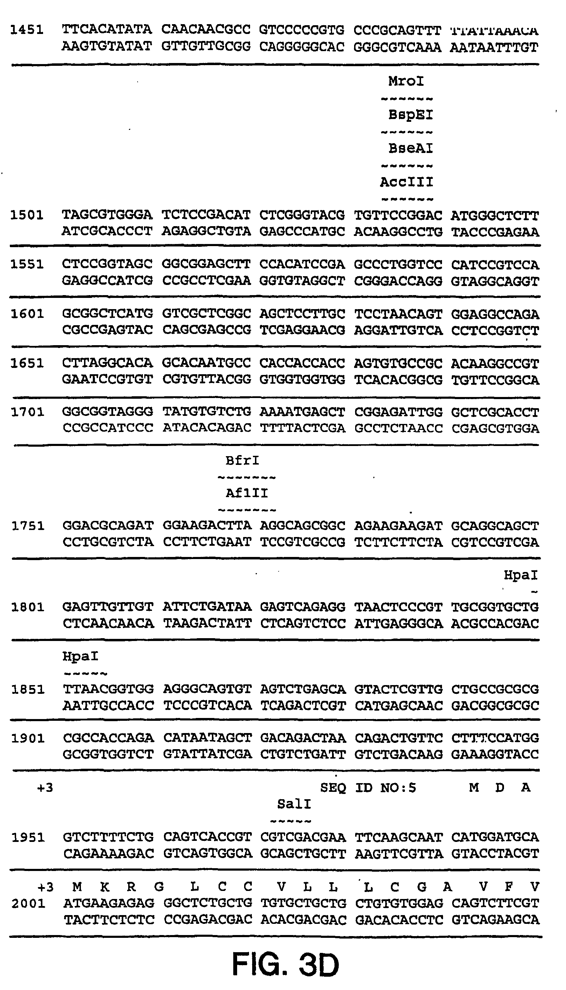

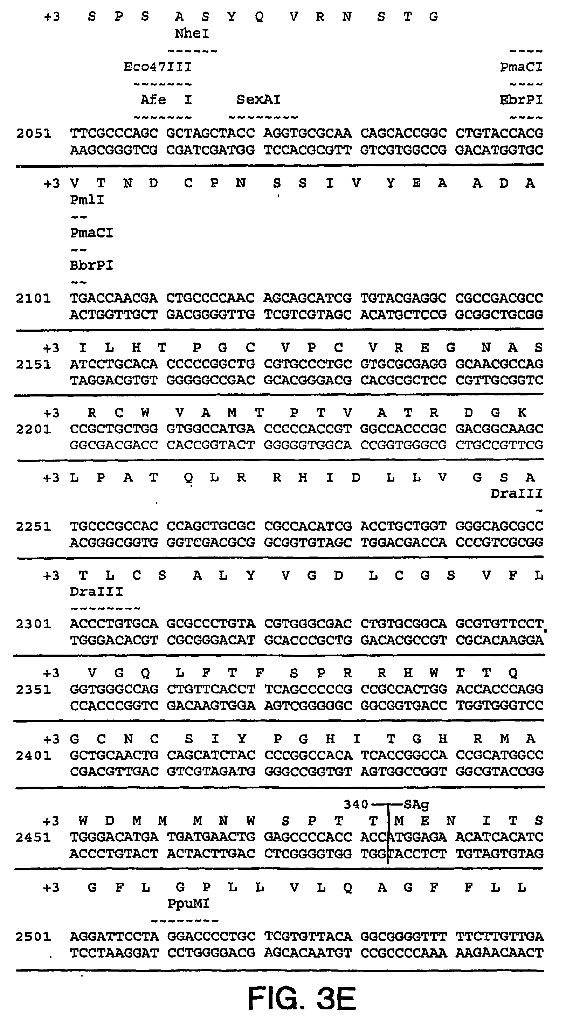

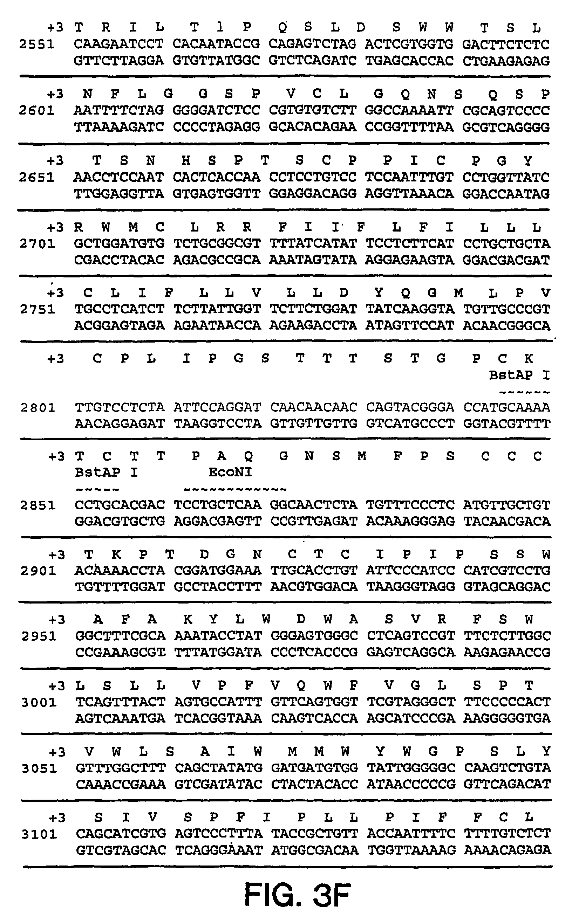

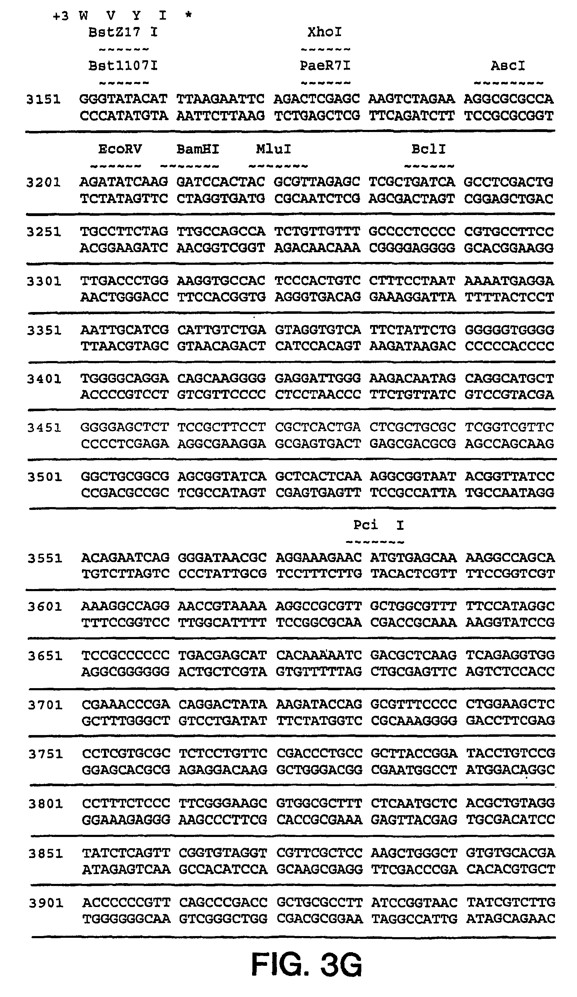

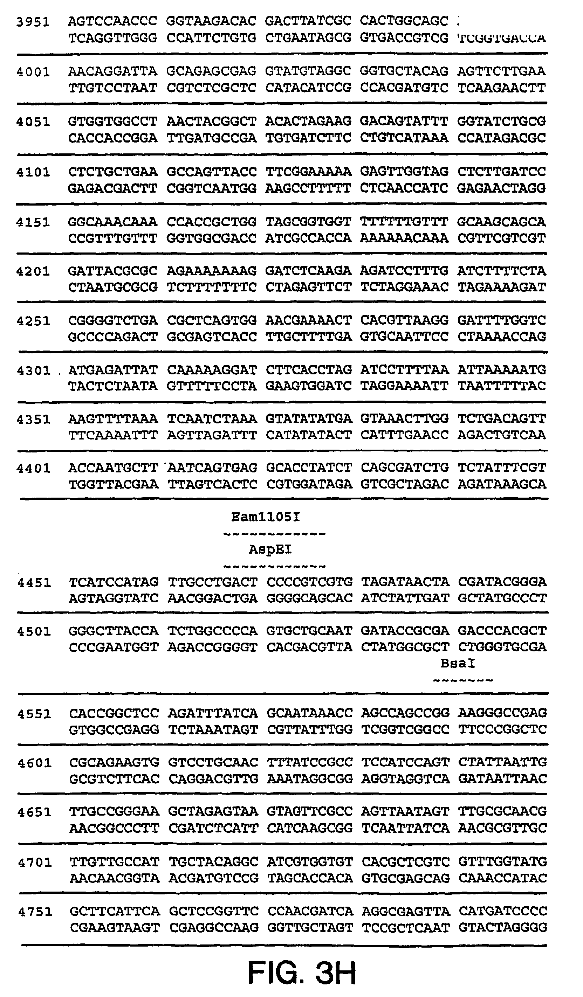

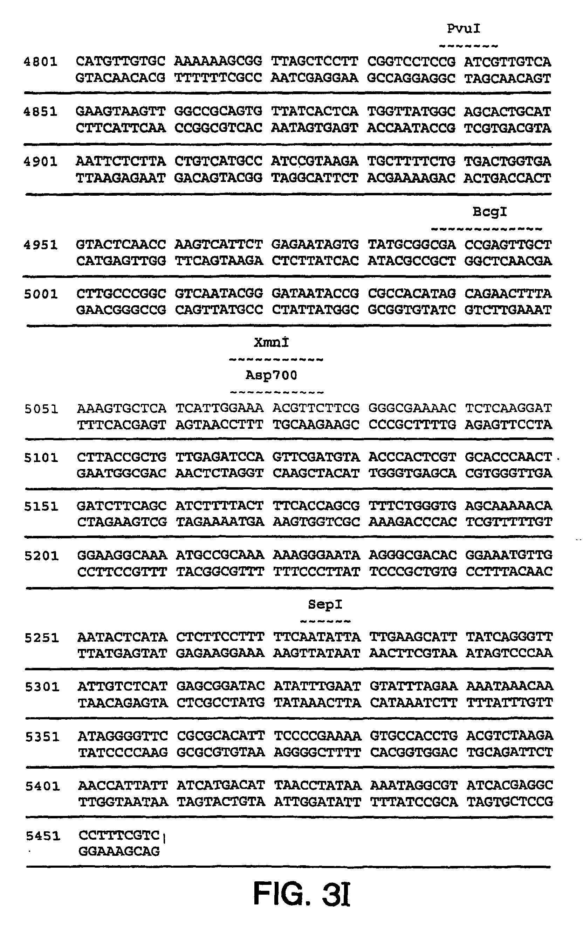

Figures 3A and 3B-3I show the expression vector pCMVIi opti 330 E1/SAg (Fig. 3A) and its nucleotide sequence (Figs. 3B-3I and SEQ ID NO:4; 330 E1 coding sequence begins at nucleotide position 1992 and ends at nucleotide base 2483 and SAg coding sequence begins at base 2484). The amino acid sequence of the encoded chimeric antigen polypeptide is also displayed in Figs. 3B-3I and in SEQ ID NO:5.

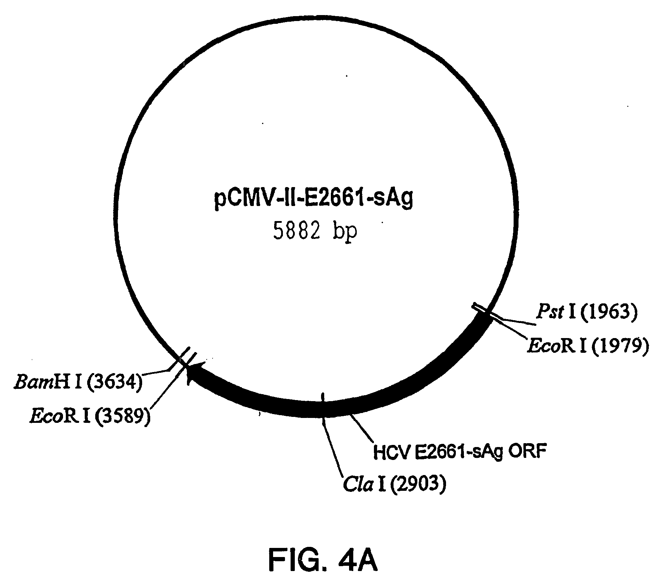

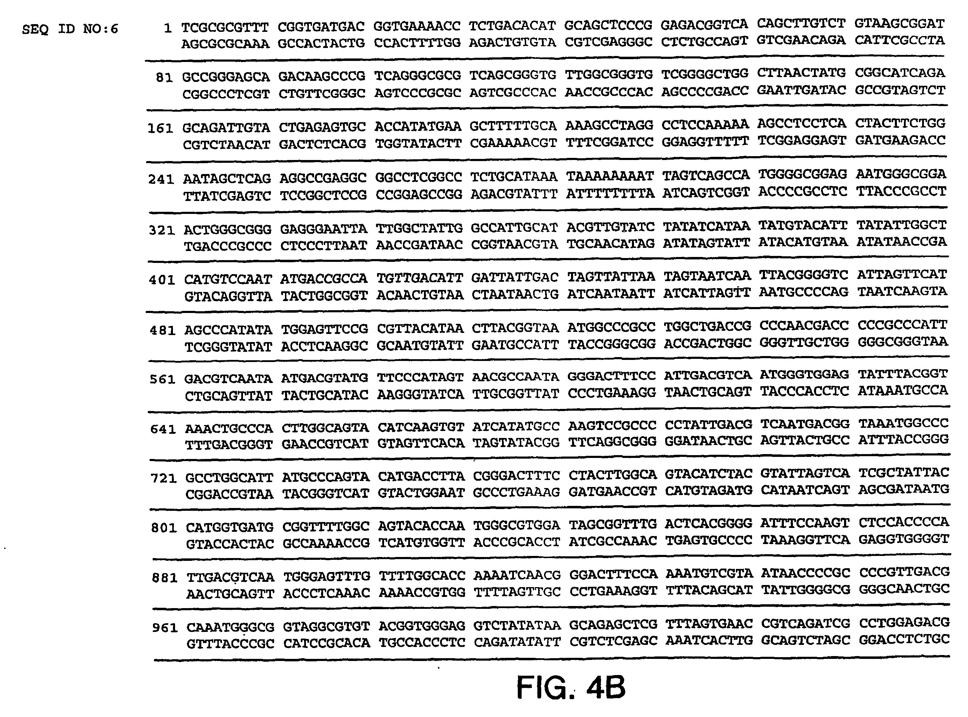

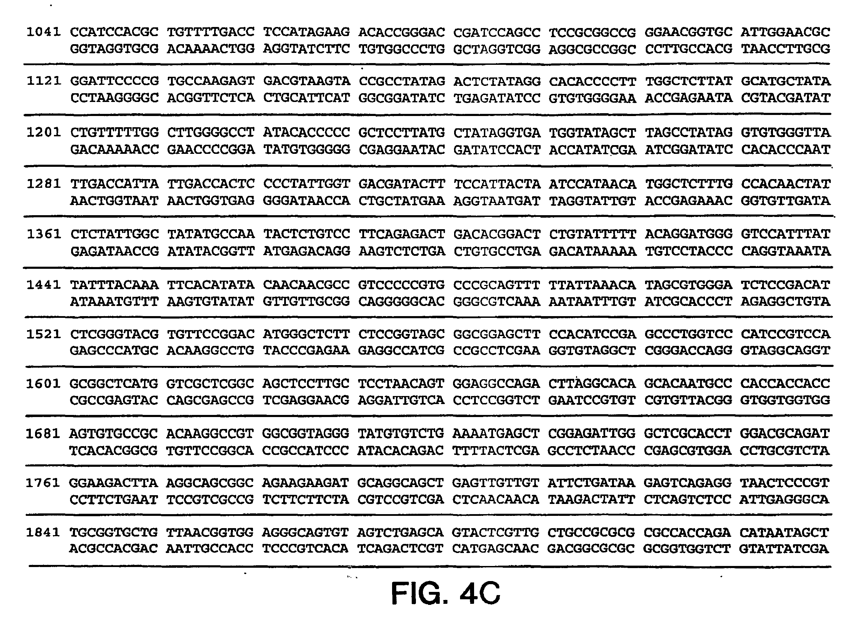

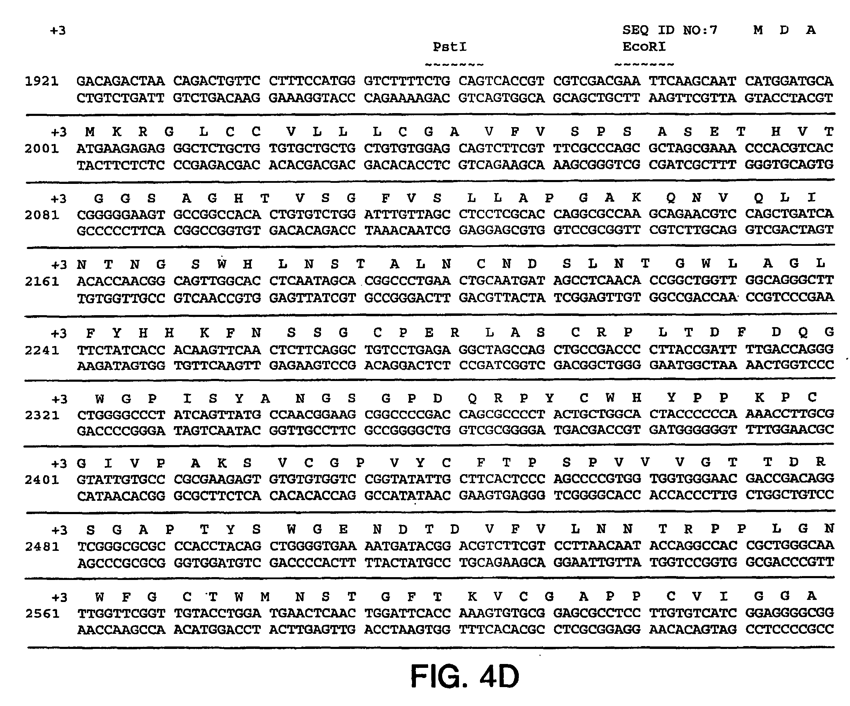

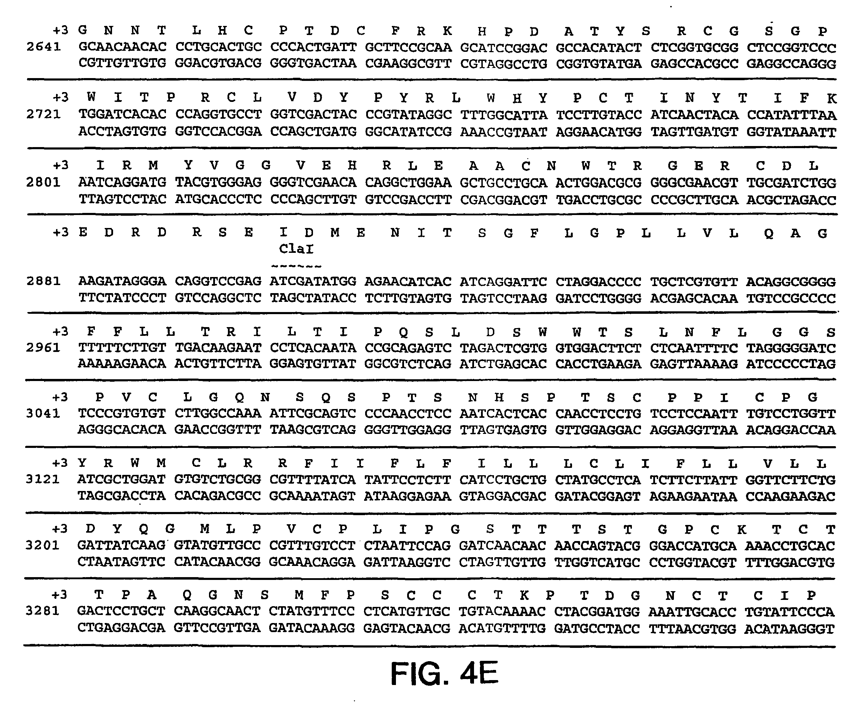

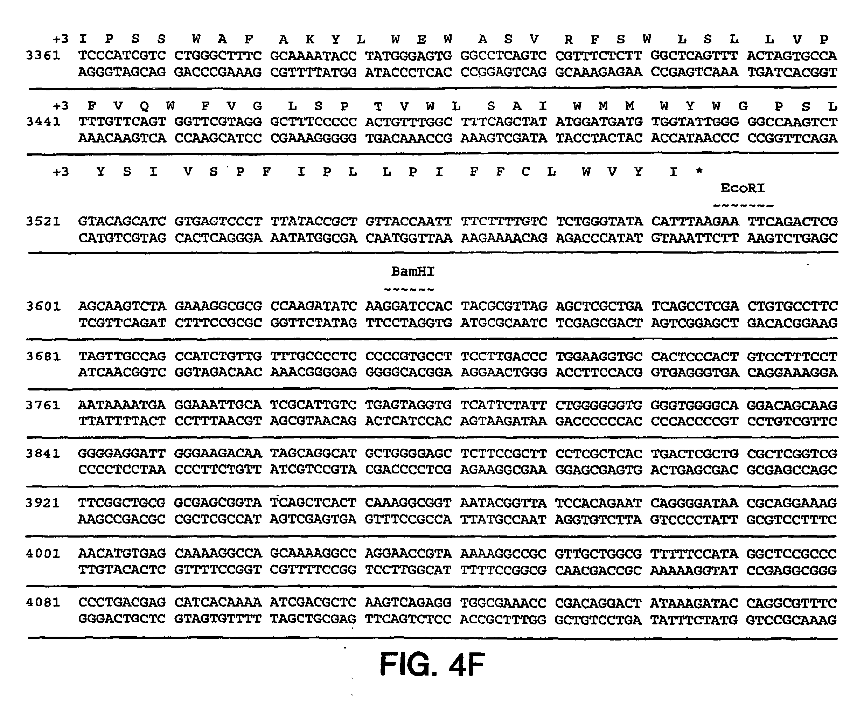

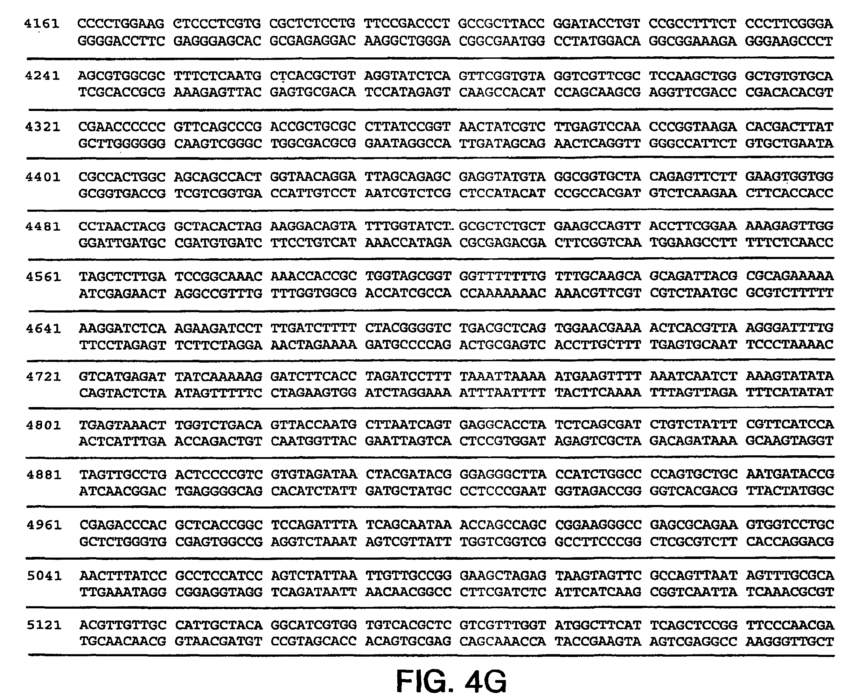

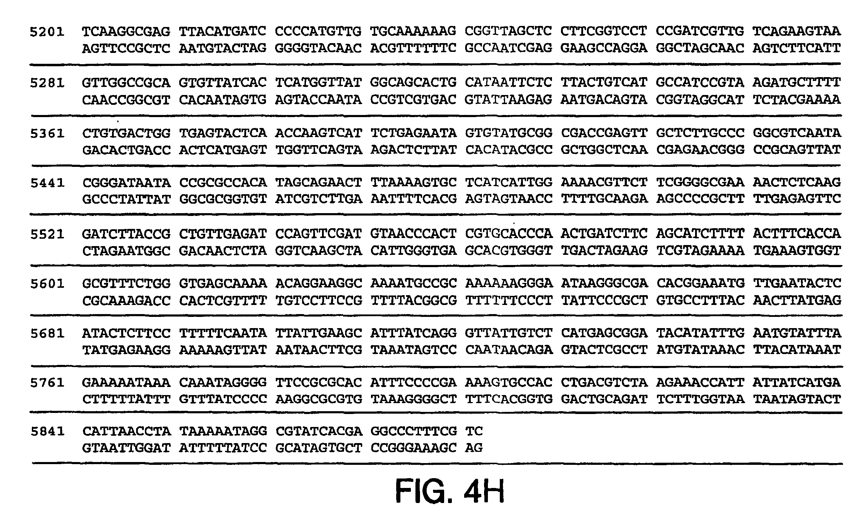

Figures 4A and 4B-4F show the expression vector pCMV-II E2661-sAg ( Fig. 4A) and its nucleotide sequence (Figs. 4B-4F and SEQ ID NO:6; 661 E2 coding sequence begins at nucleotide position 1997 and ends at nucleotide base 2900, and sAg coding sequence begins at base 2907). The amino acid sequence of the encoded chimeric antigen polypeptide is also displayed in Figs. 4B-4F and in SEQ ID NO:7.

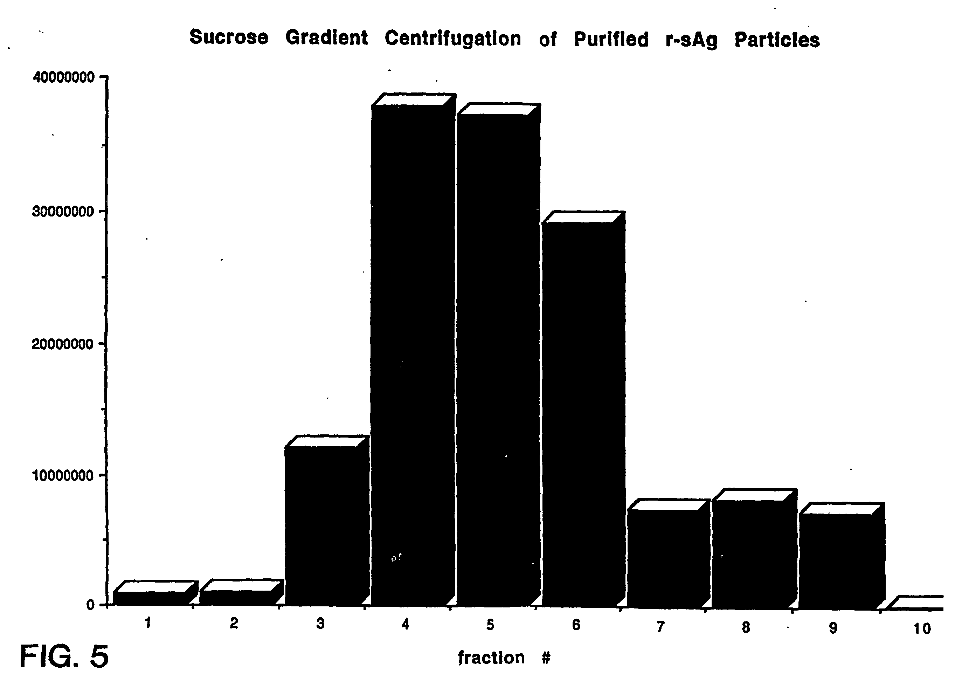

Figure 5 shows the results of sucrose density gradient centrifugation of purified recombinant HBsAg virus-like particles. The experiment is described in Example 1.

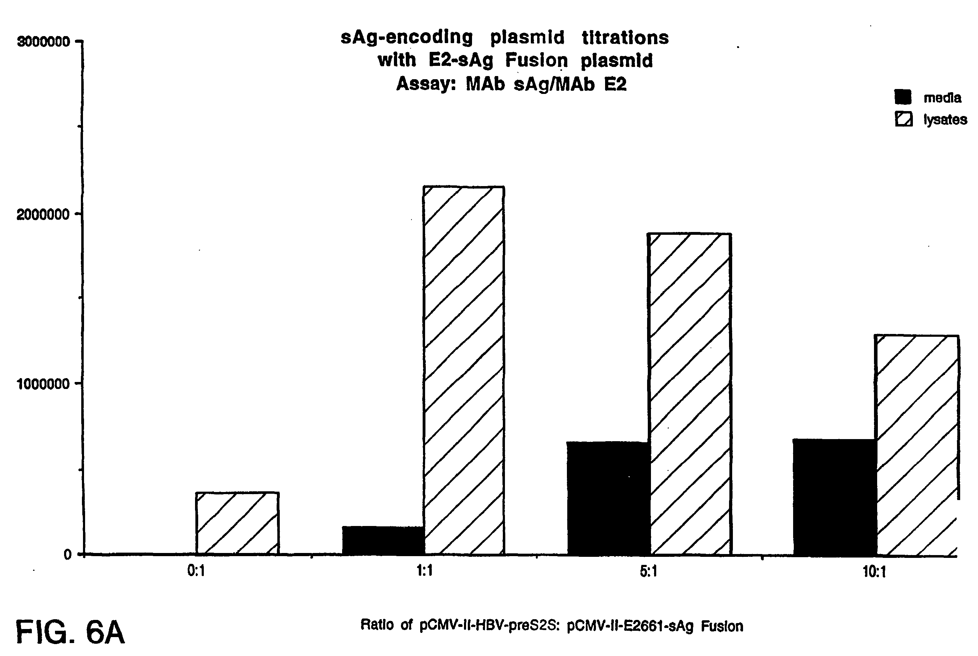

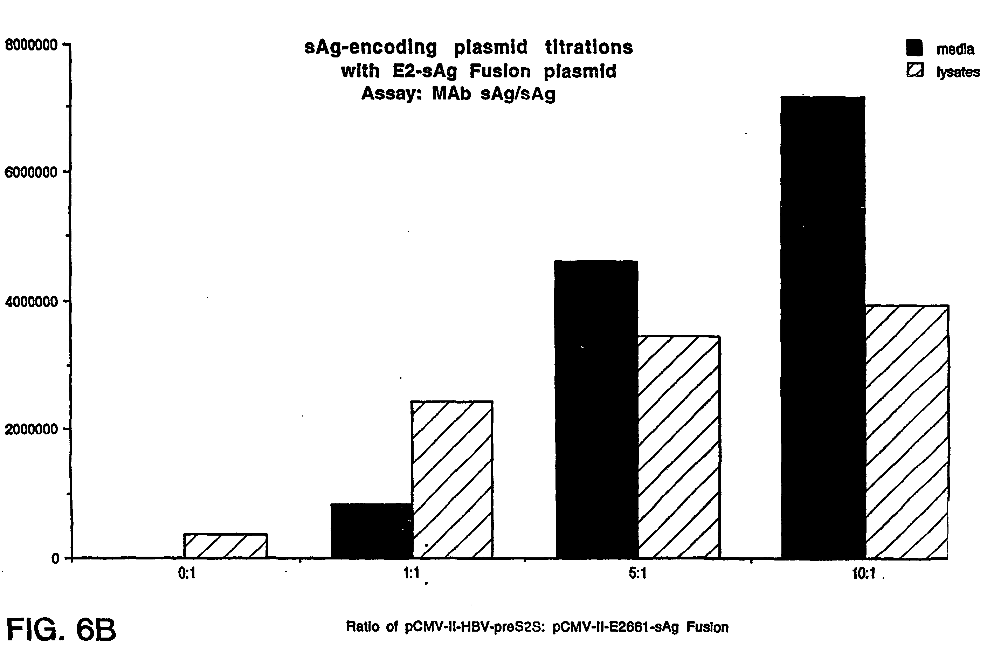

Figures 6A and 6B illustrate the expression of HCVE2-HBsAg fusion protein in COS7 cells. Increasing amounts of sAg-encoding plasmid were expressed together with HCVE2-sAg fusion plasmid. The experiment is described in Example 2. The capture antibody was MAb sAg, and the detecting antibody was MAb E2 (Fig. 6A) or MAb sAg (Fig. 6B).

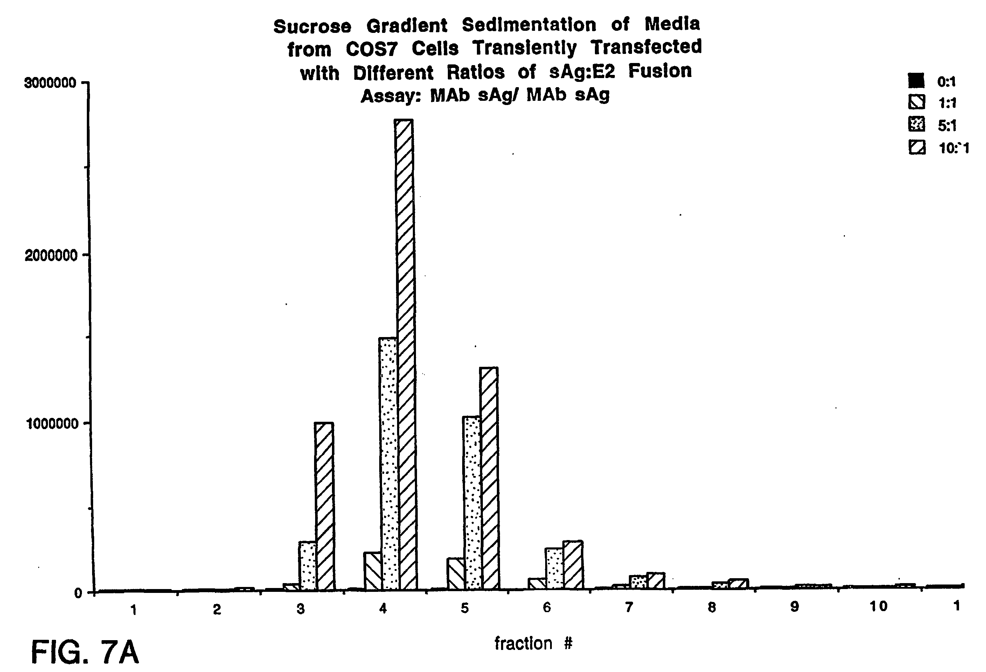

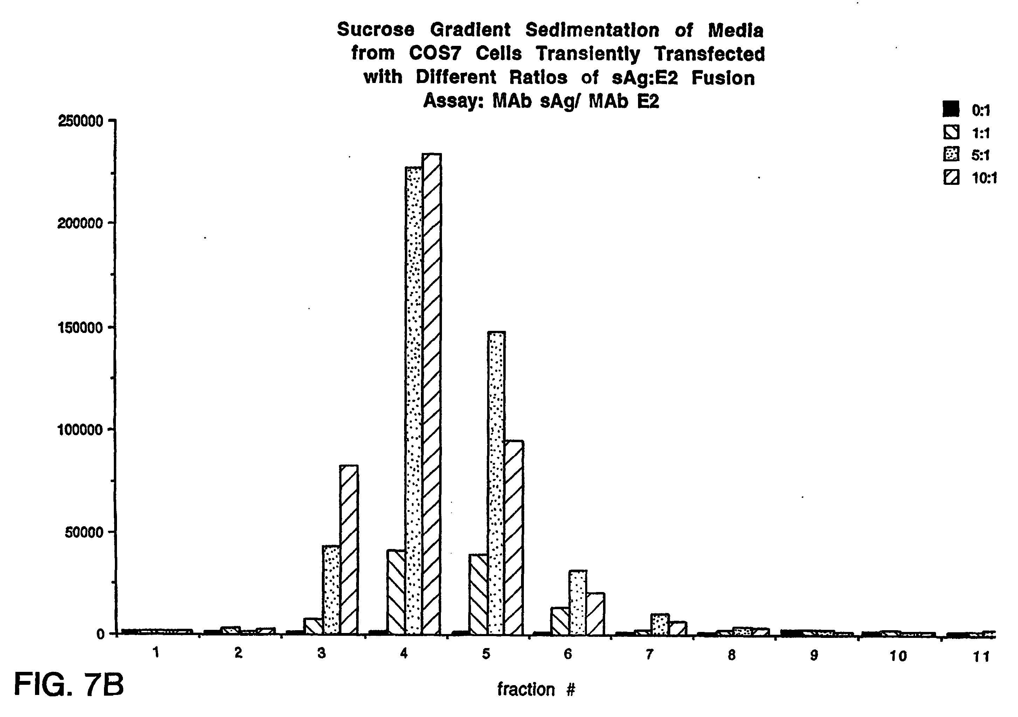

Figures 7A and 7B show the sedimentation profile of virus-like particles expressed from COS7 cells transiently transfected with different ratios of sAg:E2-sAg fusion protein. The experiment is described in Example 2. The capture antibody was MAb sAg, and the detecting antibody was MAb E2 (Fig. 7A) or MAb sAg (Fig. 7B).

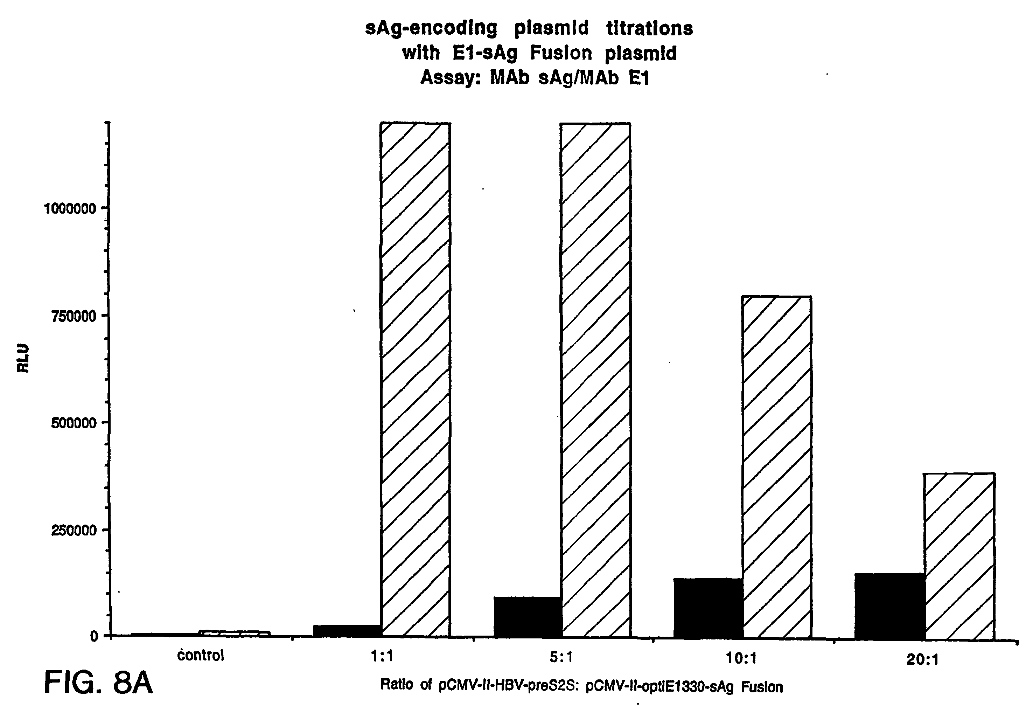

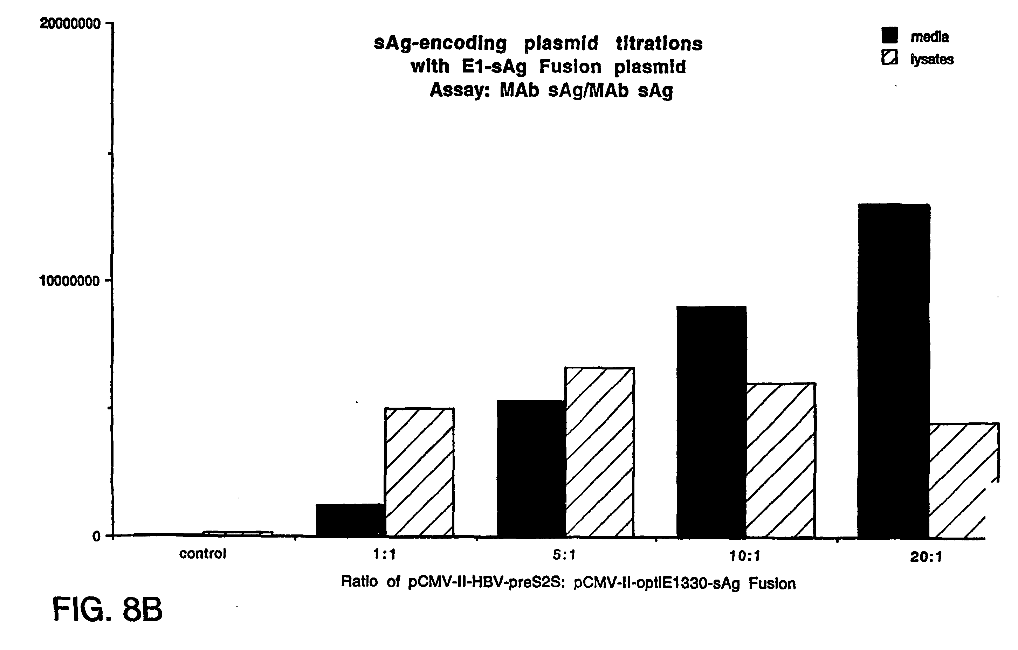

Figures 8A and 8B demonstrate the expression of HCVE1-HBsAg fusion protein in COS7 cells. Increasing amounts of sAg-encoding plasmid were expressed together with HCVE1-sAg fusion plasmid. The experiment is described in Example 3. The capture antibody was MAb sAg, and the detecting antibody was MAb E2 (Fig. 8A) or MAb sAg (Fig. 8B).

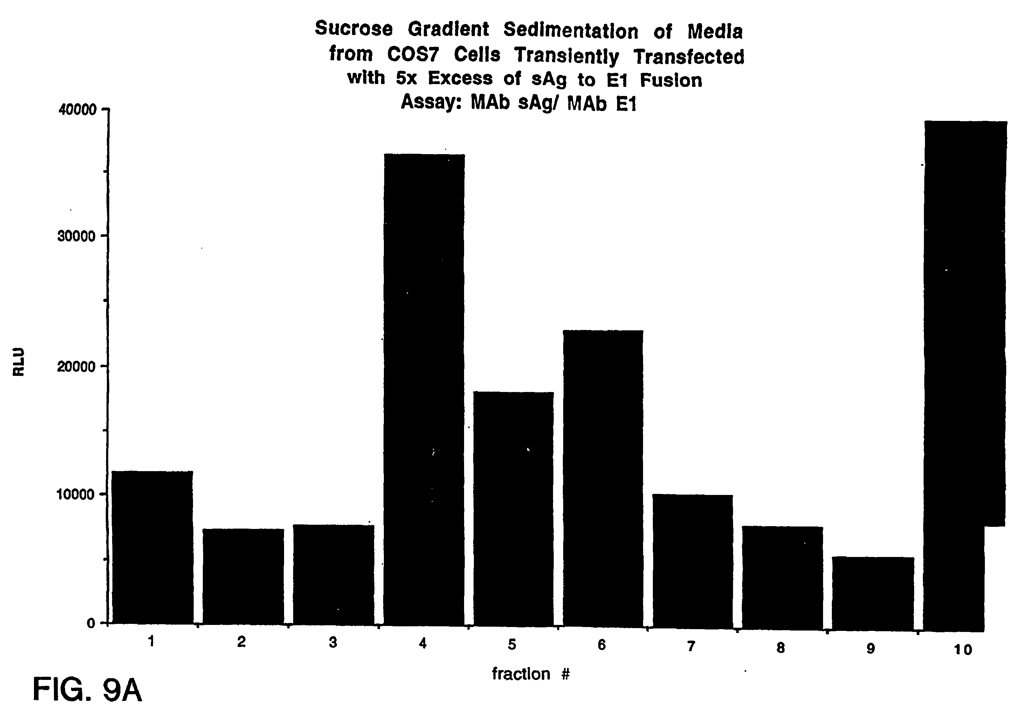

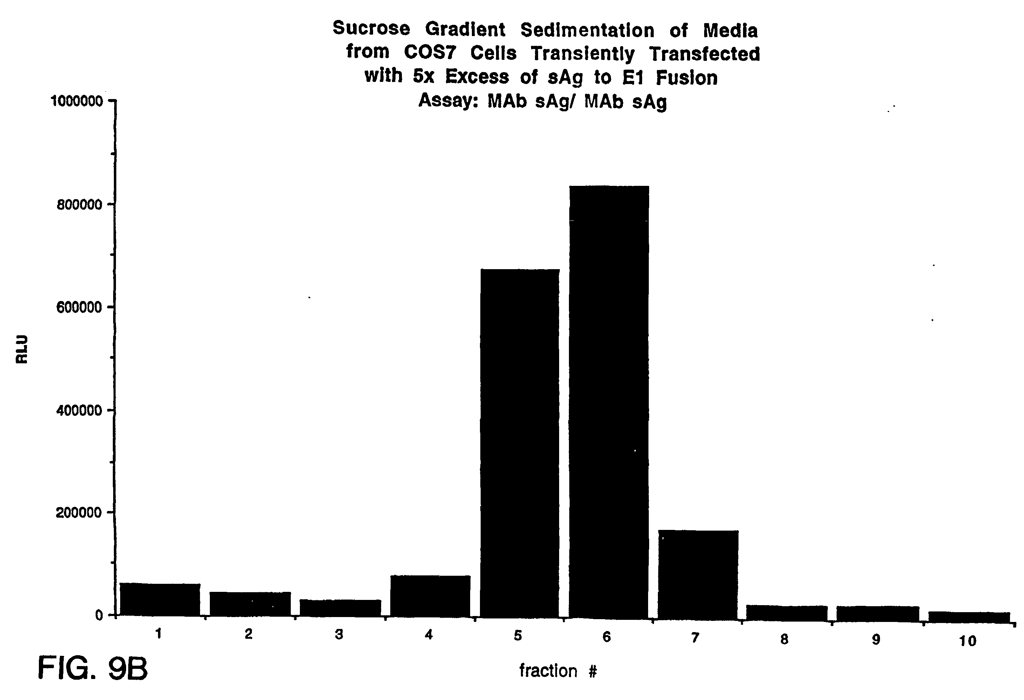

Figures 9A and 9B show the sedimentation profile of virus-like particles expressed from COS7 cells transiently transfected with a 5:1 ratio of sAg:E1-sAg fusion protein. The experiment is described in Example 3. The capture antibody was MAb sAg, and the detecting antibody was MAb E1 (Fig. 9A) or MAb sAg (Fig. 9B).

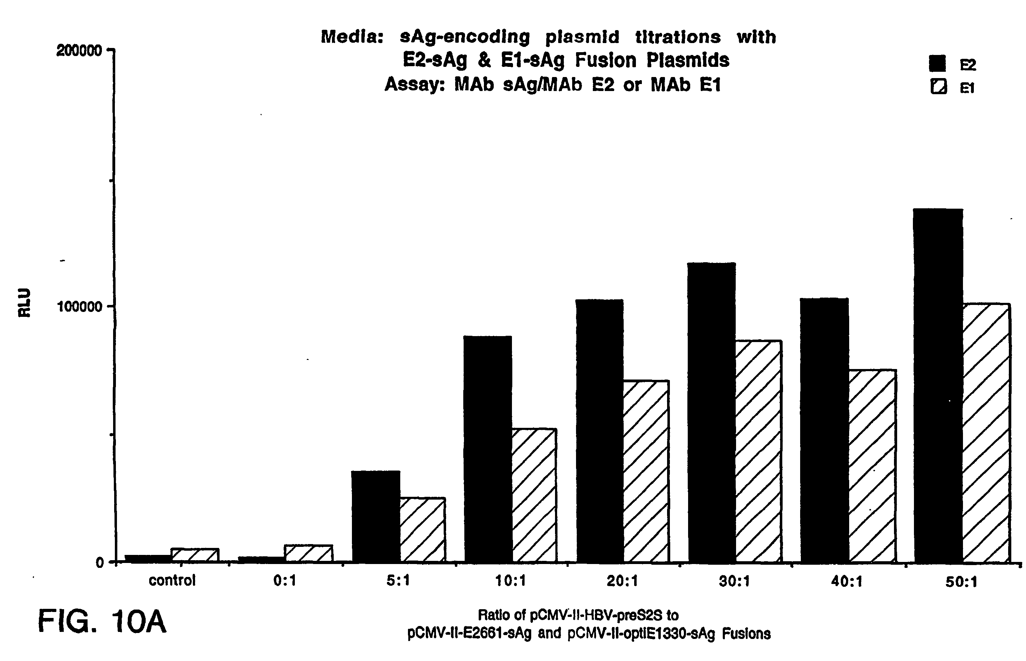

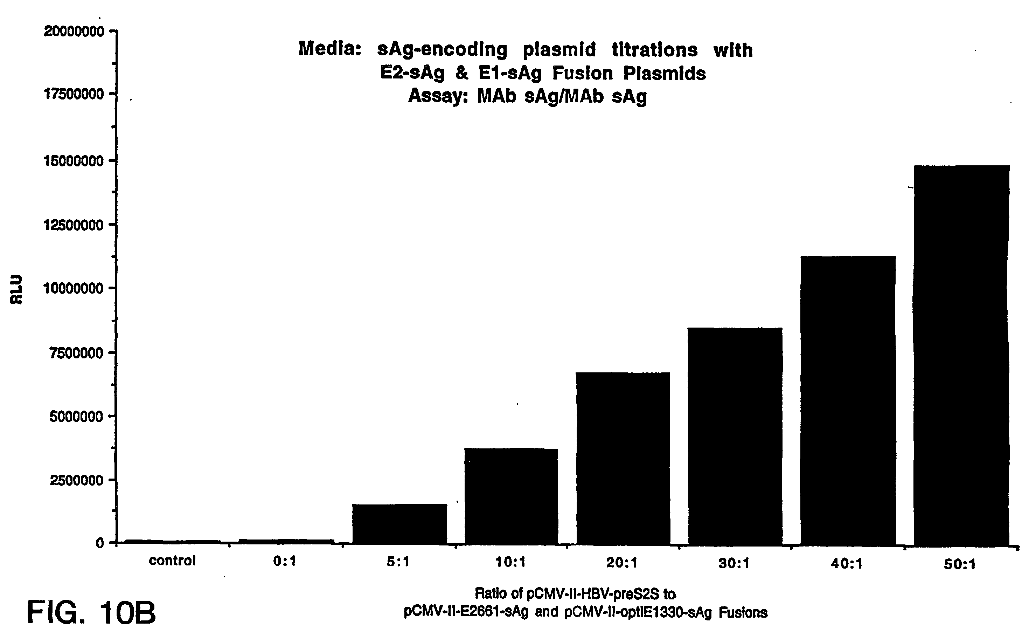

Figures 10A and 10B show the co-expression and secretion of E1-sAg and E2-sAg fusion proteins in COS7 cells. Increasing amounts of sAg-encoding plasmid were expressed together with constant amounts of both E1-sAg and E2-sAg fusion plasmids. The experiment is described in Example 4. In Fig. 10A, detecting antibodies for E1 and E2 were employed, demonstrating the secretion into the media of both E1- and E2-derived polypeptides. In Fig. 10B, the detecting antibody was MAb sAg.

### DETAILED DFSCRIPTION OF THE INVENTION

A summary of standard techniques and procedures which may be employed in order to perform the invention follows. This summary is not a limitation on the invention but, rather, gives examples which may be used, but which are not required.

The practice of the present invention will employ, unless otherwise indicated, conventional techniques of molecular biology, microbiology, recombinant DNA, and immunology, which are within the skill of the art. Such techniques are explained fully in the literature e.g., Sambrook *Molecular Cloning; A Laboratory Manual, Second Edition* (1989); *DNA Cloning, Volumes I and ii* (D.N Glover ed. 1985); *Oligonucleotide Synthesis* (M.J. Gait ed, 1984); *Animal Cell Culture* (R.I. Freshney ed. 1986); the *Methods in Enzymology* series (Academic Press, Inc.), especially volumes 154 & 155; *Gene Transfer Vectors for Mammalian Cells* (J.H. Miller and M.P. Calos eds. 1987, Cold Spring Harbor Laboratory); Mayer and Walker, eds. (1987), *Immunochemical Methods in Cell and Molecular Biology* (Academic Press, London).

It must be noted that, as used in this specification and the appended claims, the singular forms "a", "an" and "the" include plural referents unless the content clearly dictates otherwise. Thus, for example, reference to "an antigen" includes a mixture of two or more antigens, and the like.

Standard abbreviations for nucleotides and amino acids are used in this specification. For example, the following amino acid abbreviations are used throughout the text:

| | |
|---|---|
| Alanine: Ala (A) | Arginine: Arg (R) |
| Asparagine: Asn (N) | Aspartic acid: Asp (D) |
| Cysteine: Cys (C) | Glutamine: Gln (Q) |
| Glutamic acid: Glu (E) | Glycine: Gly (G) |
| Histidine: His (H) | Isoleucine: Ile (I) |
| Leucine: Leu (L) | Lysine: Lys (K) |
| Methionine: Met (M) | Phenylalanine: Phe (F) |
| Proline: Pro (P) | Serine: Ser (S) |
| Threonine: Thr (T) | Tryptophan: Trp (W) |
| Tyrosine: Tyr (Y) | Valine: Val (V) |

### L Definitions

In describing the present invention, the following terms will be employed, and are intended to be defined as indicated below.

The terms "polypeptide" and "protein" are used interchangeably herein and refer to a polymer of amino acid residues and are not limited to a minimum length of the product Thus, peptides, oligopeptides, dimers, multimers, and the like, are included within the definition. Both full-length proteins and fragments thereof are encompassed by the definition. The terms also include postexpression modifications of the polypeptide, for example, glycosylation, acetylation, phosphorylation and the like. Furthermore, for purposes of the present invention, a "polypeptide" refers to a protein which includes modifications, such as deletions, additions and substitutions (generally conservative in nature), to the native sequence, so long as the protein maintains the desired activity. These modifications may be deliberate, as through site-directed mutagenesis, or may be accidental, such as through mutations of hosts which produce the proteins or errors due to PCR amplification.

An HCV polypeptide is a polypeptide, as defined above, derived from the HCV polyprotein. HCV encodes a single polyprotein having more than 3000 amino acid residues (Choo et al. *Science* (1989) 244:359-362; Choo et al. *Proc. Natl. Acad Sci. USA* (1991) 88:2451-2455; Han et al. *Proc. Natl. Acad Sci. USA* (1991) 88:1711-1715). The polyprotein is processed co- and post-translationally into both structural and non-structural (NS) proteins.

In particular, several proteins are encoded by the HCV genome. The order and nomenclature of the cleavage products of the HCV polyprotein is as follows: NH₂-C-E1-E2-NS2-NS3-NS4a-NS4b-NSSa-NSSb-COOH. Initial cleavage of the polyprotein is catalyzed by host proteases which liberate three structural proteins, the N-terminal nucleocapsid protein (termed "Core") and two envelope glycoproteins, "E1" (also known as E) and "E2" (also known as E2/NS1), as well as nonstructural (NS) proteins that contain the viral enzymes. The NS regions are termed NS2, NS3, NS4 and NS5. NS2 is an integral membrane protein with proteolytic activity. NS2, either alone or in combination with NS3, cleaves the NS2-NS3 sissle bond which in turn generates the NS3 N-terminus and releases a large polyprotein that includes both serine protease and RNA helicase activities. The NS3 protease serves to process the remaining polyprotein. Completion of polyprotein maturation is initiated by autocatalytic cleavage at the NS3-NS4a junction, catalyzed by the NS3 serine protease. Subsequent NS3-mediated cleavages of the HCV polyprotein appear to involve recognition of polyprotein cleavage junctions by an NS3 molecule of another polypeptide. In these reactions, NS3 liberates an NS3 cofactor (NS4a), two proteins with unknown function (NS4b and NS5a), and an RNA-dependent RNA polymerase (NS5b). Any one of these proteins, as well as immunogenic fragments thereof, will find use with the subject chimeric antigens.

The polypeptide for use in the chimeric antigens need not be physically derived from HCV, but may be synthetically or recombinantly produced. Moreover, the polypeptide may be derived from any of the various HCV strains, such as from strains 1, 2, 3 or 4 of HCV (described further below). A number of conserved and variable regions are known between these strains and, in general, the amino acid sequences of epitopes derived from these regions will have a high degree of sequence homology, e.g., amino acid sequence homology of more than 30%, preferably more than 40%, preferably more than 50% , preferably more than about 75%, more preferably more than about 80%-85%, preferably more than about 90%, and most preferably at least about 95%-98% sequence identity, or more, when the two sequences are aligned. Thus, for example, the term "E2" polypeptide refers to the native E2 protein from any of the various HCV strains, as well as E2 analogs, muteins and immunogenic fragments, as defined further below.

The terms "analog" and "mutein" refer to biologically active derivatives of the reference molecule, or fragments of such derivatives, that retain desired activity, such as immunological activity as described herein. In general, the term "analog" refers to compounds having a native polypeptide sequence and structure with one or more amino acid additions, substitutions (generally conservative in nature) and/or deletions, relative to the native molecule, so long as the modifications do not destroy immunogenic activity. The term ''mutein'' refers to peptides having one or more peptide mimics ("peptoids"), such as those described in International Publ. No. WO 91/04282. Preferably, the analog or mutein has at least the same immunoactivity as the native molecule. Methods for making polypeptide analogs and muteins are known in the art and are described further below.

Particularly preferred analogs include substitutions that are conservative in nature, i.e., those substitutions that take place within a family of amino acids that are related in their side chains. Specifically, amino acids are generally divided into four families: (1) acidic - aspartate and glutamate; (2) basic - lysine, arginine, histidine; (3) non-polar - alanine, valine, leucine, isoleucine, proline, phenylalanine, methionine, tryptophan; and (4) uncharged polar - glycine, asparagine, glutamine, cysteine, serine threonine, tyrosine. Phenylalanine, tryptophan, and tyrosine are sometimes classified as aromatic amino acids. For example, it is reasonably predictable that an isolated replacement of leucine with isoleucine or valine, an aspartate with a glutamate, a threonine with a serine, or a similar conservative replacement of an amino acid with a structurally related amino acid, will not have a major effect on the biological activity. For example, the polypeptide of interest may include up to about 5-10 conservative or non-conservative amino acid substitutions, or even up to about 15-25 conservative or non-conservative amino acid substitutions, or any integer between 5-25, so long as the desired function of the molecule remains intact. One of skill in the art may readily determine regions of the molecule of interest that can tolerate change by reference to Hopp/Woods and Kyte-Doolittle plots, well known in the art.

By "fragment" is intended a polypeptide consisting of only a part of the intact full-length polypeptide sequence and structure. The fragment can include a C-terminal deletion and/or an N-terminal deletion of the native polypeptide. An "immunogenic fragment" of a particular HCV protein will generally include at least about 5-10 contiguous amino acid residues of the full-length molecule, preferably at least about 15-25 contiguous amino acid residues of the full-length molecule, and most preferably at least about 20-50 or more contiguous amino acid residues of the full-length molecule, that define an epitope, or any integer between 5 amino acids and the full-length sequence, provided that the fragment in question retains the ability to elicit an immune response as defined below. For example, preferred immunogenic fragments, include but are not limited to fragments of HCV Core that comprise, e.g., amino acids 10-45, 10-53, 67-88, 81-130, 86-100, 120-130, 121-135 and 121-170 of the polyprotein, numbered relative to the HCV-1a sequence presented in Choo et al. (1991) *Proc Natl Acad Sci USA* 88:2451, as well as defined epitopes derived from the c33c region of the HCV polyprotein, as well as any of the other various epitopes identified from the HCV core, E1, E2, NS3 and NS4 regions. See, e.g., Chien et al. *Proc. Natl. Acad. Sci. USA* (1992) 89:10011-10015; Chien et al. *J. Gastroent. Hepatol.* (1993) 8:S33-39; Chien et al. International Publ. No. WO 93/00365; Chien, D.Y. International Publ. No. WO 94/01778; and U.S. Patent No. 6,150,087. Representative fragments of E1 and E2 polypeptides include C-terminally truncated variants of these molecules, such as E1 polypeptides terminating at, e.g., amino acids 369 and lower, such as e.g., E1 polypeptides ending in amino acids 351, 352, 353 and so on, and E2 polypeptides, terminating at about amino acids 730, such as E2 polypeptides ending in for example amino acids 716, 7I7, 718 and so on. These molecules are described in, e.g., U.S. Patent No. 6,121,020. "Antigenic determinant" refers to the site on an antigen or hapten to which a specific antibody molecule or specific cell surface receptor binds.

The term "epitope" as used herein refers to a sequence of at least about 3 to 5, preferably about 5 to 10 or 15, and not more than about 1,000 amino acids (or any integer therebetween), which define a sequence that by itself or as part of a larger sequence, will stimulate a host's immune system to make a cellular antigen-specific immune response when the antigen is presented, or a humoral antibody response. An epitope for use in the subject invention is not limited to a polypeptide having the exact sequence of the portion of the parent protein from which it is derived. Indeed, viral genomes are in a state of constant flux and contain several variable domains which exhibit relatively high degrees of variability between isolates. Thus the term "epitope" encompasses sequences identical to the native sequence, as well as modifications to the native sequence, such as deletions, additions and substitutions (generally conservative in nature).

Regions of a given polypeptide that include an epitope can be identified using any number of epitope mapping techniques, well known in the art. See, e.g., *Epitope Mapping Protocols* in Methods in Molecular Biology, Vol. 66 (Glenn E. Morris, Ed., 1996) Humana Press, Totowa, New Jersey. For example, linear epitopes may be determined by e.g., concurrently synthesizing large numbers of peptides on solid supports, the peptides corresponding to portions of the protein molecule, and reacting the peptides with antibodies while the peptides are still attached to the supports. Such techniques are known in the art and described in, e.g., U.S. Patent No. 4,708,871; Geysen et al. (1984) *Proc. Natl. Acad. Sci. USA* 81:3998-4002; Geysen et al. (1986) *Molec. Immunol.* 23:709-715. Similarly, conformational epitopes are readily identified by determining spatial conformation of amino acids such as by, e.g., x-ray crystallography and 2-dimensional nuclear magnetic resonance. See, e.g., *Epitope Mapping Protocols, supra.* Antigenic regions of proteins can also be identified using standard antigenicity and hydropathy plots, such as those calculated using, e.g., the Omiga version 1.0 software program available from the Oxford Molecular Group. This computer program employs the Hopp/Woods method, Hopp et al., *Proc. Natl.* *Acad. Sci USA* (1981) 78:3824-3828 for determining antigenicity profiles, and the Kyte-Doolittle technique, Kyte et al., *J*. *Mol. Biol.* (1982) 157:105-132 for hydropathy plots.

As used herein, the term "conformational epitope" refers to a portion of a full-length protein, or an analog or mutein thereof, having structural features native to the amino acid sequence encoding the epitope within the full-length natural protein. Native structural features include, but are not limited to, glycosylation and three dimensional structure. Preferably, a conformational epitope is produced recombinantly and is expressed in a cell from which it is extractable under conditions which preserve its desired structural features, e.g. without denaturation of the epitope. 'Such cells include bacteria, yeast, insect, and mammalian cells. Expression and isolation of recombinant conformational epitopes from the HCV polyprotein are described in e.g., International Publ. Nos. WO 96/04301, WO 94/01778, WO 95/33053, WO 92/08734.

As used herein the term "T-cell epitope" refers to a feature of a peptide structure which is capable of inducing T-cell immunity towards the peptide structure or an associated hapten. T-cell epitopes generally comprise linear peptide determinants that assume extended conformations within the peptide-binding cleft of MHC molecules, (Unanue et al., *Science* (1987) 236:551-557). Conversion of polypeptides to MHC class II-associated linear peptide determinants (generally between 5 - 14 amino acids in length) is termed "antigen processing" which is carried out by antigen presenting cells (APCs). More particularly, a T-cell epitope is defined by local features of a short peptide structure, such as primary amino acid sequence properties involving charge and hydrophobicity, and certain types of secondary structure, such as helicity, that do not depend on the folding of the entire polypeptide. Further, it is believed that short peptides capable of recognition by helper T-cells are generally amphipathic structures comprising a hydrophobic side (for interaction with the MHC molecule) and a hydrophilic side (for interacting with the T-cell receptor), (Margalit et al., *Computer Prediction of T-cell Epitopes,* New Generation Vaccines Marcel-Dekker, Inc, ed. G.C. Woodrow et al., (1990) pp. 109-116) and further that the amphipathic structures have an α-helical configuration (see, e.g., Spouge et al. *J*. *Immunol.* (1987) 138:204-212; Berkower et al. *J*. *Immunol.* (1986) 136:2498-2503).

Hence, segments of proteins which include T-cell epitopes can be readily predicted using numerous computer programs. (See e.g., Margalit et al., *Computer Prediction of T-cell Epitopes,* New Generation Vaccines Marcel-Dekker, Inc, ed. G.C. Woodrow et al., (1990) pp. 109-116). Such programs generally compare the amino acid sequence of a peptide to sequences known to induce a T-cell response, and search for patterns of amino acids which are believed to be required for a T-cell epitope.

An "immunological response" to a polypeptide or composition is the development in a subject of a humoral and/or a cellular immune response to molecules present in the composition of interest. For purposes of the present invention, a "humoral immune response" refers to an immune response mediated by antibody molecules, while a "cellular immune response" is one mediated by T-lymphocytes and/or other white blood cells. One important aspect of cellular immunity involves an antigen-specific response by cytolytic T-cells ("CTLs"). CTLs have specificity for peptide antigens that are presented in association with proteins encoded by the major histocompatibility complex (MHC) and expressed on the surfaces of cells. CTLs help induce and promote the intracellular destruction of intracellular microbes, or the lysis of cells infected with such microbes. Another aspect of cellular immunity involves an antigen-specific response by helper T-cells. Helper T-cells act to help stimulate the function, and focus the activity of, nonspecific effector cells against cells displaying peptide antigens in association with MHC molecules on their surface. A "cellular immune response" also refers to the production of cytokines, chemoldnes and other such molecules produced by activated T-cells and/or other white blood cells, including those derived from CD4+ and CD8+ T-cells.

A composition, such as an immunogenic composition, or vaccine that elicits a cellular immune response may serve to sensitize a vertebrate subject by the presentation of antigen in association with MHC molecules at the cell surface. The cell-mediated immune response is directed at, or near, cells presenting antigen at their surface. In addition, antigen-specific T-lymphocytes can be generated to allow for the future protection of an immunized host.

The ability of a particular antigen or composition to stimulate a cell-mediated immunological response may be determined by a number of assays, such as by lymphoproliferation (lymphocyte activation) assays, CTL cytotoxic cell assays, or by assaying for T-lymphocytes specific for the antigen in a sensitized subject. Such assays are well known in the art. See, e.g., Erickson et al., *J. Immunol.* (1993) 151:4189-4199; Doe et al., *Eur. J. Immunol.* (1994) 24:2369-2376; and the examples below.

Thus, an immunological response as used herein may be one which stimulates the production of CTLs, and/or the production or activation of helper T-cells. The antigen of interest may also elicit an antibody-mediated immune response. Hence, an immunological response may include one or more of the following effects: the production of antibodies by B-cells; and/or the activation of suppressor T-cells and/or γδ T-cells directed specifically to an antigen or antigens present in the composition or vaccine of interest. These responses may serve to neutralize infectivity, and/or mediate antibody-complement, or antibody dependent cell cytotoxicity (ADCC) to provide protection to an immunized host. Such responses can be determined using standard immunoassays and neutralization assays, well known in the art.

An "immunogenic" polypeptide or composition is one which elicits an immunological response as defined above.

A "recombinant" protein is a protein which retains the desired activity and which has been prepared by recombinant DNA techniques as described herein. In general, the gene of interest is cloned and then expressed in transformed organisms, as described further below. The host organism expresses the foreign gene to produce the protein under expression conditions.

By "isolated" is meant, when referring to a polypeptide, that the indicated molecule is separate and discrete from the whole organism with which the molecule is found in nature or is present in the substantial absence of other biological macromolecules of the same type. The term "isolated" with respect to a polynucleotide is a nucleic acid molecule devoid, in whole or part, of sequences normally associated with it in nature; or a sequence, as it exists in nature, but having heterologous sequences in association therewith; or a molecule disassociated from the chromosome.

"Homology" refers to the percent identity between two polynucleotide or two polypeptide moieties. Two DNA, or two polypeptide sequences are "substantially homologous" to each other when the sequences exhibit at least about 50% , preferably at least about 75%, more preferably at least about 80%-85%, preferably at least about 90%, and most preferably at least about 95%-98% sequence identity over a defined length of the molecules. As used herein, substantially homologous also refers to sequences showing complete identity to the specified DNA or polypeptide sequence.

In general, "identity" refers to an exact nucleotide-to-nucleotide or amino acid-to-amino acid correspondence of two polynucleotides or polypeptide sequences, respectively. Percent identity can be determined by a direct comparison of the sequence information between two molecules by aligning the sequences, counting the exact number of matches between the two aligned sequences, dividing by the length of the shorter sequence, and multiplying the result by 100. Readily available computer programs can be used to aid in the analysis, such as ALIGN, Dayhoff; M.O. in *Atlas of Protein Sequence and Structure* M.O. Dayhoff ed., 5 Suppl. 3:353-358, National biomedical Research Foundation, Washington, DC, which adapts the local homology algorithm of Smith and Waterman *Advances in Appl. Math.* 2:482-489, 1981 for peptide analysis. Programs for determining nucleotide sequence identity are available in the Wisconsin Sequence Analysis Package, Version 8 (available from Genetics Computer Group, Madison, WI) for example, the BESTFIT, PASTA and GAP programs, which also rely on the Smith and Waterman algorithm. These programs are readily utilized with the default parameters recommended by the manufacturer and described in the Wisconsin Sequence Analysis Package referred to above. For example, percent identity of a particular nucleotide sequence to a . reference sequence can be determined using the homology algorithm of Smith and Waterman with a default scoring table and a gap penalty of six nucleotide positions.

Another method of establishing percent identity in the context of the present invention is to use the MPSRCH package of programs copyrighted by the University of Edinburgh, developed by John F. Collins and Shane S. Sturrok, and distributed by IntelliCenetics, Inc. (Mountain View, CA). From this suite of packages the Smith-Waterman algorithm can be employed where default parameters are used for the scoring table (for example, gap open penalty of 12, gap extension penalty of one, and a gap of six). From the data generated the "Match" value reflects "sequence identity." Other suitable programs for calculating the percent identity or similarity between sequences are generally known in the art, for example, another alignment program is BLAST, used with default parameters. For example, BLASTN and BLASTP can be used using the following default parameters: genetic code = standard; filter = none; strand = both; cutoff = 60; expect = 10; Matrix = BLOSUM62; Descriptions = 50 sequences; sort by = HIGH SCORE; Databases = non-redundant, GenBank + EMBL + DDBJ + PDB + GenBank CDS translations + Swiss protein + Spupdate + PIR. Details of these programs can be found at the following internet address: http:l/www.ncbi.nhn.gov/cgi-bin/BLAST.

Alternatively, homology can be determined by hybridization of polynucleotides under conditions which form stable duplexes between homologous regions, followed by digestion with single-stranded-specific nuclease(s), and size determination of the digested fragments. DNA sequences that are substantially homologous can be identified in a Southern hybridization experiment under, for example, stringent conditions, as defined for that particular system. Defining appropriate hybridization conditions is within the skill of the art. See, e.g., Sambrook et al., *supra*; *DNA Cloning, supra; Nucleic Acid Hybridization, supra.*

A "coding sequence" or a sequence which "encodes" a selected polypeptide, is a nucleic acid molecule which is transcribed (in the case of DNA) and translated (in the case of mRNA) into a polypeptide *in vitro* or *in vivo* when placed under the control of appropriate regulatory sequences. The boundaries of the coding sequence are determined by a start codon at the 5' (amino) terminus and a translation stop codon at the 3' (carboxy) terminus. A transcription termination sequence may be located 3' to the coding sequence.

"Operably linked" refers to an arrangement of elements wherein the components so described are configured so as to perform their desired function. Thus, a given promoter operably linked to a coding sequence is capable of effecting the expression of the coding sequence when the proper transcription factors, etc., are present. The promoter need not be contiguous with the coding sequence, so long as it functions to direct the expression thereof. Thus, for example, intervening untranslated yet transcribed sequences can be present between the promoter sequence and the coding sequence, as can transcribed introns, and the promoter sequence can still be considered "operably linked" to the coding sequence.

"Recombinant" as used herein to describe a nucleic acid molecule means a polynucleotide of genomic, cDNA, viral, semisynthetic, or synthetic origin which, by virtue of its origin or manipulation is not associated with all or a portion of the polynucleotide with which it is associated in nature. The team "recombinant" as used with respect to a protein or polypeptide means a polypeptide produced by expression of a recombinant polynucleotide. In general, the gene of interest is cloned and then expressed in transformed organisms, as described further below. The host organism expresses the foreign gene to produce the protein under expression conditions.

A "control element" refers to a polynucleotide sequence which aids in the expression of a coding sequence to which it is linked. The term includes promoters, transcription termination sequences, upstream regulatory domains, polyadenylation signals, untranslated regions, including 5'-UTRs and 3'-UTRs and when appropriate, leader sequences and enhancers, which collectively provide for the transcription and translation of a coding sequence in a host cell.

A "promoter" as used herein is a DNA regulatory region capable of binding RNA polymerase in a host cell and initiating transcription of a downstream (3' direction) coding sequence operably linked thereto. For purposes of the present invention, a promoter sequence includes the minimum number of bases or elements necessary to initiate transcription of a gene of interest at levels detectable above background. Within the promoter sequence is a transcription initiation site, as well as protein binding domains (consensus sequences) responsible for the binding of RNA polymerase. Eucaryotic promoters will often, but not always, contain "TATA" boxes and "CAT" boxes.

A control sequence "directs the transcription" of a coding sequence in a cell when RNA polymerase will bind the promoter sequence and transcribe the coding sequence into mRNA, which is then translated into the polypeptide encoded by the coding sequence.

"Expression cassette" or "expression construct" refers to an assembly which is capable of directing the expression of the sequence(s) or gene(s) of interest. The expression cassette includes control elements, as described above, such as a promoter which is operably linked to (so as to direct transcription of) the sequence(s) or gene(s) of interest, and often includes a polyadenylation sequence as well. Within certain embodiments of the invention, the expression cassette described herein may be contained within a plasmid construct. In addition to the components of the expression cassette, the plasmid construct may also include, one or more selectable markers, a signal which allows the plasmid construct to exist as single-stranded DNA (e.g., a M13 origin of replication), at least one multiple cloning site, and a "mammalian" origin of replication (e.g., a SV40 or adenovirus origin of replication).

''Transformation,'' as used herein, refers to the insertion of an exogenous polynucleotide into a host cell, irrespective of the method used for insertion: for example, transformation by direct uptake, transfection, infection, and the like. For particular methods of transfection, see further below. The exogenous polynucleotide may be maintained as a nonintegrated vector, for example, an episome, or alternatively, may be integrated into the host genome.

A "host cell" is a cell which has been transformed, or is capable of transformation, by an exogenous DNA sequence.

By "nucleic acid immunization" is meant the introduction of a nucleic acid molecule encoding one or more selected antigens into a host cell, for the *in vivo* expression of the antigen or antigens. The nucleic acid molecule can be introduced directly into the recipient subject, such as by injection, inhalation, oral, intranasal and mucosal administration, or the like, or can be introduced *ex vivo,* into cells which have been removed from the host. In the latter case, the transformed cells are reintroduced into the subject where an immune response can be mounted against the antigen encoded by the nucleic acid molecule.

As used herein, "treatment" refers to any of (i) the prevention of infection or reinfection, as in a traditional vaccine, (ii) the reduction or elimination of symptoms, and (iii) the substantial or complete elimination of the pathogen in question. Treatment may be effected prophylactically (prior to infection) or therapeutically (following infection).

By "vertebrate subject" is meant any member of the subphylum cordata, including, without limitation, humans and other primates, including non-human primates such as chimpanzees and other apes and monkey species; farm animals such as cattle, sheep, pigs, goats and horses; domestic mammals such as dogs and cats; laboratory animals including rodents such as mice, rats and guinea pigs; birds, including domestic, wild and game birds such as chickens, turkeys and other gallinaceous birds, ducks, geese, and the like. The term does not denote a particular age. Thus, both adult and newborn individuals are intended to be covered. The invention described herein is intended for use in any of the above vertebrate species, since the immune systems of all of these vertebrates operate similarly.

### II. Modes of Carrying out the Invention

Before describing the present invention in detail, it is to be understood that this invention is not limited to particular formulations or process parameters as such may, of course, vary. It is also to be understood that the terminology used herein is for the purpose of describing particular embodiments of the invention only, and is not intended to be limiting.

Although a number of compositions and methods similar or equivalent to those described herein can be used in the practice of the present invention, the preferred materials and methods are described herein.

As noted above, chimeric antigens which combine HBsAg with portions of HCV proteins enhance the presentation to the immune system of weakly immunogenic HCV proteins due to the strong antigenicity of HBsAg. Virus-like particles typically contain a membrane envelope in which are embedded one or more viral envelope proteins. Virus-like particles are secreted by a cell infected with a virus or a cell transfected with a nucleic acid molecule encoding one or more viral proteins.

Virus-like particles are an especially advantageous form of antigen presentation. Virus-like particles containing HBsAg chimeras combine the highly antigenic nature of HBsAg itself with the desirable presentation of other antigens at the surface of a particulate preparation.

The present invention provides methods and materials which enable the production of chimeric HBV/HCV antigens in the form of virus-like particles for the production of immunogenic compositions, such as vaccines. Utilizing co-expression of HBsAg together with the chimeric antigens, it is now possible to include large segments of HCV envelope glycoproteins fused to HBsAg in the form of virus-like particles. Such particles are especially well suited for use in immunogenic compositions.

The strategy for preparing HBV/HCV virus-like particles depends first on the production of one or more chimeric antigens for display in the particles. Such chimeric antigens are fusion proteins that comprise a substantially complete S domain of an HBsAg polypeptide (termed "sAg" herein) and an immunogenic HCV polypeptide or fragment thereof. An S domain ofHBsAg, or any other polypeptide of the invention, is "substantially complete" if it contains the native sequence of the polypeptide with or without minor deletions of one or a few amino acids from either the N-terminal or C-terminal regions or within the polypeptide. For example the HBsAg S domain can be truncated by a few amino acids, *i*.*e*., up to about 3, 5, 7, or 10 amino acids, without greatly affecting either its antigenicity or ability to cause the formation of virus-like particles. Linker sequences of any desired length, preferably no more than a few amino acids, can be added between the polypeptides joined together to form the fusion protein. Either preS2 (formerly called preS) or both preS2 and preS1 domains of HBsAg can also be included at the amino terminus of the HBsAg polypeptide if desired.

Valenzuela, et al. (1982) *Nature* 298:347-350, describes the gene for HbsAg. See, also, Valenzuela, et al. (1979) *Nature* 280:815-819. Proteins derived from the HBV surface, such as the surface antigen, sAg, as well as the presurface sequences, preS1 and preS2, and any combination of these sequences, can spontaneously form particles upon expression in a suitable host cell, such as upon expression in mammalian, insect, yeast or Xenopus cells. Thus, as explained above, HCV/HBV virus-like particles for use in the present invention can include particle-forming polypeptides of sAg, preS1 and/or preS2, as well as particle-forming polypeptides from any combination of the above, such as sAg/preS1, sAg/preS2, and sAg/preS1/preS2. See, e.g., "HBV Vaccines - from the laboratory to license: a case study" in Mackett, M. and Williamson, J.D., *Human Vaccines and Vaccination,* pp. 159-176, for a discussion of HBV structure; and U.S. Patent Nos. 4,722,840, 5,098,704,5,324,513,5,965,140, Beames et al., *J*. *Virol.* (1995) 69:6833-6838, Birnbaum et al., *J. Virol.* (1990) 64:3319-3330, Zhou et al., *J. Virol.* (1991) 65:5457-5464, for descriptions of the recombinant production of various HBV particles.

HCV has a genome that contains a single open reading frame of approximately 9.5 kb, which is transcribed into a polyprotein. The HCV polyprotein is cleaved to form at least ten distinct products, which are NH₂--Core--E1-E2--p7--NS2--NS3--NS4a-NS4b-NS5a-NS5b-COOH. The HCV E1 and E2 proteins are postranslationally glycosylated. The full-length sequence of the polyprotein is disclosed in European Publ. No. 388,232 and U.S. Patent No. 6,150,087. Moreover, sequences for the above HCV polyprotein products, and immunogenic polypeptides derived therefrom, are known (see, e.g., U.S. Patent No. 5,350,671). For example, a number of general and specific immunogenic polypeptides, derived from the HCV polyprotein, have been described. See, e.g., Houghton et al., European Publ. Nos. 318,216 and 388,232; Choo et al. *Science* (1989) 244:359-362; Kuo et al. *Science* (1989) 244:362-364; Houghton et al. *Hepatology* (1991) 14:381-388; Chien et al. *Proc. Natl. Acad Sci. USA* (1992) 89:10011-10015; Chien et al. *J*. *Gastroent. Hepatol.* (1993) 8:S33-39; Chien et al., International Publ. No. WO 93/00365; Chien, D.Y., International Publ. No. WO 94/01778. These publications provide an extensive background on HCV generally, as well as on the manufacture and uses of HCV polypeptide immunological reagents.

Any desired HCV polypeptide can be utilized as part of the chimeric antigen, including, for example, the E1 and/or E2 envelope glycoproteins of HCV. The E1 glycoprotein corresponds to amino acid residues 192 to 383, and E2 extends from approximately amino acid 384 to amino acid 746 in most HCV strains. *See* Choo *et al.* (1991) Proc. Natl. Acad. Sci. USA 88:2451-2455. Preferred E1 polypeptides are those beginning at any amino acid residue from any of positions 192-360 of the HCV polyprotein and extending any desired length up to and including residue 383. Preferred E2 polypeptides are those beginning at any amino acid residue from positions 384-725 of the HCV polyprotein and extending any desired length up to and including residue 746. A fragment of the E1 or E2 glycoproteins which is utilized in the chimeric antigen should preferably comprise an epitope, domain, or other structural unit which is immunogenic.

An E1 or E2 glycoprotein or fragment thereof for use in a chimeric antigen, will preferably retain or resemble its native conformation. If a substantially native conformation is retained for the HCV polypeptide in the fusion protein containing HBsAg, then antibodies generated to the HCV polypeptide will recognize and bind to the corresponding polypeptide in HCV.

Other HCV polypeptides may also be used in the chimeric antigens of the invention. For example, HCV polypeptides derived from the Core region, such as polypeptides derived from the region found between amino acids 1-191; amino acids 10-53; amino acids 10-45; amino acids 67-88; amino acids 86-100; 81-130; amino acids 121-135; amino acids 120-130; amino acids 121-170; and any of the Core epitopes identified in, e.g., Houghton et al., U.S. Patent No. 5,350,671; Chien et al. *Proc. Natl. Acad. Sci. USA* (1992) 89:10011-10015; Chien et al. *J. Gastroent. Hepatol.* (1993) 8:S33-39; Chien et al., International Publ. No. WO 93/00365; Chien, D.Y., International Publ. No. WO 94/01778; and U.S. Patent No. 6,150,087, will find use with the subject chimeric molecules.

Additionally, polypeptides derived from the nonstructural regions of the virus will also find use herein. The NS3/4a region of the HCV polyprotein has been described and the amino acid sequence and overall structure of the protein are disclosed in Yao et al. *Structure* (November 1999) 7:1353-1363. See, also, Dasmahapatra et al., U.S. Patent No. 5,843,752. As explained above, either the native sequence or immunogenic analogs can be used in the subject formulations. Dasmahapatra et al., U.S. Patent No. 5,843,752 and Zhang et al., U.S. Patent No. 5,990,276, both describe analogs of NS3/4a and methods ofmaking the same.

Additionally, multiple epitope fusion antigens (termed "MEFAs"), as described in International Publ. No. WO 97/44469, may be used in the subject chimeras. Such MEFAs include multiple epitopes derived from two or more of the various viral regions. The epitopes are preferably from more than one HCV strain, thus providing the added ability to protect against multiple strains of HCV in a single vaccine.

Moreover, polypeptides for use in the subject chimeras may be derived from the NS3 region of the HCV polyprotein. A number of such polypeptides are known, including, but not limited to polypeptides derived from the c33c and c100 regions, as well as fusion proteins comprising an NS3 epitope, such as c25. These and other NS3 polypeptides are useful in the present compositions and are known in the art and described in, e.g., Houghton et al, U.S. Patent No. 5,350,671; Chien et al. *Proc. Natl.* *Acad. Sci. USA* (1992) 89:10011-10015; Chien et al. *J. Gastroent. Hepatol.* (1993) 8:S33-39; Chien et al., International Publ. No. WO 93/00365; Chien, D.Y., International Publ. No. WO 94/01778; and U.S. Patent No. 6,150,087.

It is readily apparent that a multitude ofHCV polypeptides may be used in the subject chimeric molecules or may be coadministered therewith, in order to provide an immune response against the HCV antigen in question.

As explained above, the HCV antigens may be used in their entireties or immunogenic fragments thereof, as well as immunogenic variants, can be combined with an HBsAg polypeptide to form the chimeric antigen by fusion of the gene sequences encoding the desired polypeptide sequences, in proper reading frame, using standard techniques available in the art. Thus, the HBV or HCV polypeptides described for use in the invention can be modified by deletions, insertions, or conservative amino acid substitutions, provided that a substantially native conformation is retained for the HCV epitope intended for use as an immunogen. Preferably, the HCV polypeptide or polypeptide fragment is chosen so that, when expressed in a fusion protein with HBsAg, it substantially retains the native conformation of the corresponding portion of the HCV protein from which it was derived, *i*.*e*., the approximate conformation existing in a mature HCV virion.

It should be noted that for convenience, the various HCV regions are generally defined with respect to the amino acid number relative to the polyprotein encoded by the genome of HCV-1a, as described in Choo et al. (1991) *Proc Natl Acad Sci USA* 88:2451, with the initiator methionine being designated position 1. However, the polypeptides for use with the present invention are not limited to those derived from the HCV-1a sequence. Any strain or isolate of HCV can serve as the basis for providing antigenic sequences for use with the invention. In this regard, the corresponding regions in another HCV isolate can be readily determined by aligning sequences from the two isolates in a manner that brings the sequences into maximum alignment.

Various strains and isolates of HCV are known in the art, which differ from one another by changes in nucleotide and amino acid sequence. For example, isolate HCV J1.1 is described in Kubo *et al.* (1989) Japan. Nucl. Acids Res. 17:10367-10372; Takeuchi *et al*. (1990) Gene 91:287-291; Takeuchi et *al*. (1990) J. Gen. Virol. 71:3027-3033; *and Takeuchi et al.* (1990) Nucl. Acids Res. 18:4626. The complete coding sequences of two independent isolates, HCV-J and BK, are described by Kato *et al*., (1990) Proc. Natl. Acad. Sci. USA 87:9524-9528 and Takamizawa *et al*., (1991) J. Virol. 65:1105-1113, respectively. HCV-1 isolates are described by Choo et *al*. (1990) Brit. Med. Bull. 46:423-441; Choo *et al.* (1991) Proc. Natl. Acad. Sci. USA 88:2451-2455 and Han *et al.* (1991) Proc. Natl. Acad. Sci. USA 88:1711-1715. HCV isolates HC-J1 and HC-J4 are described in Okamoto *et al.* (1991) Japan J. Exp. Med. 60:167-177. HCV isolates HCT 18-, HCT 23, Th, HCT 27, EC1 and EC10 are described in Weiner *et al.* (1991) Virol. 180:842-848. HCV isolates Pt-1, HCV-K1 and HCV-K2 are described in Enomoto *et al.* (1990) Biochem. Biophys. Res. Commun. 170:1021-1025. HCV isolates A, C, D & E are described in Tsukiyama-Kohara *et al.* (1991) Virus Genes 5:243-254.

Coding sequences for naturally occurring HCV polypeptides for use in the nucleic acid molecules or vectors of the invention can be obtained from any of the above cited strains of HCV or from newly discovered isolates isolated from tissues or fluids of infected patients. In choosing HCV polypeptides or their fragments from different HCV strains for use in a chimeric antigen of the invention, it is preferred that the nucleotide or amino acid sequences be aligned for maximum overlap between strains. This can be accomplished, for example, by compensating for insertions or deletions of amino acids so that the greatest possible overlap between strains is obtained prior to selecting a sequence for incorporation in the chimeric antigen. If a sequence disclosed herein is selected from another HCV strain, then preferably the corresponding sequence, *i*.*e*., the sequence which is aligned to be identical at the greatest possible number of residues to the disclosed sequence, will be selected. Modified sequences which do not occur in nature can also be used. A "nucleic acid molecule" according to the invention can be any nucleic acid such as DNA or RNA, either single or double stranded, or any analog or chemical derivative thereof which encodes a fusion protein of the invention or portion thereof or any HBsAg or portion thereof.

Nucleic acid molecules of the invention can be cloned into expression vectors and transformed into, for example, bacterial, yeast, plant, insect, or mammalian cells so that the chimeric antigens and virus-like particles of the invention can be expressed in and isolated from cell culture. Nucleic acid molecules can be contained within a plasmid, such as pBR322, pUC, ColE1, or related plasmids such as pCMV6a (see U.S. Patent 5,688,688) or plasmids derived therefrom. Nucleic acid molecules can also be contained within a viral vector, such as any vector derived from adenovirus, Sindbis virus, simian virus 40, cytomegalovirus, and retroviruses such as murine sarcoma virus, mouse mammary tumor virus, Moloney murine leukemia virus, and Rous sarcoma virus. Bacterial vectors, such as *Salmonella ssp., Yersinia enterocolitica, Shigella spp., Vibrio cholerae, Mycobacterium* strain BCG, and *Listeria monocytogenes* can also be used. Minichromosomes such as MC and MC1, bacteriophages, cosmids, and replicons can be used as well.

Any suitable expression vector can be constructed or utilized to express any form of HBsAg or any chimeric antigen of the invention. A preferred vector is pCMVII, a pUC19-based cloning vector designed for expression in mammalian cells. pCMVII comprises the following elements: human CMV IE enhancer/promoter, human CMV intron A, a human tissue plasminogen activator (tPA) leader, a bovine growth hormone poly A terminator (BGHt), a ColE1 origin of replication, and an *Amp R* ampicillin resistance gene (Figs. 1A and 1B-1F, SEQ ID NO:1). pCMVII-pS2-sAg can be used for expression of preS2-HBsAg (Figs. 2A and 2B-2I, SEQ ID NOS:2 and 3). In pCMVII-pS2-sAg, the coding sequences for the preS2 and S domains ofHBsAg have been inserted into pCMVII between CMV intron A and BGHt; this vector can also be modified by adding the coding sequences for the preS1 domain or removing the preS2 domain. Chimeric antigens containing HBsAg together with epitopes ofHCV proteins can be constructed similar to pCMVII opti 330 E1/sAg (Figs. 3A and 3B-3I, SEQ ID NOS:4 and 5) or pCMVII-E2661-sAg (Figs. 4A and 4B-4F, SEQ ID NOS:6 and 7). These vectors are provided by way of example and are not intended to limit the scope of the invention. Isolated and purified pCMVII vectors containing an insert encoding HBsAg or a chimeric antigen can be dissolved in sterile 0.9% saline buffer or another suitable buffer prior to use.

The expression of chimeric antigens for use as immunogens requires transfection of eukaryotic cells with an expression vector. Any suitable eukaryotic cell line can be used, for example CHO cells or COS cells. A nucleic acid molecule that encodes a chimeric antigen can be incorporated into any vector suitable for transfection, for example a plasmid vector or a viral vector. If a viral vector is used, it will preferably produce defective, non-infectious viral particles so that the risk of contamination with infectious virus in a vaccine preparation is minimized.

Transfection can be performed by any known method and can result in either transient transfection or stable transfection. Stable transfection is preferred to establish a cell line producing HBV/HCV virus-like particles. Methods for obtaining stable transfection are well known and include, for example, selection for spontaneously stable transfectants, transfection with immortalizing genes (see, *e.g.,* Katakura et al., Methods Cell Biol. (1998) 57:69-91), and selection for genes providing resistance to antibiotics such as hygromycin B and neomycin. A preferred method for CHO cells involves selection for cells transfected with dihydrofolate reductase followed by amplification of the transgene using methotrexate (see Wurm et al., Ann. N.Y. Acad. Sci. (1996) 782:70-78).

Co-expression of chimeric antigens with HBsAg results in formation and secretion of virus-like particles. Optimum secretion of virus-like particles requires a sufficient quantity of expression of HBsAg in relation to the expression of chimeric antigen, and higher quantities ofHBsAg expression generally improve the efficiency of chimeric antigen secretion. Secretion of chimeric antigen as virus-like particles can be optimized by varying the ratio of the coding sequences for HBsAg to the coding sequences for the chimeric antigen. Generally, useful secretion of virus-like particles will be obtained at a ratio of HBsAg coding sequences to chimeric antigen coding sequences of about 1:1,2:1,3:1,4:1,5:1,7:1,10:1,20:1,30:1,40:1,50:1, or 100:1. Ratios less than 1:1 provide reduced yield of virus-like particles containing chimeric antigen, while high ratios greater than 100:1 dilute out the chimeric antigen and eventually will limit the utility of the particles because of dilution of the chimeric antigen with HBsAg.

One strategy for transfecting host cells with the vectors of the invention, either for in vitro production of virus-like particles or for immunization of a host organism using a nucleic acid vaccine, is to provide two or more separate vectors, one that encodes HBsAg and one or more that each encode a chimeric antigen. Another strategy is to include both HBsAg and one or more chimeric antigens in a single construct. For example, an expression vector having a single open reading frame could have a coding sequence for HBsAg under control of a promoter, followed by sequences encoding one or more chimeric antigens, each preceded by an internal ribosomal entry site (IRES, see, *e.g.,* Martinez-Salas (1999) Curr. Op. Biotechnol. 10: 458-464). Thus, the invention encompasses the use either of a single immunogenic polypeptide or multiple distinct immunogenic polypeptides in a single virus-like particle. Optionally, expression of immunogenic polypeptides can be regulated by including promoter and/or enhancer sequences in the expression vector which respond to activators introduced into the transfected cells.

Cells that have been transfected with plasmid or viral vectors expressing chimeric HBV/HCV antigens together with HBsAg can be analyzed to determine whether each antigen is being expressed and secreted in the form of virus-like particles. Any standard immunological assay can be used to detect HBV and HCV antigens, including, for example, chemiluminescence assays (*see, e*.*g*., Magic Lite assay in Examples 1-4; Woodhead, J.S. and Weeks, I. (1989) J. Biolumin. Chemilumin. 4:611-14), ELISA, and radioimmunoassays. Where an antigen is produced by transfected cells in culture, the amount of secretion of the antigen into the culture medium can be ascertained by comparing the amount of antigen in the culture medium with the amount remaining in lysed cells. The presence of virus-like particles can be confirmed by sedimentation of such particles from the culture medium into a density gradient, for example a sucrose density gradient (see Examples 1-3).

While expression in a mammalian cell line is preferred, other systems may also be employed to express virus-like particles of the invention. For example, yeast cell culture may be used (*see* U.S. Patent 5,098,704), in which case virus-like particles can be harvested by disrupting the cells using agitation with glass beads or another suitable method.

Generally, the proteins of the subject invention will naturally aggregate to form particles in the expression host. The particles may be enveloped having a lipid membrane coat, which may or may not include membrane proteins encoded by the virus. Alternatively, the particles may not include the lipid membrane or the membrane may be present initially or may be removed, in whole or in part. U.S. Patent Nos. 4,722,840 and 5,965,140, describe hybrid particles comprised of a particle-forming fragment of a structural protein from a virus, such as a particle-forming fragment of hepatitis B virus (HBV) surface antigen (HBsAg), fused to a heterologous polypeptide.

HBV/HCV virus-like particles can be purified after being harvested from a culture medium or cell suspension and before being used in an immunogenic composition. Any method can be used that is known to separate virus-like particles or viruses from surrounding proteins, lipids, nucleic acids, membranes, intact cells, and the like. Especially preferred are affinity chromatography methods; for example, an immobilized monoclonal antibody specific for HBsAg can be used. Additional suitable methods are gel filtration chromatography, ion exchange chromatography, and density gradient sedimentation. Methods for isolated chimeric virus-like particles are described in, e.g., U.S. Patent Nos. 4,722,840 and 5,965,140.

Any composition of the invention, such as a virus-like particle, or a nucleic acid molecule, can be used as an "immunogenic composition." An immunogenic composition preferably generates an immune response, as defined above, such as an antibody or a T-cell response, in a mammal to whom it is administered. An immunogenic composition of the invention can be, but is not limited to, a vaccine or a combination vaccine, *e.g.*, an immunogenic composition that produces an immune response to more than one immunogen. Moreover, the chimeric proteins of the invention may be formulated into "antigenic compositions," e.g., compositions that include epitopes to which a specific antibody molecule or specific cell surface receptor binds.

Immunogenic and antigenic compositions containing HBV/HCV virus-like particles or a nucleic acid molecule encoding proteins which form virus-like particles can be administered to a mammal, such as a mouse, rabbit, baboon, chimpanzee, or human, to elicit anti-HCV antibodies in vivo. Injection of an immunogenic composition of the invention preferably results in the synthesis of virus-like particles in the host. Therefore, Compositions comprising nucleic acids should include sequences encoding both HBsAg and chimeric antigens such as HBsAg-E1 and/or HBsAg-E2 fusion proteins, as well as fusions with other HCV polypeptides. The weight:weight ratio of nucleic acids encoding HBsAg to nucleic acids encoding chimeric antigens is preferably 1:1, 2-1, 5:1, 10:1, 20:1, 30:1, 50: 1, or 100:1 and results in the formation of virus-like particles in the cells of the host and their secretion within the host. More preferably, the ratio is in the range of 5:1 to 20:1.

Virus-like particles or nucleic acid molecules of an antigenic or immunogenic composition can be combined with adjuvants, immunostimulatory molecules, or carriers, including but not limited to, MF59 (described below), poly(dl-lactide-co-glycolide) microparticles (PLG, *see, e.g*., Delgado et al. (1999) Vaccine 17: 2927-2938), LT toxins, immune stimulating complexes (ISCOMS, *see,* e.g., Mowat et al. (1999) Immunol. Lett. 65: 133-140), and QS21 (see Singh & O'Hagan (1999) Nat. Biotechnol. 17: 1075-1081).

For example, preferred adjuvants to enhance effectiveness of the composition include, but are not limited to: (1) aluminum salts (alum), such as aluminum hydroxide, aluminum phosphate, aluminum sulfate, etc; (2) oil-in-water emulsion formulations (with or without other specific immunostimulating agents such as muramyl peptides (see below) or bacterial cell wall components), such as for example (a) MF59 (PCT Publ. No. WO 90/14837), containing 5% Squalene, 0.5% Tween 80, and 0.5% Span 85 (optionally containing various amounts of MTP-PE (see below), although not required) formulated into submicron particles using a microfluidizer such as Model 110Y microfluidizer (Microfluidics, Newton, MA), (b) SAF, containing 10% Squalane, 0.4% Tween 80, 5% pluronic-blocked polymer L121, and thr-MDP (see below) either microfluidized into a submicron emulsion or vortexed to generate a larger particle size emulsion, and (c) Ribi^{TM} adjuvant system (RAS), (Ribi Immunochem, Hamilton, MT) containing 2% Squalene, 0.2% Tween 80, and one or more bacterial cell wall components from the group consisting of monophosphorylipid A (MPL), trehalose dimycolate (TDM), and cell wall skeleton (CWS), preferably MPL + CWS (Detox^{TM}); (3) saponin adjuvants, such as Stimulon^{TM} (Cambridge Bioscience, Worcester, MA) may be used or particles generated therefrom such as ISCOMs (immunostimulating complexes); (4) Complete Freund's Adjuvant (CFA) and Incomplete Freund's Adjuvant (IFA); (5) cytokines, such as interleukins (e.g., IL-1, IL-2, IL-4, IL-5, IL-6, IL-7, IL-12, *etc.*), interferons (e.g., gamma interferon), macrophage colony stimulating factor (M-CSF), tumor necrosis factor (TNF), etc; (6) detoxified mutants of a bacterial ADP-ribosylating toxin such as a cholera toxin (CT), a pertussis toxin (PT), or an E. coli heat-labile toxin (LT), particularly LT-K63, LT-R72, CT-S109, PT-K9/G129; see, e.g., WO 93/13302 and WO 92/19265; (7) other substances that act as immunostimulating agents to enhance the effectiveness of the composition; and (8) microparticles with adsorbed macromolecules, as described in copending U.S. Patent Application Serial No. 09/285,855 (filed April 2,1999) and international Patent Application Serial No. PCT/US99/17308 (filed July 29,1999). Alum and MF59 are preferred.

As mentioned above, muramyl peptides include, but are not limited to, N-acetyl-muramyl-L-threonyl-D-isoglutamine (thr-MDP), N-acetyl-normuramyl-L-alanyl-n-isoglutamine (nor-MDP), N-acetyhnuramyl-L-alanyl-D-isoglutaminyl-L-alanine-2-(1'-2'-dipalmitoyl-sn-glycero-3-hydroxyphosphoryloxy)-ethylamine (MTP-PE), *etc.*

An immunogenic composition may also comprise a pharmaceutically acceptable carrier. Pharmaceutically acceptable carriers are well known to those in the art. Such carriers include, but are not limited to, large, slowly metabolized macromolecules, such as proteins, polysaccharides, polylactic acids, polyglycolic acids, polymeric amino acids, amino acid copolymers, and inactive virus particles. Liposomes, such as those described in U.S. 5,422,120, WO 95/13796, WO 91/14445, or EP 524,968 B1, as well as poly(dl-lactide-co-glycolide) microparticles (PLG, *see,* e.g., Delgado et al. (1999) Vaccine 17: 2927-2938), can also be used as a carrier for a composition of the invention.

Pharmaceutically acceptable salts can also be used in compositions of the invention, for example, mineral salts such as hydrochlorides, hydrobromides, phosphates, or sulfates, as well as salts of organic acids such as acetates, proprionates, malonates, or benzoates.

Compositions of the invention generally contain pharmaceutically acceptable excipients, such as water, saline, glycerol, and ethanol, as well as substances such as wetting agents, emulsifying agents, or pH buffering agents.

The chimeric molecules of the present invention may be used for nucleic acid immunization, to generate an appropriate immune response, such as to activate HCV-specific T cells, using standard gene delivery protocols. Any method known in the art can be employed to package and deliver nucleic acid molecules of the invention, including nucleic acid molecules in an immunogenic composition. Methods for gene delivery are known in the art. See, e.g., U.S. Patent Nos. 5,399,346, 5,580,859, 5,589,466. For example, the coding sequences can be packaged in a viral vector, combined with lipid, peptoid excipients, PLG formulations, or gold particles. Preferably, an immunogenic composition is delivered as naked DNA or naked RNA. The expressed immunogenic polypeptide is preferably presented to the host immune system with native post-translational modifications, structure, and conformation.

For example, the constructs can be packaged in liposomes prior to delivery to the cells. Lipid encapsulation is generally accomplished using liposomes which are able to stably bind or entrap and retain nucleic acid. The ratio of condensed DNA to lipid preparation can vary but will generally be around 1:1 (mg DNA:micromoles lipid), or more of lipid. For a review of the use of liposomes as carriers for delivery of nucleic acids, see, Hug and Sleight, *Biochim. Biophys. Acta.* (1991) 1097:1-17; Straubinger et al., in *Methods of Enzymology* (1983), Vol. 101, pp. 512-527.

Liposomal preparations for use with the present invention include cationic (positively charged), anionic (negatively charged) and neutral preparations, with cationic liposomes particularly preferred. Cationic liposomes are readily available. For example, N[1-2,3-dioleyloxy)propyl]-N,N,N-triethylammonium (DOTMA) liposomes are available under the trademark Lipofectin, from GIBCO BRL, Grand Island, NY. (See, also, Felgner et al., *Proc Natl. Acad. Sci. USA* (1987) 84:7413-7416). Other commercially available lipids include transfectace (DDAB/DOPE) and DOTAP/DOPE (Boerhinger). Other cationic liposomes can be prepared from readily available materials using techniques well known in the art. See, e.g., Szoka et al., *Proc. Natl. Acad. Sci. USA* (1978) 75:4194-4198; PCT Publication No. WO 90/11092 for a description of the synthesis of DOTAP (1,2-bis(oleoyloxy)-3-(trimethylammonio)propane) liposomes. The various liposome-nucleic acid complexes are prepared using methods known in the art. See, e.g., Straubinger et al., in METHODS OF IMMUNOLOGY (1983), Vol. 101, pp. 512-527; Szoka et al., *Proc. Natl. Acad. Sci. USA* (1978) 75:4194-4198; Papahadjopoulos et al., *Biochim. Biophys. Acta* (1975) 394:483; Wilson et al., *Cell* (1979) 17:77); Deamer and Bangham, *Biochim. Biophys. Acta* (1976) 443:629; Ostro et al., *Biochem. Biophys. Res. Commun.* (1977) 76:836; Fraley et al., *Proc. Natl. Acad Sci. USA* (1979) 76:3348); Enoch and Strittmatter, *Proc. Natl. Acad. Sci. USA* (1979) 76:145); Fraley et al., *J. Biol. Chem.* (1980) 255:10431; Szoka and Papahadjopoulos, *Proc. Natl. Acad. Sci. USA* (1978) 75:145; and Schaefer-Ridder et *al*., *Science* (1982) 215:166.

The DNA can also be delivered in cochleate lipid compositions similar to those described by Papahadjopoulos et al., *Biochem. Biophys. Acta.* (1975) 394:483-491. See, also, U.S. Patent Nos. 4,663,161 and 4,871,488.

A number of viral based systems have been developed for gene transfer into mammalian cells. For example, retroviruses provide a convenient platform for gene delivery systems, such as murine sarcoma virus, mouse mammary tumor virus, Moloney murine leukemia virus, and Rous sarcoma virus. A selected gene can be inserted into a vector and packaged in retroviral particles using techniques known in the art. The recombinant virus can then be isolated and delivered to cells of the subject either *in vivo* or *ex vivo.* A number of retroviral systems have been described (U.S. Patent No. 5,219,740; Miller and Rosman, *BioTechniques* (1989) 7:980-990; Miller, A.D., *Human Gene Therapy* (1990) 1:5-14; Scarpa et al., *Virology* (1991) 180:849-852; Bums et al., *Proc. Natl. Acad. Sci. USA* (1993) 90:8033-8037; and Boris-Lawrie and Temin, *Cur. Opin. Genet. Develop.* (1993) 3:102-109. Briefly, retroviral gene delivery vehicles of the present invention may be readily constructed from a wide variety of retroviruses, including for example, B, C, and D type retroviruses as well as spumaviruses and lentiviruses such as FIV, HIV, HIV-1, HIV-2 and SIV (see RNA Tumor Viruses, Second Edition, Cold Spring Harbor Laboratory, 1985). Such retroviruses may be readily obtained from depositories or collections such as the American Type Culture Collection ("ATCC"; 10801 University Blvd., Manassas, VA 20110-2209), or isolated from known sources using commonly available techniques.

A number of adenovirus vectors have also been described, such as adenoviras Type 2 and Type 5 vectors. Unlike retroviruses which integrate into the host genome, adenoviruses persist extrachromosomally thus minimizing the risks associated with insertional mutagenesis (Haj-Ahmad and Graham, *J*. *Virol.* (1986) 57:267-274; Bett et al., *J. Virol.* (1993) 67:5911-5921; Mittereder et al., *Human Gene Therapy* (1994) 5:717-729; Seth et al., *J. Virol.* (1994) 68:933-940; Barr et al., *Gene Therapy* (1994) 1:51-58; Berkner, K.L. *BioTechniques* (1988) 6:616-629; and Rich et al., *Human Gene Therapy* (1993) 4:461-476).

Molecular conjugate vectors, such as the adenovirus chimeric vectors described in Michael et al., *J. Biol. Chem.* (1993) 268:6866-6869 and Wagner et al., *Proc. Natl. Acad. Sci. USA* (1992) 89:6099-6103, can also be used for gene delivery.

Members of the Alphavirus genus, such as but not limited to vectors derived from the Sindbis and Semliki Forest viruses, VEE, will also find use as viral vectors for delivering the gene of interest. For a description of Sindbis-virus derived vectors useful for the practice of the instant methods, see, Dubensky et al., *J*. *Virol.* (1996) 70:508-519; and International Publication Nos. WO 95/07995 and WO 96/17072.

Other vectors can be used, including but not limited to simian virus 40, cytomegalovirus. Bacterial vectors, such as Salmonella ssp. *Yersinia enterocolitica,* Shigella spp., *Vibrio cholerae,* Mycobacterium strain BCG, and *Listeria monocytogenes* can be used. Minichromosomes such as MC and MC1, bacteriophages, cosmids (plasmids into which phage lambda cos sites have been inserted) and replicons (genetic elements that are capable of replication under their own control in a cell) can also be used.

The chimeric constructs may also be encapsulated, adsorbed to, or associated with, particulate carriers. Such carriers present multiple copies of a selected molecule to the immune system and promote trapping and retention of molecules in local lymph nodes. The particles can be phagocytosed by macrophages and can enhance antigen presentation through cytokine release. Examples of particulate carriers include those derived from polymethyl methacrylate polymers, as well as microparticles derived from poly(lactides) and poly(lactide-co-glycolides), known as PLG. See, e.g., Jeffery et al., *Pharm. Res.* (1993) 10:362-368; and McGee et al., *J*. *Microencap.* (1996).

A wide variety of other methods can be used to deliver the constructs to cells. Such methods include DEAE dextran-mediated transfection, calcium phosphate precipitation, polylysine- or polyomithine-mediated transfection, or precipitation using other insoluble inorganic salts, such as strontium phosphate, aluminum silicates including bentonite and kaolin, chromic oxide, magnesium silicate, talc, and the like. Other useful methods of transfection include electroporation, sonoporation, protoplast fusion, liposomes, peptoid delivery, or microinjection. See, e.g., Sambrook et al., *supra,* for a discussion of techniques for transforming cells of interest; and Felgner, P.L., *Advanced Drug Delivery Reviews* (1990) 5:163-187, for a review of delivery systems useful for gene transfer. One particularly effective method of delivering DNA using electroporation is described in International Publication No.

### WO/0045823.

Additionally, biolistic delivery systems employing particulate carriers such as gold and tungsten, are useful for delivering the constructs of the present invention. The particles are coated with the construct to be delivered and accelerated to high velocity, generally under a reduced atmosphere, using a gun powder discharge from a "gene gun." For a description of such techniques, and apparatuses useful therefore, see, e.g., U.S. Patent Nos. 4,945,050; 5,036,006; 5,100,792; 5,179,022; 5,371,015; and 5,478,744..

The chimeric constructs can be delivered either directly to the vertebrate subject or, alternatively, delivered *ex vivo,* to cells derived from the subject and the cells reimplanted in the subject. For example, the constructs can be delivered as plasmid DNA, e.g., contained within a plasmid, such as pBR322, pUC, or ColE1.

An immunogenic composition of the invention is administered in a manner compatible with the particular composition used and in an amount which is effective to elicit an anti-HCV polypeptide antibody titer, such as an anti-E2 or anti-E1antibody titer. Administration can be by any means known in the art, including intramuscular, intradermal, intraperitoneal, or subcutaneous injection, including injection using a biological ballistic gun ("gene gun"). Electroporation or iontophoresis can also be used. Administration may also be intranasal or oral. For oral administration of an immunogenic composition, a protein carrier is preferably included. An immunogenic composition, including compositions comprising naked DNA or RNA, is preferably injected intramuscularly to a large mammal, such as a baboon, chimpanzee, or human, at a dose of 0.5, 0.75, 1.0, 1.5, 2.0, 2.5, 5 or 10 mg/kg. A composition comprising virus-like particles is preferably injected intramuscularly to a large mammal, such as a human, at a dose of 0.01, 0.05, 0.5, 0.75, 1.0, 1.5, 2.0, 2.5, 5 or 10 mg proteinlkg body weight.

Direct delivery of the compositions will generally be accomplished by injection, either subcutaneously, intraperitoneally, intravenously or intramuscularly or delivered to the interstitial space of a tissue. The compositions can also be administered into a lesion. Other modes of administration include oral, intranasal, and pulmonary administration, suppositories, and transdermal or transcutaneous applications (*e.g.,* see WO98/20734), needles, and gene guns or hyposprays. Dosage treatment may be a single dose schedule or a multiple dose schedule. The composition may be administered in conjunction with other immunoregulatory agents.

Methods for the *ex vivo* delivery and reimplantation of transformed cells into a subject are known in the art and described in e.g., WO93/14778. Examples of cells useful in *ex vivo* applications include, for example, stem cells, particularly hematopoetic, lymph cells, macrophages, dendritic cells, or tumor cells. Generally, delivery of nucleic acids for both *ex vivo* and *in vitro* applications can be accomplished by the following procedures, for example, dextran-mediated transfection, calcium phosphate precipitation, polybrene mediated transfection, protoplast fusion, electroporation, encapsulation of the polynucleotide(s) in liposomes, and direct microinjection of the DNA into nuclei, all well known in the art.

Immunogenic compositions comprising either HBV/HCV virus-like particles or nucleic acids encoding and expressing such virus-like particles can be administered either to a mammal that is not infected with HCV or can be administered to an HCV-infected mammal. The particular dosages of virus-like particles or nucleic acids will depend on a number of factors including, but not limited to, the species, age, and general condition of the mammal to which the composition is administered and the mode of administration of the composition. An effective amount of the composition of the invention can be readily determined using routine experimentation. *In vivo* models are well known and can be employed to identify appropriate doses. If desired, co-stimulatory molecules or adjuvants can also be provided before, after, or together with the immunogenic compositions. Immunogenic compositions can be administered more than once, as required for effective immunization. Administration of immunogenic compositions containing virus-like particles can be performed either before or after administration of immunogenic compositions containing nucleic acids, such that one form (protein or nucleic acid) provides a primary immunization and the other form boosts the immune response.

### III. Experimental

Below are examples of specific embodiments for carrying out the present invention. The examples are offered for illustrative purposes only, and are not intended to limit the scope of the present invention in any way. Those of skill in the art will readily appreciate that the invention may be practiced in a variety of ways given the teaching of this disclosure.

Efforts have been made to ensure accuracy with respect to numbers used (e.g., amounts, temperatures, etc.), but some experimental error and deviation should, of course, be allowed for.

### EXAMPLE 1

### Detection of HBV Virus-Like Particles Using Sucrose Density Gradient Sedimentation

Purified recombinant HBsAg particles were obtained in the form of a hepatitis B vaccine from Chiron Corp. Clinical Department Two hundred fifty µL of a 37 µg/mL suspension in 0.03 M citrate, 0.26 M NaCl, 0.005% polysorbate 80 was loaded onto a 5-30% (wt-vol) sucrose gradient and centrifuged 4 hrs at 40,000 rpm in a Beckman SW41 rotor. Ten fractions were removed and assayed for HBsAg by the Magic Lite Assay. The results are shown in Fig. 5.

### Magic Lite Assay

This describes a highly sensitive immunochemiluminescence assay used to detect HBV and HCV antigens. One hundred µL of sample was added to a 12 x 75 mm polystyrene tube. One hundred µL (30 µg) of a "capture" antibody was added to the sample. The capture antibody was covalently linked to paramagnetic particles by glutaraldehyde crosslinking a mixture of 6:1 paramagnetic particles:antibody. The tubes were vortexed and incubated in a 37°C water bath for 20 minutes. Next the tubes were placed on a magnetic rack where the paramagnetic particles (PMP) can aggregate. This allowed for washing and decanting. After the third wash, 100 µL of a "detecting" antibody, which was covalently linked to dimethylacridinium, was added and the tube was vortexed. The tubes were again incubated at 37°C for 20 minutes and then placed on a magnetic rack for washing and decanting. After the third wash, the tubes were placed in a Ciba Coming Magic Lite chemiluminometer to measure the analyte bound. Results were expressed in arbitrary units of light intensity (relative light units, RLU).

### Antibodies

Anti-sAg was provided by Chiron Diagnostics (Walpole, MA) and was directed against serotypes AYW and ADW. MAb 5E5/H7 was obtained from mice using HeLa E1/E2 amino acids 1-967 as an immunogen and recognizes the E2 epitope 384-655. MAb 3D5/C3 was also obtained from mice immunized with HeLa E1/E2 amino acids 1-967 and recognizes E1 epitope 211-217.

### EXAMPLE 2

### Expression of HBV/HCV Virus-Like Particles Containing Chimeric HBsAg-E2-661 Antigen in COS7 Cells

Five µg of pCMV-II-E2661-sAg (Fig. 4A) and increasing amounts of pCMV-II-pS2-sAg (Fig. 2A) were prepared. Expression from pCMV-II-pS2-sAg resulted in a mixture of about 5-20% preS2-S-polypeptide, with the remainder being S-polypeptide. Ratios of sAg to E2661-sAg plasmids used were 0:1,1:1, 5:1, and 10:1 on a µg DNA basis. The total amount of DNA in each tube was normalized to 55 µg by the addition of pCMV-km-Bgal. This mixture was transfected into COS7 cells using the LT1 transfection reagent from Panvera, as described below. At 48 hours post-transfection, media and soluble lysates (obtained by incubating cell monolayers in PBS with 0.1% NP40 and centrifuging to remove insoluble debris) were removed and assayed by the Magic Lite Assay, with capture by anti-sAg followed by detection with a conjugated anti-E2 MAb (5E5/H7, see Example 1) (Fig. 6A) or MAb sAg (Fig. 6B). Both E2 and sAg were increasingly secreted into the medium as the ratio of sAg:E2661-sAg was increased, with an optimum secretion at ratios in the range of 5:1 to 10:1.

In order to characterize the virus-like particles, 1 mL of culture medium from each condition was loaded onto a 5-30% sucrose gradient. The samples were centrifuged 4 hrs at 40,000 rpm using a Beckman SW41 rotor. Eleven fractions were removed and assayed for E2 (Fig. 7A) or sAg (Fig. 7B) by the Magic Lite Assay. Both E2 and sAg were observed in highest amount in fraction 4, which is the same gradient position for the peak distribution of HBsAg-containing virus-like particles (Fig. 5).

### Transfection Protocol

Cells were seeded in 6-well plates the day prior to transfection so as to achieve 50-60% confluency at the time of transfection. Opti-mem (100 µL) and LT-1 transfection reagent (12 µL) were added to a sterile polypropylene tube and incubated at room temperature for five minutes. Three µg of DNA was then added to the tube, which was incubated another five minutes at room temperature. During this incubation period, the cells were washed with Opti-mem (2 mLs per well). Two mLs Opti-mem were added to each well, followed by 100 µL of the Opti-mem/LT-1/DNA mixture. The cells were then incubated for four hours at 37°C, followed by aspiration and addition of culture medium (1.5 mL/well of DMEM + 10% FBS). At 48 hours post transfection, the medium was harvested and the cell monolayers were solubilized using PBS containing 0.1% NP-40. Both the harvested medium and the solubilized cells were centrifuged to remove debris.

### EXAMPLE 3

### Expression of HBV/HCV Virus-Like Particles Containing Chimeric HBsAg-E1-330 Antigen in COS7 Cells

Mixtures of pCMV-II-optiE1330-sAg (2.5 µg DNA) and various amounts of pCMV-II-pS2-sAg were prepared to provide ratios of sAg plasmid to E1-sAg plasmid of 0:1 (control), 1:1,5:1,10:1, and 20:1. Expression from pCMV-II-pS2-sAg resulted in a mixture of about 5-20% preS2-S-polypeptide, with the remainder being S-polypeptide. The total amount of DNA in each tube was normalized to 52.5 µg by the addition of pCMV-km-βgal. The mixtures were transfected into COS7 cells using the LT1 transfection reagent from Panvera. At 48 hours post-transfection, the media and soluble lysates were recovered and assayed by the Magic Lite Assay. Capture was with anti-sAg followed by detection with a conjugated anti-E1 MAb (eD5/C3) (Fig. 8A) or MAb sAg (Fig. 8B). Both E1 and sAg were increasingly secreted into the medium as the ratio of sAg:E2661-sAg was increased, with an optimum secretion at ratios in the range of 5:1 to 20:1.

In order to characterize the virus-like particles, 1 mL of culture medium from each condition was loaded onto a 5-30% sucrose gradient. The samples were centrifuged 4 hrs at 40,000 rpm using a Beclanan SW41 rotor. Eleven fractions were removed and assayed for E1 (Fig. 9A) or sAg (Fig. 9B) by the Magic Lite Assay. Both E1 and sAg were observed in highest amount in fractions 4-6, which is similar to the gradient position for the peak distribution of HBsAg-containing virus-like particles (Fig. 5).

### EXAMPLE 4

### Expression of HBV/HCV Virus-Like Particles Containing Both HBsAg-E2-661 and HBsAg-E1-330 Chimeric Antigens in COS7 Cells

A mixture of pCMVII opti 330 E1-sAg and pCMV-II-E2661-sAg (0.5 µg DNA of each plasmid) was combined with increasing amounts of pCMV-II-pS2-sAg. The total amount of DNA in each tube was normalized to 26 µg by the addition of pCMV-kM-βgal. The mixture was transfected into COS7 cells using the LT1 transfection reagent from Panvera. At 48 hours post-transfection, media and soluble lysates were recovered and assayed by the Magic Lite Assay, with capture by anti-sAg followed by detection with a conjugated anti-E2 or anti-E1 MAb (Fig. 10A) or a conjugated anti-sAg (Fig. 10B).

### EXAMPLE 5

### Use of HBV/HCV Virus-Like Particles to Generate an Immune Response

In order to test the ability of the subject HBV/HCV virus-like particles to generate an immune response *in vivo,* the following experiment was done. Four groups of 10 mice (Group 2 had 9 mice) were administered either DNA encoding HCV E2661 and pCMV-II, at a ratio of 5 times pCMV-II to E2661 (Group 1); pCMV-II-E2661-sAg (Fig. 4A) and pCMV-II, at a ratio of 5 times pCMV-II to pCMV-II-E2661-sAg (Group 2); pCMV-II-E2661-sAg and pCMV-II-pS2-sAg (Fig. 2A) at a ratio of 5 times pCMV-II-pS2-sAg to pCMV-II-E2661-sAg (Group 3); and pCMV-II and pCMV-II-pS2-sAg, at a ratio of 5 times pCMV-II-pS2-sAg to pCMV-II (Group 4).

All were immunized twice with 90 µg (45 µg/leg) per immunization, at day 0 and day 21, and blood was collected. Antibody titers were assessed by ELISA using a truncated E2 molecule, E2₇₁₅, which had been expressed in CHO cells, using the Magic Lite Assay described above. Results are shown in Table 1.

| Group # | Treatment | GM +/- SE |
|---|---|---|
| 1 | E2661 + 5x vector | 1300 +/- 85 |
| 2 | E2661-sAg + 5x vector | 1547 +/- 690 |
| 3 | E2661-sAg + 5x pS2sAg | 4175 +/- 603 |
| 4 | Vector + 5x pS2sAg | 0 |

A second experiment was run, again using 10 animals per group, except Group 5, which had 5 mice. The groups and results are shown in Table 2. Treatment protocol and antibody determination was as described above.

| Group # | Treatment | GM +/- SE |
|---|---|---|
| 1 | E2661 + 5x vector | 724+/96 |
| 2 | E2661 + 5x pS2sAg | 222 +/ 90 |
| 3 | E2661-sAg + 5x vector | 1018 +/ 305 |
| 4 | E2661-sAg + 5x pS2sAg | 1290 +/ 292 |
| 5 | mock | 0 |

As can be seen, titers to E2 in the E2₆₆₁-immunized mice were reduced in the presence of 5x sAg. This might have resulted from competition for docking to the endoplasmic reticulum. The E2 titers were higher when the E2 was fused to sAg. The addition of sAg in place of vector resulted in little change in titer. While this difference is small, comparison to the control with sAg is warranted.. Since excess sAg caused a reduction in E2 titers in the unfused antigen, in the context of the fusion there was an apparent compensation. This presumably reflects the ability of sAg to promote secretion of the fusion.

Thus, chimeric HCV/HBV virus-like particles, as well as methods of making and using the same are disclosed. From the foregoing, it will be appreciated that, although specific embodiments of the invention have been described herein for purposes of illustration, various modifications may be made without deviating from the spirit and scope of the appended claims.

## Claims

1. A virus-like particle for use as an immunogen, comprising a first hepatitis B virus surface antigen (HBsAg) and a chimeric antigen, wherein the Chimeric antigen comprises a second HBsAg which is covalently linked to an HCV immunogenic polypeptide, and wherein the first and the second HBsAg each comprise a substantial complete S domain.

2. The virus-like particle of claim 1, wherein the first HBsAg consists essentially of preS2 and S domains.

3. The virus-like particle of claim 2, wherein the first HBsAg consists essentially of preS1, preS2, and S domains.

4. The virus-like particle of claim 1, wherein the carboxy terminus of the HCV immunogenic polypeptide is linked to the amino terminus of the second HBsAg.

5. The virus-like particle of claim 1; wherein the first HBsAg expressed in excess relative to the chimeric antigen.

6. The virus-like particle of claim 5, wherein the first HBsAg is expressed using an amount of DNA that is between and 100 times the amount of DNA used to express the chimeric antigen.

7. The virus-like particle of claim 1, wherein the HCV immunogenic. polypeptide comprises an HCV E1 glycoprotein, an immunogenic fragment of an HCV E1 glycoprotein, an HCV E2 glycoprotein, or an immunogenic fragment of an HCV E2 glycoprotein.

8. The virus-like particle of claim 1, wherein the HCV immunogenic polypeptide consists essentially of an HCV E1 glycoprotein, an immunogenic fragment of an HCV E1 glycoprotein, an HCV E2 glycoprotein, or an immunogenic fragment of an HCV E2 glycoprotein.

9. The virus-like particle of claim 7, wherein the HCV immunogenic polypeptide comprises (a) amino acid residues 192 to 330 of an HCV-1 polyprotein; or (b) the corresponding residues of other HCV isolates; or (c) an immunogenic sequence having at least about 80% sequence identity to (a) or (b).

10. The virus-like particle of claim 7, wherein the HCV immunogenic polypeptide comprises (a) amino acid residues 384 to 661 of an HCV-1 polyprotein; or (b) the corresponding residues of other HCV isolates; or (c) an immunogenic sequence having at least about 80% sequence identity to (a) or (b).

11. The virus-like particle of claim 10, wherein the HCV immunogenic polypeptide consists essentially of amino acid residues 384 to 661 of an HCV polyprotein.

12. The virus-like particle of claim 1, wherein the HCV immunogenic polypeptide comprises (1) an HCV E1 glycoprotein or a fragment thereof and (2) an HCV E2 glycoprotein or a fragment thereof.

13. A virus-like particle for use as an immunogen, comprising a first HBsAg and first and second chimeric antigens, wherein the first chimeric antigen comprises a second HBsAg which is covalently linked to a first immunogenic polypeptide comprising an HCV E1 glycoprotein or an immunogenic fragment thereof, wherein the second chimeric antigen comprises a third HBsAg which is covalently linked to a second immunogenic polypeptide comprising an HCV E2 glycoprotein or an immunogenic fragment thereof, and wherein the first, second, and third HBsAg each comprise a substantially complete S domain.

14. The virus-like particle of claim 13, wherein the first HBsAg consists essentially of preS2 and S domains.

15. The virus-like particle of claim 13, wherein the first HBsAg consists essentially of preS1, preS2, and S domains.

16. The virus-like particle of claim 13, wherein the carboxy terminus of the first immunogenic polypeptide is linked to the amino terminus of the second HBsAg and the carboxy terminus of the second immunogenic polypeptide is linked to the amino terminus of the third HBsAg.

17. The virus-like particle of claim 13, wherein the first HBsAg is expressed in excess relative to the total amount of the first and second chimeric antigens.

18. The virus-like particle of claim 17, wherein the first HBsAg is expressed using an amount of DNA that is between 1 and 100 times the total amount of the DNA used to express the first and second chimeric antigens.

19. The virus-like particle of claim 13, wherein the first immunogenic polypeptide comprises (a) amino acid residues 192 to 330 of an HCV-1 polyprotein; or (b) the corresponding residues of other HCV isolates; or (c) an immunogenic sequence having at least about 80% sequence identity to (a) or (b).

20. The virus-like particle of claim 13, wherein the second immunogenic polypeptide comprises (a) amino acid residues 384 to 661 of an HCV-1 polyprotein; or (b) the corresponding residues of other HCV isolates; or (c) an immunogenic sequence having at least about 80% sequence identity to (a) or (b).

21. The virus-like particle of claim 13, wherein the first immunogenic polypeptide consists essentially of amino acid residues 192 to 330 of an HCV-1 polyprotein and the second immunogenic polypeptide consists essentially of amino acid residues 384 to 661 of an HCV-1 polyprotein.

22. An immunogenic composition comprising:
a virus-like particle comprising a first HBsAg and a chimeric antigen, wherein the chimeric antigen comprises a second HBsAg which is linked to an HCV immunogenic polypeptide, and wherein the first and the second HBsAg each comprise a substantially complete S domain; and
a pharmaceutically acceptable carrier.

23. The immunogenic composition of claim 22, wherein the first HBsAg is expressed in excess compared to the chimeric antigen.

24. The immunogenic composition of claim 23, wherein the first HBsAg is expressed using an amount of DNA that is between 1 and 100 times the amount of DNA used to express the chimeric antigen.

25. The immunogenic composition of claim 22, wherein the HCV immunogenic polypeptide comprises an HCV E1 glycoprotein, an immunogenic fragment of an HCV E1 glycoprotein, an HCV E2 glycoprotein, or an immunogenic fragment of an HCV E2 glycoprotein.

26. The immunogenic composition of claim 22, wherein the HCV immunogenic polypeptide comprises (a) amino acid residues 192 to 330 of an HCV-1 polyprotein; or (b) the corresponding residues of other HCV isolates; or (c) an immunogenic sequence having at least about 80% sequence identity to (a) or (b).

27. The immunogenic composition of claim 22, wherein the HCV immunogenic polypeptide consists essentially of amino acid residues 192 to 330 of an HCV-1 polyprotein; or (b) the corresponding residues of other HCV isolates.

28. The immunogenic composition of claim 22, wherein the HCV immunogenic polypeptide comprises (a) amino acid residues 384 to 661 of an HCV-1 polyprotein; or (b) the corresponding. residues of other HCV isolates; or (c) an immunogenic sequence having at least about 80% sequence identity to (a) or (b).

29. The immunogenic composition of claim 22, wherein the HCV immunogenic polypeptide comprises (a) amino acid residues 384 to 661 of an HCV-1 polyprotein; or (b) the corresponding residues of other HCV isolates.

30. An immunogenic composition comprising virus-like particles, comprising a first HBsAg and Urst and second chimeric antigens, wherein the first chimeric antigen comprises a second HBsAg which is linked to a first immunogenic polypeptide comprising an HCV E1 glycoprotein or an immunogenic fragment thereof, wherein the second chimeric antigen comprises a third HBsAg which is linked to a second immunogenic polypeptide comprising an HCV E2 glycoprotein or an immunogenic fragment thereof, and wherein the first, second, and third HBsAg each comprise a substantially complete S domain.

31. The immunogenic composition of claim 30, wherein the first HBsAg consists essentially of preS2 and S domains.

32. The immunogenic composition of claim 30, wherein the first HBsAg consists essentially of preS1, preS2, and S domains.

33. The immunogenic composition of claim 30, wherein the carboxy terminus of the first immunogenic polypeptide is linked to the amino terminus of the second HBsAg and the carboxy terminus of the second immunogenic polypeptide is linked to the amino terminus of the third HBsAg.

34. The immunogenic composition of claim 30, wherein the first HBsAg is expressed in excess relative to the total amount of the first and second chimeric antigens.

35. The immunogenic composition of claim 34, wherein the first HBsAg is expressed using an amount of DNA that is between 1 and 100 times the amount of DNA used to express the chimeric antigen.

36. The immunogenic composition of claim 30, wherein the first immunogenic polypeptide comprises (a) amino acid residues 192 to 330 of an HCV-1 polyprotein; or (b) the corresponding residues of other HCV isolates; or (c) an immunogenic sequence having at least about 80% sequence identity to (a) or (b).

37. The immunogenic composition of claim 30, wherein the second immunogenic polypeptide comprises (a) amino acid residues 384 to 661 of an HCV-1. polyprotein; or (b) the corresponding residues of other HCV isolates; or (c) an immunogenic sequence having at least about 80% sequence identity to (a) or (b).

38. An in vitro method of producing virus-like particles comprising the steps of:
culturing a cell in a culture medium, whereby the cell expresses virus-like particles comprising a first HBsAg and a chimeric antigen, wherein the chimeric antigen comprises a second HBsAg which is covalently linked to an HCV immunogenic polypeptide, and wherein the first and the second HBsAg each comprise a substantially complete S domain; and
isolating the virus-like particles from the culture medium.

39. The method of claim 38, wherein the cell is a CHO cell or a COS cell.

40. An in vitro method of producing a cell line that expresses virus-like particles, comprising the steps of:
transfecting a cell with a vector that expresses virus-like particles comprising a first HBsAg and a chimeric antigen, wherein the chimeric antigen comprises a second HBsAg which is covalently linked to an HCV immunogenic polypeptide, and wherein the first and the second HBsAg each comprise a substantially complete S domain; and
culturing the cell to produce a cell line that expresses the virus-like particles.

41. The method of claim 40, wherein the vector is a plasmid vector.

42. The method of claim 40, wherein the plasmid vector is pCMVII opti 330 E (SEQ ID NO:4) or pCMV-II-E2661-sAg (SEQ ID NO:6).

43. The method of claim 40, wherein the vector is a viral vector.

44. The method of claim 43, wherein the viral vector is essentially free of infectious virus.

45. A cell line that expresses a virus-like particle comprising a first HBsAg and a chimeric antigen, wherein the chimeric antigen comprises a second HBsAg which is linked to an HCV immunogenic polypeptide, and wherein the first and the second HBsAg each comprise a substantially complete S domain.

46. A cell line that expresses a virus-like particle comprising a first HBsAg and first and second chimeric antigens, wherein the first chimeric antigen comprises a second HBsAg which is linked to a first immunogenic polypeptide comprising an HCV E1 glycoprotein or an immunogenic fragment thereof, wherein the second chimeric antigen comprises a third HBsAg which is linked to a second immunogenic polypeptide comprising an HCV E2 glycoprotein or an immunogenic fragment thereof, and wherein the first, second, and third HBsAg each comprise a substantially complete S domain.

## Patentansprüche

1. Virusähnliches Partikel zur Verwendung als Immunogen, umfassend ein erstes Hepatitis-B-Virus-Oberflächen-Antigen (HBsAg) und ein chimäres Antigen, wobei das chimäre Antigen ein zweites HBsAg umfasst, das kovalent mit einem immunogenen HCV-Polypeptid verbunden ist, und wobei das erste und das zweite HBsAg jeweils eine im Wesentlichen vollständige S-Domäne umfassen.

2. Virusähnliches Partikel nach Anspruch 1, wobei das erste HBsAg im Wesentlichen aus preS2- und S-Domänen besteht.

3. Virusähnliches Partikel nach Anspruch 2, wobei das erste HBsAg im Wesentlichen aus preS1-, preS2- und S-Domänen besteht.

4. Virusähnliches Partikel nach Anspruch 1, wobei der Carboxy-Terminus des immunogenen HCV-Polypeptids mit dem Amino-Terminus des zweiten HBsAg verbunden ist.

5. Virusähnliches Partikel nach Anspruch 1, wobei das erste HBsAg im Vergleich zum chimären Antigen im Überschuss exprimiert wird.

6. Virusähnliches Partikel nach Anspruch 5, wobei das erste HBsAg unter Verwendung einer Menge von DNA exprimiert wird, die zwischen 1 und 100 Mal der Menge der DNA liegt, die verwendet wird, um das chimäre Antigen zu exprimieren.

7. Virusähnliches Partikel nach Anspruch 1, wobei das immunogene HCV-Polypeptid ein HCV-E1-Glycoprotein, ein immunogenes Fragment eines HCV-E1-Glycoproteins, ein HCV-E2-Glycoprotein oder ein immunogenes Fragment eines HCV-E2-Glycoproteins umfasst.

8. Virusähnliches Partikel nach Anspruch 1, wobei das immunogene HCV-Polypeptid im Wesentlichen aus einem HCV-E1-Glycoprotein, einem immunogenen Fragment eines HCV-E1-Glycoproteins, einem HCV-E2-Glycoprotein oder einem immunogenen Fragment eines HCV-E2-Glycoproteins besteht.

9. Virusähnliches Partikel nach Anspruch 7, wobei das immunogene HCV-Polypeptid (a) Aminosäurereste 192 bis 330 eines HCV-1-Polyproteins; oder (b) die korrespondierenden Reste anderer HCV-Isolate; oder (c) eine immunogene Sequenz, die mindestens 80% Sequenzidentität mit (a) oder (b) hat, umfasst.

10. Virusähnliches Partikel nach Anspruch 7, wobei das immunogene HCV-Polypeptid (a) Aminosäurereste 384 bis 661 eines HCV-1-Polyproteins; oder (b) die korrespondierenden Reste anderer HCV-Isolate; oder (c) eine immunogene Sequenz, die mindestens 80% Sequenzidentität mit (a) oder (b) hat, umfasst.

11. Virusähnliches Partikel nach Anspruch 10, wobei das immunogene HCV-Polypeptid im Wesentlichen aus Aminosäureresten 384 bis 661 eines HCV-Polyproteins besteht.

12. Virusähnliches Partikel nach Anspruch 1, wobei das immunogene HCV-Polypeptid (1) ein HCV-E1-Glycoprotein oder ein Fragment davon und (2) ein HCV-E2-Glycoprotein oder ein Fragment davon umfasst.

13. Virusähnliches Partikel zur Verwendung als Immunogen, umfassend ein erstes HBsAg und erste und zweite chimäre Antigene, wobei das erste chimäre Antigen ein zweites HBsAg umfasst, das mit einem ersten immunogenen Polypeptid kovalent verbunden ist, wobei das erste immunogene Polypeptid ein HCV-E1-Glycoprotein oder ein immunogenes Fragment davon umfasst, und wobei das zweite chimäre Antigen ein drittes HBsAg umfasst, das kovalent mit einem zweiten immunogenen Polypeptid verbunden ist, wobei das zweite immunogene Polypeptid ein HCV-E2-Glycoprotein oder ein immunogenes Fragment davon umfasst und wobei das erste, zweite und dritte HBsAg jeweils eine im Wesentlichen vollständige S-Domäne umfassen.

14. Virusähnliches Partikel nach Anspruch 13, wobei das erste HBsAg im Wesentlichen aus preS2- und S-Domänen besteht.

15. Virusähnliches Partikel nach Anspruch 13, wobei das erste HBsAg im Wesentlichen aus preS1-, preS2- und S-Domänen besteht.

16. Virusähnliches Partikel nach Anspruch 13, wobei der Carboxy-Terminus des ersten immunogenen Polypeptids mit dem Amino-Terminus des zweiten HBsAg und der Carboxy-Terminus des zweiten immunogenen Polypetids mit dem Amino-Terminus des dritten HBsAg verbunden ist.

17. Virusähnliches Partikel nach Anspruch 13, wobei das erste HBsAg im Vergleich zur Gesamtmenge der ersten und zweiten chimären Antigene im Überschuss exprimiert wird.

18. Virusähnliches Partikel nach Anspruch 17, wobei das erste HBsAg unter Verwendung einer Menge von DNA exprimiert wird, die zwischen 1 und 100 Mal der Gesamtmenge der DNA liegt, die verwendet wird, um die ersten und zweiten chimären Antigene zu exprimieren.

19. Virusähnliches Partikel nach Anspruch 13, wobei das erste immunogene Polypeptid (a) Aminosäurereste 192 bis 330 eines HCV-1-Polyproteins; oder (b) die korrespondierenden Reste anderer HCV-Isolate; oder (c) eine immunogene Sequenz, die mindestens 80% Sequenzidentität mit (a) oder (b) hat, umfasst.

20. Virusähnliches Partikel nach Anspruch 13, wobei das zweite immunogene Polypeptid (a) Aminosäurereste 384 bis 661 eines HCV-1-Polyproteins; oder (b) die korrespondierenden Reste anderer HCV-Isolate; oder (c) eine immunogene Sequenz, die mindestens 80% Sequenzidentität mit (a) oder (b) hat, umfasst.

21. Virusähnliches Partikel nach Anspruch 13, wobei das erste immunogene Polypeptid im Wesentlichen aus Aminosäureresten 192 bis 330 eines HCV-1-Polyproteins und das zweite immunogene Polypeptid im Wesentlichen aus Aminosäureresten 384 bis 661 eines HCV-1-Polyproteins besteht.

22. Immunogene Zusammensetzung, umfassend:
ein virusähnliches Partikel, umfassend ein erstes HBsAg und ein chimäres Antigen, wobei das chimäre Antigen ein zweites HBsAg umfasst, das mit einem immunogenen HCV-Polypeptid verbunden ist, und wobei das erste und das zweite HBsAg jeweils eine im Wesentlichen vollständige S-Domäne umfassen; und
einen pharmazeutisch verträglichen Träger.

23. Immunogene Zusammensetzung nach Anspruch 22, wobei das erste HBsAg im Vergleich mit dem chimären Antigen im Überschuss exprimiert wird.

24. Immunogene Zusammensetzung nach Anspruch 23, wobei das erste HBsAg unter Verwendung einer Menge von DNA exprimiert wird, die zwischen 1 und 100 Mal der Menge der DNA liegt, die verwendet wird, um das chimäre Antigen zu exprimieren.

25. Immunogene Zusammensetzung nach Anspruch 22, wobei das immunogene HCV-Polypeptid ein HCV-E1-Glycoprotein, ein immunogenes Fragment eines HCV-E1-Glycoproteins, ein HCV-E2-Glycoprotein oder ein immunogenes Fragment eines HCV-E2-Glycoproteins umfasst.

26. Immunogene Zusammensetzung nach Anspruch 22, wobei das immunogene HCV-Polypeptid (a) Aminosäurereste 192 bis 330 eines HCV-1-Polyproteins; oder (b) die korrespondierenden Reste anderer HCV-Isolate; oder (c) eine immunogene Sequenz, die mindestens 80% Sequenzidentität mit (a) oder (b) hat, umfasst.

27. Immunogene Zusammensetzung nach Anspruch 22, wobei das immunogene HCV-Polypeptid im Wesentlichen aus Aminosäureresten 192 bis 330 eines HCV-1-Polyproteins; oder (b) den korrespondierenden Resten anderer HCV-Isolate besteht.

28. Immunogene Zusammensetzung nach Anspruch 22, wobei das immunogene HCV-Polypeptid (a) Aminosäurereste 384 bis 661 eines HCV-1-Polyproteins; oder (b) die korrespondierenden Reste anderer HCV-Isolate; oder (c) eine immunogene Sequenz, die mindestens 80% Sequenzidentität mit (a) oder (b) hat, umfasst.

29. Immunogene Zusammensetzung nach Anspruch 22, wobei das immunogene HCV-Polypeptid (a) Aminosäurereste 384 bis 661 eines HCV-1-Polyproteins; oder (b) die korrespondierenden Reste anderer HCV-Isolate umfasst.

30. Immunogene Zusammensetzung, umfassend virusähnliche Partikel, umfassend ein erstes HBsAg und erste und zweite chimäre Antigene, wobei das erste chimäre Antigen ein zweites HBsAg umfasst, das mit einem ersten immunogenen Polypeptid verbunden ist, wobei das erste immunogene Polypeptid ein HCV-E1-Glycoprotein oder ein immunogenes Fragment davon umfasst, und wobei das zweite chimäre Antigen ein drittes HBsAg umfasst, das mit einem zweiten immunogenen Polypeptid verbunden ist, wobei das zweite immunogene Polypeptid ein HCV-E2-Glycoprotein oder ein immunogenes Fragment davon umfasst und wobei das erste, zweite und dritte HBsAg jeweils eine im Wesentlichen vollständige S-Domäne umfassen.

31. Immunogene Zusammensetzung nach Anspruch 30, wobei das erste HBsAg im Wesentlichen aus preS2- und S-Domänen besteht.

32. Immunogene Zusammensetzung nach Anspruch 30, wobei das erste HBsAg im Wesentlichen aus preS1-, preS2- und S-Domänen besteht.

33. Immunogene Zusammensetzung nach Anspruch 30, wobei der Carboxy-Terminus des ersten immunogenen Polypeptids mit dem Amino-Terminus des zweiten HBsAg und der Carboxy-Terminus des zweiten immunogenen Polypeptids mit dem Amino-Terminus des dritten HBsAg verbunden ist.

34. Immunogene Zusammensetzung nach Anspruch 30, wobei das erste HBsAg im Vergleich mit der Gesamtmenge des ersten und zweiten chimären Antigens im Überschuss exprimiert wird.

35. Immunogene Zusammensetzung nach Anspruch 34, wobei das erste HBsAg unter Verwendung einer Menge von DNA exprimiert wird, die zwischen 1 und 100 Mal der Menge DNA liegt, die verwendet wird, um das chimäre Antigen zu exprimieren.

36. immunogene Zusammensetzung nach Anspruch 30, wobei das erste immunogene Polypeptid (a) Aminosäurereste 192 bis 330 eines HCV-1-Polyproteins; oder (b) die korrespondierenden Reste anderer HCV-Isolate; oder (c) eine immunogene Sequenz, die mindestens 80% Sequenzidentität mit (a) oder (b) hat, umfasst.

37. Immunogene Zusammensetzung nach Anspruch 30, wobei das zweite immunogene Polypeptid (a) Aminosäurereste 384 bis 661 eines HCV-1-Polyproteins; oder (b) die korrespondierenden Reste anderer HCV-Isolate; oder (c) eine immunogene Sequenz, die mindestens 80% Sequenzidentität mit (a) oder (b) hat, umfasst.

38. In vitro-Verfahren zur Herstellung virusähnlicher Partikel, umfassend die folgenden Schritte:
Züchten einer Zelle in einem Kulturmedium, wobei die Zelle virusähnliche Partikel exprimiert, die ein erstes HBsAg und ein chimäres Antigen umfassen, wobei das chimäre Antigen ein zweites HBsAg umfasst, das kovalent mit einem immunogenen HCV-Polypeptid verbunden ist, und wobei das erste und das zweite HBsAg jeweils eine im Wesentlichen vollständige S-Domäne umfassen;
und Isolieren der virusähnlichen Partikel aus dem Kulturmedium.

39. Verfahren nach Anspruch 38, wobei die Zelle eine CHO-Zelle oder eine COS-Zelle ist.

40. In vitro-Verfahren zur Herstellung einer Zelllinie, die virusähnliche Partikel exprimiert, umfassend die folgenden Schritte: Transfizieren einer Zelle mit einem Vektor, der virusähnliche Partikel exprimiert, die ein erstes HBsAg und ein chimäres Antigen umfassen, wobei das chimäre Antigen ein zweites HBsAg umfasst, das kovalent mit einem immunogenen HCV-Polypeptid verbunden ist, und wobei das erste und das zweite HBsAg jeweils eine im Wesentlichen vollständige S-Domäne umfassen; und Züchten der Zelle, um eine Zelllinie zu erzeugen, die die virusähnlichen Partikel exprimiert.

41. Verfahren nach Anspruch 40, wobei der Vektor ein Plasmidvektor ist.

42. Verfahren nach Anspruch 40, wobei der Plasmidvektor pCMVII opti 330 E1 (SEQ ID NR: 4) oder pCMV-II-E2661-sAg (SEQ ID NR: 6) ist.

43. Verfahren nach Anspruch 40, wobei der Vektor ein viraler Vektor ist.

44. Verfahren nach Anspruch 43, wobei der virale Vektor im Wesentlichen von infektiösen Viren frei ist.

45. Zelllinie, die ein virusähnliches Partikel exprimiert, das ein erstes HBsAg und ein chimäres Antigen umfasst, wobei das chimäre Antigen ein zweites HBsAg umfasst, das mit einem immunogenen HCV-Polypeptid verbunden ist, und wobei das erste und das zweite HBsAg jeweils eine im Wesentlichen vollständige S-Domäne umfassen.

46. Zelllinie, die ein virusähnliches Partikel umfasst, das ein erstes HBsAg und erste und zweite chimäre Antigene umfasst, wobei das erste chimäre Antigen ein zweites HBsAg umfasst, das mit einem ersten immunogenen Polypeptid verbunden ist, wobei das erste immunogene Polypeptid ein HCV-E1-Glycoprotein oder ein immunogenes Fragment davon umfasst, und wobei das zweite chimäre Antigen ein drittes HBsAg umfasst, das mit einem zweiten immunogenen Polypeptid verbunden ist, wobei das zweite immunogene Polypeptid ein HCV-E2-Glycoprotein oder ein immunogenes Fragment davon umfasst, und wobei das erste, zweite und dritte HBsAg jeweils eine im Wesentlichen vollständige S-Domäne umfassen.

## Revendications

1. Particule du type virus pour son utilisation en tant qu'immunogène, comprenant un premier antigène de surface du virus de l'hépatite B (HBsAg) et un antigène chimère, l'antigène chimère comprenant un second HBsAg lié de façon covalente à un polypeptide immunogène du VHC et le premier et le second HBsAg comprenant chacun un domaine S substantiellement complet.

2. Particule du type virus selon la revendication 1, le premier HBsAg étant essentiellement constitué par les domaines preS2 et S.

3. Particule du type virus selon la revendication 2, le premier HBsAg étant essentiellement constitué par les domaines preS1, preS2 et S.

4. Particule du type virus selon la revendication 1, la terminaison carboxy du polypeptide immunogène du VHC étant liée à la terminaison amino du second HBsAg.

5. Particule du type virus selon la revendication 1, le premier HBsAg étant exprimé en excès par rapport à l'antigène chimère.

6. Particule du type virus selon la revendication 5, le premier HBsAg étant exprimé à l'aide d'une quantité d'ADN qui est égale entre 1 et 100 fois la quantité d'ADN utilisée pour exprimer l'antigène chimère.

7. Particule du type virus selon la revendication 1, le polypeptide immunogène du VHC comprenant une glycoprotéine E1 du VHC, un fragment immunogène d'une glycoprotéine E1 du VHC, une glycoprotéine E2 du VHC, ou un fragment immunogène d'une glycoprotéine E2 du VHC.

8. Particule du type virus selon la revendication 1, le polypeptide immunogène du VHC étant constitué essentiellement par une glycoprotéine E1 du VHC, un fragment immunogène d'une glycoprotéine E1 du VHC, une glycoprotéine E2 du VHC, ou un fragment immunogène d'une glycoprotéine E2 du VHC.

9. Particule du type virus selon la revendication 7, le polypeptide immunogène du VHC comprenant (a) les restes d'aminoacides 192 à 330 d'une polyprotéine de VHC-1 ; ou (b) les restes correspondants d'autres isolats de VHC ; ou (c) une séquence immunogène ayant au moins 80 % d'identité par rapport aux séquences (a) ou (b).

10. Particule du type virus selon la revendication 7, le polypeptide immunogène du VHC comprenant (a) les restes d'aminoacides 384 à 661 d'une polyprotéine de VHC-1 ; ou (b) les restes correspondants d'autres isolats de VHC ; ou (c) une séquence immunogène ayant au moins 80 % d'identité par rapport aux séquences (a) ou (b).

11. Particule du type virus selon la revendication 10, le polypeptide immunogène du VHC étant constitué essentiellement par les restes d'aminoacides 384 à 661 d'une polyprotéine du VHC.

12. Particule du type virus selon la revendication 1, le polypeptide immunogène du VHC comprenant (1) une glycoprotéine E1 du VHC ou un de ses fragments et (2) une glycoprotéine E2 du VHC ou un de ses fragments.

13. Particule du type virus pour son utilisation en tant qu'immunogène, comprenant un premier HBsAg et un premier et un second antigènes chimères, le premier antigène chimère comprenant un second HBsAg lié de façon covalente à un premier polypeptide immunogène comprenant une glycoprotéine E1 du VHC ou un de ses fragments immunogènes, le second antigène chimère comprenant un troisième HBsAg lié de façon covalente à un second polypeptide immunogène comprenant une glycoprotéine E2 du VHC ou un de ses fragments immunogènes, et le premier, le second et le troisième HBsAg comprenant chacun un domaine S substantiellement complet.

14. Particule du type virus selon la revendication 13, le premier HBsAg étant essentiellement constitué par les domaines preS2 et S.

15. Particule du type virus selon la revendication 13, le premier HBsAg étant essentiellement constitué par les domaines preS1, preS2 et S.

16. Particule du type virus selon la revendication 13, la terminaison carboxy du premier polypeptide immunogène étant liée à la terminaison amino du second HBsAg et la terminaison carboxy du second polypeptide immunogène étant liée à la terminaison amino du troisième HBsAg.

17. Particule du type virus selon la revendication 13, le premier HBsAg étant exprimé en excès par rapport à la somme des quantités du premier et du second antigènes chimères.

18. Particule du type virus selon la revendication 17, le premier HBsAg étant exprimé à l'aide d'une quantité d'ADN qui est égale entre 1 et 100 fois la quantité d'ADN utilisée pour exprimer le premier et le second antigènes chimères.

19. Particule du type virus selon la revendication 13, le premier polypeptide immunogène comprenant (a) les restes d'aminoacides 192 à 330 d'une polyprotéine de VHC-1 ; ou (b) les restes correspondants d'autres isolats de VHC ; ou (c) une séquence immunogène ayant au moins 80 % d'identité par rapport aux séquences (a) ou (b).

20. Particule du type virus selon la revendication 13, le second polypeptide immunogène comprenant (a) les restes d'aminoacides 384 à 661 d'une polyprotéine de VHC-1 ; ou (b) les restes correspondants d'autres isolats de VHC ; ou (c) une séquence immunogène ayant au moins 80 % d'identité par rapport aux séquences (a) ou (b).

21. Particule du type virus selon la revendication 13, le premier polypeptide immunogène étant constitué essentiellement par les restes d'aminoacides 192 à 330 d'une polyprotéine de VHC-1 et le second polypeptide immunogène étant constitué essentiellement par les restes d'aminoacides 384 à 661 d'une polyprotéine du VHC-1.

22. Composition immunogène comprenant :
une particule du type virus comprenant un premier HBsAg et un antigène chimère, l'antigène chimère comprenant un second HBsAg lié à un polypeptide immunogène du VHC et le premier et le second HBsAg comprenant chacun un domaine S substantiellement complet ; et
un support pharmaceutiquement acceptable.

23. Composition immunogène selon la revendication 22, le premier HBsAg étant exprimé en excès par rapport à l'antigène chimère.

24. Composition immunogène selon la revendication 23, le premier HBsAg étant exprimé à l'aide d'une quantité d'ADN qui est égale entre 1 et 100 fois la quantité d'ADN utilisée pour exprimer l'antigène chimère.

25. Composition immunogène selon la revendication 22, le polypeptide immunogène du VHC comprenant une glycoprotéine E1 du VHC, un fragment immunogène d'une glycoprotéine E1 du VHC, une glycoprotéine E2 du VHC, ou un fragment immunogène d'une glycoprotéine E2 du VHC.

26. Composition immunogène selon la revendication 22, le polypeptide immunogène du VHC comprenant (a) les restes d'aminoacides 192 à 330 d'une polyprotéine de VHC-1 ; ou (b) les restes correspondants d'autres isolats de VHC ; ou (c) une séquence immunogène ayant au moins 80 % d'identité par rapport aux séquences (a) ou (b).

27. Composition immunogène selon la revendication 22, le polypeptide immunogène du VHC étant constitué essentiellement par les restes d'aminoacides 192 à 330 d'une polyprotéine de VHC-1 ; ou (b) les restes correspondants d'autres isolats de VHC.

28. Composition immunogène selon la revendication 22, le polypeptide immunogène du VHC comprenant (a) les restes d'aminoacides 384 à 661 d'une polyprotéine de VHC-1 ; ou (b) les restes correspondants d'autres isolats de VHC ; ou (c) une séquence immunogène ayant au moins 80 % d'identité par rapport aux séquences (a) ou (b).

29. Composition immunogène selon la revendication 22, le polypeptide immunogène du VHC comprenant (a) les restes d'aminoacides 384 à 661 d'une polyprotéine de VHC-1 ; ou (b) les restes correspondants d'autres isolats de VHC.

30. Composition immunogène comprenant des particules du type virus, comprenant un premier HBsAg et un premier et un second antigènes chimères, le premier antigène chimère comprenant un second HBsAg lié à un premier polypeptide immunogène comprenant une glycoprotéine E1 du VHC ou un de ses fragments immunogènes, le second antigène chimère comprenant un troisième HBsAg lié à un second polypeptide immunogène comprenant une glycoprotéine E2 du VHC ou un de ses fragments immunogènes, et le premier, le second et le troisième HBsAg comprenant chacun un domaine S substantiellement complet.

31. Composition immunogène selon la revendication 30, le premier HBsAg étant essentiellement constitué par les domaines preS2 et S.

32. Composition immunogène selon 1a revendication 30, le premier HBsAg étant essentiellement constitué par les domaines preS1, preS2 et S.

33. Composition immunogène selon la revendication 30, la terminaison carboxy du premier polypeptide immunogène étant liée à la terminaison amino du second HBsAg et la terminaison carboxy du second polypeptide immunogène étant liée à la terminaison amino du troisième HBsAg.

34. Composition immunogène selon la revendication 30, le premier HBsAg étant exprimé en excès par rapport à la somme des quantités du premier et du second antigènes chimères.

35. Composition immunogène selon la revendication 34, le premier HBsAg étant exprimé à l'aide d'une quantité d'ADN qui est égale entre 1 et 100 fois la quantité d'ADN utilisée pour exprimer l'antigène chimère.

36. Composition immunogène selon la revendication 30, le premier polypeptide immunogène comprenant (a) les restes d'aminoacides 192 à 330 d'une polyprotéine de VHC-1; ou (b) les restes correspondants d'autres isolats de VHC ; ou (c) une séquencé immunogène ayant au moins 80 % d'identité par rapport aux séquences (a) ou (b).

37. Composition immunogène selon la revendication 30, lé second polypeptide immunogène comprenant (a) les restes d'aminoacides 384 à 661 d'une polyprotéine de VHC-1 ; ou (b) les restes correspondants d'autres isolats de VHC ; ou (c) une séquence immunogène ayant au moins 80 % d'identité par rapport aux séquences (a) ou (b).

38. Méthode *in vitro* pour la production de particules du type virus, comprenant les étapes de :
la culture d'une cellule dans un milieu de culture, la cellule exprimant des particules du type virus comprenant un premier HBsAg et un antigène chimère, l'antigène chimère comprenant un second HBsAg lié de façon covalente à un polypeptide immunogène du VHC et le premier et le second HBsAg comprenant chacun un domaine S substantiellement complet ; et
l'isolement des particules du type virus du milieu de culture.

39. Méthode selon la revendication 38, dans laquelle la cellule est une cellule CHO ou une cellule COS.

40. Méthode *in vitro* pour la production d'une lignée cellulaire exprimant des particules du type virus, comprenant les étapes de :
la transfection d'une cellule par un vecteur qui exprime des particules du type virus comprenant un premier HBsAg et un antigène chimère, l'antigène chimère comprenant un second HBsAg lié de façon covalente à un polypeptide immunogène du VHC et le premier et le second HBsAg comprenant chacun un domaine S substantiellement complet ; et
la culture de la cellule pour produire une lignée cellulaire qui exprime des particules du type virus.

41. Méthode selon la revendication 40, dans laquelle le vecteur est un vecteur plasmide.

42. Méthode selon la revendication 40, dans laquelle le vecteur plasmide est pCMVII opti 330 E1 (SEQ ID n°:4) ou pCMV-II-E2661-sAg (SEQ ID n°:6).

43. Méthode selon la revendication 40, dans laquelle le vecteur est un vecteur viral.

44. Méthode selon la revendication 43, dans laquelle le vecteur viral est essentiellement exempt de virus infectieux.

45. Lignée cellulaire qui exprime des particules du type virus comprenant un premier HBsAg et un antigène chimère, l'antigène chimère comprenant un second HBsAg lié à un polypeptide immunogène du VHC et le premier et le second HBsAg comprenant chacun un domaine S substantiellement complet.

46. Lignée cellulaire qui exprime des particules du type virus comprenant un premier HBsAg et un premier et un second antigènes chimères, le premier antigène chimère comprenant un second HBsAg lié à un premier polypeptide immunogène comprenant une glycoprotéine E1 du VHC ou un de ses fragments immunogènes, le second antigène chimère comprenant un troisième HBsAg lié à un second polypeptide immunogène comprenant une glycoprotéine E2 du VHC ou un de ses fragments immunogènes, et le premier, le second et le troisième HBsAg comprenant chacun un domaine S substantiellement complet.
